(19) Europäisches Patentamt
European Patent Office
Office européen des brevets

(11) Publication number : 0 467 714 A1

# (12) EUROPEAN PATENT APPLICATION

(21) Application number : **91306618.9**

(22) Date of filing : **19.07.91**

(51) Int. Cl.$^5$ : **C07K 13/00,** C07K 3/28, C12N 15/09, A61K 39/39, A61K 39/095

A request for addition of figures 1 to 3 has been filed pursuant to Rule 88 EPC. A decision on the request will be taken during the proceedings before the Examining Division (Guidelines for Examination in the EPO, A-V, 2.2).

Amended claims in accordance with Rule 86 (2) EPC for the following Contracting States: ES + GR.

(30) Priority : **19.07.90 US 555329**
**19.07.90 US 555204**
**19.07.90 US 555978**
**10.01.91 US 639457**
**19.06.91 US 715274**

(43) Date of publication of application :
**22.01.92 Bulletin 92/04**

(84) Designated Contracting States :
**AT BE CH DE DK ES FR GB GR IT LI LU NL SE**

(71) Applicant : **MERCK & CO. INC.**
**126, East Lincoln Avenue P.O. Box 2000**
**Rahway New Jersey 07065-0900 (US)**

(72) Inventor : **Oliff, Allen I.**
**1412 Florence Drive**
**Gwynedd Valley, PA 19437 (US)**
Inventor : **Liu, Margaret A.**
**4 Cushman Road**
**Rosemont, PA 19190 (US)**
Inventor : **Friedman, Arther**
**121 Froghollow Road**
**Churchville, PA 18966 (US)**
Inventor : **Tai, Joseph Y.**
**1370 Cinnamon Drive**
**Fort Washington, PA 19034 (US)**
Inventor : **Donnelly, John J.**
**1505 Briarwood Road**
**Havertown, PA 19083 (US)**
Inventor : **Jones, Deborah D.**
**1126 Canterbury Drive**
**Lansdale, PA 19446 (US)**
Inventor : **Montgomery, Donna L.**
**9 Hickory Lane**
**Chalfont, PA 18914 (US)**
Inventor : **Lowe, Robert S.**
**232 Maple Avenue**
**Harleysville, PA 19438 (US)**

(74) Representative : **Barrett-Major, Julie Diane et al**
**Merck & Co., Inc. European Patent Department Terlings Park Eastwick Road**
**Harlow Essex CM20 2QR (GB)**

## (54) The class II protein of the outer membrane of neisseria meningitidis.

(57) The Class II major immuno-enhancing protein (MIEP) of Neisseria meningitidis, purified directly from the outer membrane of Neisseria meningitidis, or obtained through recombinant cloning and expression of DNA encoding the MIEP of Neisseria meningitidis, has immunologic carrier as well as immunologic enhancement and mitogenic properties.

EP 0 467 714 A1

## BACKGROUND OF THE INVENTION

The outer membrane protein complex (OMPC) of Neisseria meningitidis is used as an immunologic carrier in vaccines for human use. OMPC consists of vesicles containing a variety of proteins as well as membranous lipids, including lipopolysaccharide (LPS or endotoxin).

OMPC has the property of immune enhancement, and when an antigen is chemically coupled to it, an increased antibody response to the antigen results. OMPC is currently used in vaccines for human infants against infectious agents such as Haemophilus influenzae, and renders the infants capable of mounting an IgG and memory immune response to polyribosyl ribitol phosphate (PRP) of H. influenzae, when PRP is chemically coupled to OMPC.

OMPC is a mixture of a variety of proteins and lipids, and it was not known which component or components of OMPC bestows the beneficial immune enhancing effect to the coupled antigens. However, some potentially negative aspects of using OMPC in human vaccines include LPS related reactions such as fever, endotoxic shock, hypotension, neutropenia, activation of the alternative complement pathway, intravascular coagulation, and possibly death.

Furthermore, OMPC-antigen conjugates are quite heterogeneous in that the antigen may become conjugated to any of the protein moieties which make up OMPC.

## OBJECTS OF THE INVENTION

It is an object of the present invention to provide substantially pure Class II, major immuno enhancing protein (MIEP) derived directly from the outer membrane of Neisseria meningtidis, free from other Neisseria meningitidis outer membrane components. It is another object of the present invention to provide substantially pure recombinant MIEP of the outer membrane of Neisseria meningitidis, produced in a recombinant host cell, completely free of all other Neisseria meningitidis proteins. A further object of the present invention is to provide an efficient immunocarrier protein for the enhancement of an immune response to antigens, comprising either MIEP purified directly from the outer membrane of Neisseria meningitidis, or recombinant MIEP of Neisseria meningitidis produced in a recombinant host cell. Another object of the present invention is to provide a protein which possesses immune mitogenic activity, comprising either MIEP purified directly from the outer membrane Neisseria meningitidis, or recombinant MIEP of Neisseria meningitidis produced in a recombinant host cell. Another object of the present invention is to provide a protein which possesses the ability to induce the production of cytokines such as I1-2, comprising either MIEP purified directly from the outer membrane of Neisseria meningitidis, or recombinant MIEP of Neisseria meningitidis produced in a recombinant host cell.

An additional object of the present invention is to provide vaccine compositions containing either the recombinant MIEP, or MIEP purified directly from the outer membrane of Neisseria meningitidis. These and other objects will be apparent from the following description.

## SUMMARY OF THE INVENTION

The present invention relates to the Class II major immuno enhancing protein (MIEP) of the outer membrane of Neisseria meningitidis, in substantially pure form, free from other contaminating N. meningitidis outer membrane proteins and LPS. The MIEP of the present invention, whether purified directly from the outer membrane of Neisseria menigitidis cells, or derived from a recombinant host cell producing recombinant MIEP of Neisseria meningitidis, possesses immunologic carrier cytokine (I1-2) inducing activity and mitogenic activity. The MIEP of the present invention, when coupled to an antigen, is capable of immune enhancement in that the antibody response to the coupled antigen is augmented or the antigen is transformed to a T-dependent antigen which ensures that immunoglobulins of the IgG class are produced. The antigens which may be coupled to the MIEP of the present invention include viral proteins, bacterial proteins and polysacharides, synthetic peptides, other immunogenic antigens, and weak or non-immunogenic antigens.

## BRIEF DESCRIPTION OF THE DRAWING

Figure 1 - Antibody responses of adoptive transfer recipients receiving spleen cells primed separately with PRP-DT and MIEP, or OMPC, or IAA-OMPC, were measured by ELISA and RIA in blood samples taken on the indicated days post-immunization with PRP-OMPC.

Figure 2 - Lymphocyte proliferation assay for mitogenic activity of MIEP, in vitro. The increase in $^3$H-thymidine incorporation into cellular DNA was measured following exposure of the cells to bovine serum albumin (BSA), PRP-OMPC, OMPC, MIEP, or CNBr.

Figure 3 - PRP-MIEP conjugates were tested for immunogenicity in mice as well as infant rhesus monkeys. Antiboby responses were measured by ELISA and RIA.

Figure 4 - Induction of I1-2 production by mouse spleen cells in culture following exposure to MIEP as an example of the induction of cytokine production. The results were obtained using samples from the three day culture timepoints.

## DETAILED DESCRIPTION OF THE INVENTION

It is known that certain substances which by themselves elicit an immune response which consists of only IgM class antibodies and no memory, can be transformed into fully immunogenic antigens which elicit IgM and IgG anitbodies as well as memory, by chemical coupling to a strongly (T-cell dependent) antigenic substance. This immunologic phenomenon is termed the "carrier effect", while the weak or non-immunogenic moiety, and the strongly antigenic substance are termed "hapten" and "carrier", respectively.

Injection of the hapten-carrier complex into an animal will result in the formation of antibodies by B-lymphocytes, some of which will be specific for, and bind to the hapten, and others which will be specific for, and bind to the carrier. An additional aspect of the carrier effect is that upon a subsequent exposure to the hapten-carrier complex, a vigourous antibody response to the hapten ensues. This is termed a memory, or anamnestic response.

The carrier effect appears to involve functions mediated by certain T-lymphocytes, called "helper T-lymphocytes". The carrier molecule stimulates the helper T-lymphocytes to assist, in some way, formation of anti-hapten IgG class antibody-producing B-lymphocytes and a memory response.

Helper T-lymphocytes are normally involved in the production by B-lymphocytes, of antibodies specific for a certain type of antigens, termed "T-dependent" antigens, but not for other antigens termed "T-independent" antigens. A carrier molecule can convert a T-independent, weak or non-immunogenic hapten into a T-dependent, strongly antigenic molecule. Furthermore, a memory response will follow a subsequent exposure to the hapten-carrier complex and will consist primarily of IgG, which is characteristic of T-dependent antigens and not T-independent antigens.

The utility of carrier molecules is not limited to use with T-independent antigens but can also be used with T-dependent antigens. The antibody response to a T-dependent antigen may be enhanced by coupling the antigen to a carrier, even if the antigen can, by itself, elicit an antibody response.

Certain other molecules have the ability to generally stimulate the overall immune system. These molecules are termed "mitogens" and include plant proteins as well as bacterial products. Mitogens cause T and/or B-lymphocytes to proliferate, and can broadly enhance many aspects of the immune response including increased phagocytosis, increased resistance to infection, augmented tumor-immunity, and increased antibody production.

The production of I1-2 by certain T-helper lymphocytes can cause the stimulation of growth and activity of other lymphocytes. I1-2 production by T-helper cells can be induced when the T cells are activated by certain substances or antigens presented by antigen presenting cells. The effects of I1-2 include, but are not limited to the progression to proliferation of T cells, and the proliferation and differentiation of B cells.

Many infectious disease causing agents can, by themselves, elicit protective antibodies which can bind to and kill, render harmless, or cause to be killed or rendered harmless, the disease causing agent and its byproducts. Recuperation from these diseases usually results in long lasting immunity by virtue of protective antibodies generated against the highly antigenic components of the infectious agent.

Protective antibodies are part of the natural defense mechanism of humans and many other animals, and are found in the blood as well as in other tissues and bodily fluids. It is the primary function of most vaccines to elicit protective antibodies against infectious agents and/or their byproducts, without causing disease.

OMPC from N. meningitidis has been used successfully to induce antibody responses in humans when OMPC is chemically coupled to T-cell independent antigens, including bacterial polysaccharides. OMPC contains several bacterial outer membrane proteins as well as bacterial lipids. In addition, OMPC has a vesicular three dimensional structure.

The efficacy of OMPC as an immunologic carrier was thought to depend on one or more of the bacterial membrane proteins, bacterial lipids, the vesicular three dimensional structure, or a combination of bacterial proteins, lipids, and vesicular structure. Applicants have discovered that one of the proteins, MIEP, possesses the immunologic carrier and immune enhancement properties of OMPC vesicles, and is effective in purified form, free from other N. memingitidis membrane proteins and lipids, and in a non-vesicular three dimensional structure. Applicants have also discovered that MIEP, when chemically coupled to bacterial polysaccharide, functions as well as OMPC in inducing an antibody response to the polysaccharide. Applicants have further discovered that MIEP is the Class II protein of the outer membrane of N. meningitidis. The Class II protein of N.

meningitidis is a porin protein [Murakami, K., et al., (1989), Infection And Immunity, 57, pp.2318-23]. Porins are found in the outer membrane of all Gram negative bacteria.

While the present invention is exemplified by MIEP of N. meningitidis, it is readily apparent to those skilled in the art that any outer membrane protein from any Gram negative bacterium, which has immunologic carrier and immune enhancement activity, is encompassed by the present invention. Examples of Gram negative bacteria include but are not limited to species of the genera Neisseria, Escherichia, Pseudomonas, Hemophilus, Salmonella, Shigella, Bordetella, Klebsiella, Serratia, Yersinia, Vibrio, and Enterobacter.

MIEP may be employed to potentiate the antibody response to highly antigenic, weakly antigenic, and non-antigenic materials. The term "antigen" and "antigenic material" which are used interchangeably herein include one or more non-viable, immunogenic, weakly immunognic, non-immunogenic, or desensitizing (antiallergic) agents of bacterial, viral, or other origin. The antigen component may consist of a dried powder, an aqueous phase such as an aqueous solution, or an aqueous suspension and the like, including mixtures of the same, containing a non-viable, immunogenic, weakly immunogenic, non-immunogenic, or desensitizing agent or agents.

The aqueous phase may conveniently be comprised of the antigenic material in a parenterally acceptable liquid. For example, the aqueous phase may be in the form of a vaccine in which the antigen is dissolved in a balanced salt solution, physiological saline solution, phosphate buffered saline solution, tissue culture fluids, or other media in which an organism may have been grown. The aqueous phase also may contain preservatives and/or substances conventionally incorporated in vaccine preparations. Adjuvant emulsions containing MIEP conjugated antigen may be prepared employing techniques well known to the art.

The antigen may be in the form of purified or partially purified antigen including but not limited to antigens derived from bacteria, viruses, mammalian cells, fungi, rickettsia; or the antigen may be an allergen including but not limited to pollens, dusts, danders, or extracts of the same; or the antigen may be in the form of a poison or a venom including but not limited to poisons or venoms derived from poisonous insects or reptiles. The antigen may also be in the form of a synthetic peptide, or a fragment of a larger polypeptide, or any subportion of a molecule or component derived from bacteria, mammalian cell, fungi, viruses, rickettsia, allergen, poison or venom. In all cases, the antigens will be in the form in which their toxic or virulent properties have been reduced or destroyed and which when introduced into a suitable host will either induce active immunity by the production therein of antibodies against the specific proteins, peptides, microorganisms, extract, or products of microorganisms used in the preparation of the antigen, poisons, venoms, or, in the case of allergens, they will aid in alleviating the symptoms of the allergy due to the specific allergen.

The antigens can be used either singly or in combination, for example, multiple bacterial antigens, multiple viral antigens, multiple mycoplasmal antigens, multiple rickettsial antigens, multiple bacterial or viral toxoids, multiple allergens, multiple proteins, multiple peptides or combinations of any of the foregoing products can be conjugated to MIEP.

Antigens of particular importance are derived from bacteria including but not limited to B. pertussis, Leptospira pomona, and icterohaemorrhagiae, S. paratyphi A and B, C. diphtheriae, C. tetani, C. botulinum, C. perfringens, C. feseri, and other gas gangrene bacteria, B. anthracis, P. pestis, P. multocida, V. cholerae, Nesseria meningitidis, N. gonorrheae, Hemophilus, influenzae, Treponema palidum, and the like; from mammalian cells including but not limited to tumor cells, virus infected cells, genetically engineered cells, cells grown in culture, cell or tissue extracts, and the like; from viruses including but not limited t human T lymphotropic virus (multiple types), human immunodeficiency virus (multiple variants and types), polio virus (multiple types), adeno virus (multiple types), parainfluenza virus (multiple types), measles, mumps, respiratory syncytial virus, influenza virus (various types), shipping fever virus ($SF_4$), Western and Eastern equine encephalomyelitis virus, Japanese B. encephalomyelitis, Russian Spring-Summer encephalomyelitis, hog cholera virus, Newcastle disease virus, fowl pox, rabies, feline and canine distemper and the like viruses, from rickettsiae including but not limited to epidemic and endemic typhus or other members of the spotted fever group, from various spider and snake venoms or any of the known allergens, including but not limited to those from ragweed, house dust, pollen extracts, grass pollens, and the like.

The polysaccharides of this invention may be any bacterial polysaccharides with acid groups, but are not intended to be limited to any particular types. Examples of such bacterial polysaccharides include Streptococcus pneumoniae (pneumococcal) types 6A, 6B, 10A, 11A, 18C, 19A, 19f, 20, 22F, and 23F, polysaccharides; Group B Streptococcus types Ia, Ib, II and III; Haemophilus influenzae serotype b polysaccharide; Neisseria meningitidis serogroups A, B, C, X, Y, W135 and 29E polysaccharides; and Escherichia coli K1, K12, K13, K92 and K100 polysaccharides. Particularly preferred polysaccharides, however, are those capsular polysaccharides selected from the group consisting of H. influenzae serotype b polysaccharides, such as described in Rosenberg et al., J. Biol. Chem., 236, 2845-2849 (1961) and Zamenhof et al., J. Biol. Chem., 203, 695-704 (1953). Streptococcus pneumoniae (pneumococcal) type 6B or type 6A polysaccharide, such as described in

Robbins et al., Infection and Immunity, 26, No. 3 1116-1122 (Dec., 1979); pnemococcal type 19F polysaccharide, such as described C. J. Lee et al., Reviews of Infectious Diseases, 3, No. 2, 323-331 (1981); and pneumococcal type 23F polysaccharide, such as described in O. Larm et al., Adv. Carbohyd Chem and Biochem., 33, 295-321, R. S. Tipson et al., ed., Academic Press 1976.

MIEP can be purified from OMPC derived from cultures of N. meningitidis grown in the usual manner as described in U.S. Patent number 4,459,286 and U.S. Patent number 4,830,852. OMPC purification can be done according to the methods described in U.S. Patent number 4,271,147, 4,459,286, and 4,830,852.

MIEP can also be obtained from recombinant DNA engineered host cells by expression of recombinant DNA encoding MIEP. The DNA encoding MIEP can be obtained from N. meningitidis cells [Murakami, K. et al., (1989), Infection And Immunity, 57, pp. 2318], or the DNA can be produced synthetically using standard DNA synthysis techniques. DNA encoding MIEP can be expressed in recombinant host cells including but not limited to bacteria, yeast, insect, mammalian or other animal cells, yielding recombinant MIEP. The preferred methods of the present invention for obtaining MIEP are purification of MIEP from OMPC and recombinant DNA expression of DNA encoding MIEP derived from N. meningitidis, with purification from OMPC most preferred.

Purified MIEP was prepared from OMPC vesicles by sodium dodecylsulfate (SDS) lysis of the vesicles followed by SDS polyacrylamide gel electrophoresis (PAGE). The MIEP was eluted from the gel, dialysed against a high pH buffer and concentrated. Standard methods of polyacrylamide gel electrophoresis can be utilized to purify MIEP from OMPC vesicles. Such methods are described in Molecular Cloning: A Laboratory Manual, Sambrook, J. et al., (1989), Cold Spring Harbor Laboratory Press, New York, and Current Protocols In Molecular Biology, (1987) Ausubel F.M. et al., editors, Wiley and Sons, New York.

Standard methods of eluting proteins from SDS-polyacrylamide gels are described in Hunkapiller, M.W., and Lujan, E., (1986), Purification Of Microgram Quantities Of Proteins By Polyacrylamide Gel Electrophoresis, in Methods of Protein Microcharacterization (J. Shively editor) Humanna Press, Clifton N.J., and Current Protocols In Molecular Biology (1987), Ausubel, F.M., et al., editors, Wiley and Sons, New York.

MIEP prepared in this manner is readily suitable for conjugation to antigens derived from bacteria, viruses, mammalian cells, rickettsia, allergens, poisons or venoms, fungi, peptides, proteins, polysaccharides, or any other antigen.

Recombinant MIEP can be prepared by expression of genomic N. meningitidis DNA encoding MIEP in bacteria, for example E. coli or in yeast, for example S. cerevisiae. To obtain genomic DNA encoding MIEP, genomic DNA is extracted from N. meningitidis and prepared for cloning by either random fragmentation of high molecular weight DNA following the technique of Maniatis, T. et al., (1978), Cell, 15, pp. 687, or by cleavage with a restriction endonuclease by the method of Smithies, et al., (1978), Science, 202, pp. 1248. The genomic DNA is then incorporated into an appropriate cloning vector, for example lambda phage [see Sambrook, J. et al., (1989), Molecular Cloning, A Laboratory Manual. Cold Spring Harbor Press, New York]. Alternatively, the polymerase chain reaction (PCR) technique (Perkin Elmer) can be used to amplify specific DNA sequences in the genomic DNA [Roux, et al., (1989), Biotechniques, 8, pp. 48]. PCR treatment requires a DNA oligonucleotide which can hybridize with specific DNA sequences in the genomic DNA. The DNA sequence of the DNA oligonucleotides which can hybridize to MIEP DNA in the N. meningitidis genomic DNA can be determined from the amino acid sequence of MIEP or by reference to the determined DNA sequence for the Class II major membrane protein of N. meningitidis [Musakami, k. et al., (1989), Infection and Immunity, 57, pp. 2318].

Recombinant MIEP can be separated from other cellular proteins by use of an affinity column made with monoclonal or polyclonal antibodies specific for MIEP. These affinity columns are made by adding the antibodies to Affigel-10 (Biorad), a gel support which is pre-activated with N-hydroxysuccinimide esters such that the antibodies form covalent linkages with the agarose gel bead support. The antibodies are then coupled to the gel via amide bonds with the spacer arm. The remaining activated esters are then quenched with 1M ethanolamine HCl (pH 8). The column is washed with water followed by 0.23 M glycine HCl (pH 2.6) to remove any non-conjugated antibody or extraneous protein. The column is then equilibrated in phosphate buffered saline (pH 7.3) and the cell culture supernatants or cell extracts containing MIEP are slowly passed through the column. The column is then washed with phosphate buffered saline until the optical density ($A_{280}$) falls to background, then the protein is eluted with 0.23 M glycine-HCl (pH 2.6). The protein is then dialyzed against phosphate buffered saline.

The conjugates of the present invention may be any stable polysaccharide-MIEP conjugates, or any other antigen-MIEP conjugates, including synthetic peptide antigens. The synthetic peptides may possess one or more antigenic determinants of any antigen including antigenic determinants from bacteria, rickettsia, viruses (including human immunodeficiency viruses), mammalian cells or other eukaryotic cells including parasites, toxins or poisons, or allergens. The antigen-MIEP conjugates are coupled through bigeneric spacers containing a thioether group and primary amine, which form hydrolytically-labile covalent bonds with the polysaccharide and the MIEP. Preferred conjugates according to this invention, however, are those which may be represented

by the formulae, Ps-A-E-S-B-Pro or Ps-A′-S-E′-B′-Pro, wherein Ps represents a poly-saccharide or any other antigen; Pro represents the bacterial protein MIEP; and A-E-S-B and A′-S-E′-B′ constitute bigeneric spacers which contain hydrolytically-stable covalent thioether bonds, and which form covalent bonds (such as hydrolytically-labile ester or amide bonds) with the macromolecules, Pro and Ps. In the spacer, A-E-S-B, S is sulfur; E is the transformation product of a thiophilic group which has been reacted with a thiol group, and is represented by

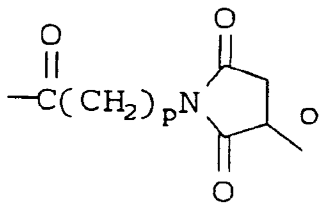 or 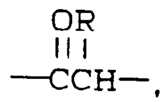

wherein R is H or $CH_3$, and p is 1 to 3; A is

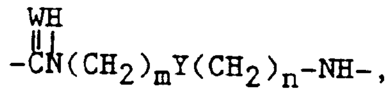

wherein W is O or NH, m is O to 4, n is O to 3, and Y is $CH_2$,O,S,NR′, or $CHCO_2H$, where R′ is H or $C_1$- or $C_2$-alkyl, such that if Y is $CH_2$, then both m and n cannot equal zero, and if Y is O or S, then m is greater than 1 and n is greater than 1; and B is

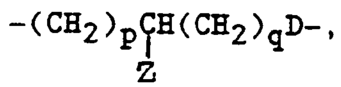

wherein q is O to 2, Z is $NH_2$,

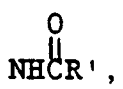

COOH, or H, where R′ and p are as defined above, and D is

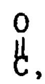

NR′,

or 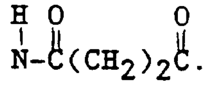

Then in the spacer, A′-S-E′-B′, S is sulfur; A′ is

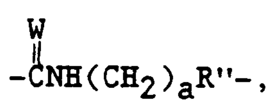

wherein a is 1 to 4, and R″ is CH2, or

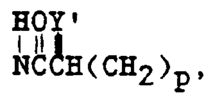

where Y′ is $NH_2$ or NHCOR′, and W, p and R′ are as defined above, and E′ is the transformation product of a thiophilic group which has been reacted with a thiol group, and is represented by

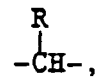

wherein R is as defined above, and B′ is

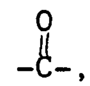

or E′ is

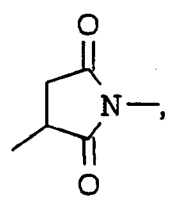

B′ is

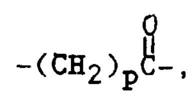

wherein p is 1 to 3. Further, of the bigeneric spacers, A-E-S-B and A′-S-E′-B′, the E-S-B and A′-S-E′ components are determinable and quantifiable, with this identification reflecting the covalency of the conjugate bond linking the side of the thioethersulfur which originates from the covalently-modified polysaccharide with the side of the spacer which originates from the functionalized protein.

Then the conjugates, Ps-A-E-S-B-Pro, according to this invention may contain spacers whose components include derivatives of, inter alia: carbon dioxide, 1,4-butanediamine, and S-carboxymethyl-N-acetylhomocysteine; carbon dioxide, 1,5-pentanediamine, and S-carboxymethyl-N-acetylhomocysteine; carbon dioxide, 3-oxa-1,5-pentanediamine, and S-carboxymethyl-N-acetylhomocysteine; carbon dioxide, 1,4-butane-diamine, and S-carboxymethyl-N-acetylcysteine; carbon dioxide, 1,3-propanediamine, and S-carboxymethyl-N-benzoylhomocysteine; carbon dioxide, 3-aza-1,5-pentanediamine, and S-carboxymethyl-N-acetylcysteine; and carbon dioxide, 1,2-ethanediamine, glycine, and S-(succin-2-yl)-N-acetylhomocysteine. The conjugates, Ps-A′-S-E′-B′-Pro, according to this invention, may contain spacers whose components include derivatives of, inter alia: carbon dioxide and S-carboxymethylcysteamine; carbon dioxide and S-(α-carboxyethyl)cysteamine; carbon dioxide and S-carboxymethylhomocysteamine; carbon dioxide, S-(succin-2-yl)cysteamine, and glycine; and carbon dioxide and S-carboxymethylcysteine.

In the process of the present invention, the polysaccharide is covalently-modified by (a) solubilizing it in a non-hydroxylic organic solvent, then (b) activating it with a bifunctional reagent, (c) reacting this activated polysaccharide with a bis-nucleophile, and finally, if necessary, further (d) functionalizing this modified polysaccharide by either reaction, (i) with a reagent generating electrophilic (e.g., thiolphilic) sites or, (ii) with a reagent generating thiol groups. The protein is conversely either reacted (i) with a reagent generating thiol groups or (ii) with a reagent generating thiolphilic sites, then the covalently-modified polysaccharide and the functionalized protein are reacted together to form the stable covalently-bonded conjugate and the final mixture is purified to remove unreacted polysaccharides and proteins.

The process of this invention also includes selection of a nucleophile or bis-nucleophile which will react with the activated polysaccharide to form a covalently-modified polysaccharide with pendant electrophilic sites or pendant thiol groups, thereby obviating the need to further functionalize the bis-nucleophile-modified polysaccharide prior to reacting the covalently-modified polysaccharide with the covalently-modified protein. Also, the functionalization of the protein to either moiety form may be accomplished in more than one step according to the selection of reactants in these steps.

In the first step toward covalently-modifying the polysaccharide, the solid polysaccharide must be solubilized.

Since the nucleophilic alcoholic hydroxyl groups of a polysaccharide cannot compete chemically for electrophilic reagents with the hydroxyls of water in an aqueous solution, the polysaccharide should be dissolved in non-aqueous (non-hydroxylic) solvents. Suitable solvents include dimethylformamide, dimethylsulfoxide, dimethylacetamide, formamide, N,N′-dimethylimidazolidinone, and other similar polar, aprotic solvents, preferably dimethylformamide.

In addition to the use of these solvents, converting the polysaccharides (e.g., the capsular polysaccharides of H. influenzae type b, which are a ribose-ribitol phosphate polymers), which have acid hydrogens, such as phosphoric acid mono- and diesters, into an appropriate salt form, causes the polysaccharides to become readily soluble in the above solvents. The acidic hydrogens in these macro-molecules may be replaced by large hydrophobic cations, such as tri- or tetra-($C_1$- to $C_5$)alkyl-ammonium, 1-azabicyclo[2.2.2]octane,1,8-diazabicyclo [5.4.0]undec-7-ene or similar cations, particularly tri- or tetra-($C_1$- to $C_5$)alkylammonium, and the resultant tri- or tetraalkylammonium or similar salts of phosphorylated polysaccharides readily dissolve in the above solvents at about 17°-50°C, while being stirred for from one minute to one hour.

Partially-hydrolyzed H. influenzae serotype B polysaccharide has been converted into the tetrabutyl-ammonium salt, then dissolved in dimethylsulfoxide (Egan et al., J. Amer Chem. Soc., 104, 2898 (1982)), but this product is no longer antigenic, and therefore useless for preparing vaccines. By contrast, Applicants accomplish the solubilization of an intact, unhydrolyzed polysaccharide by passing the polysaccharide through a strong acid cation exchange resin, in the tetraalkylammonium form, or by careful neutralization of the polysaccharide with tetraalkyl-ammonium hydroxide, preferably by the former procedure, and thereby preserve the viability of the polysaccharide for immunogenic vaccine use.

Subsequent steps are then directed to overcoming the other significant physico-chemical limitation to making covalent bonds to polysaccharides, that being the lack of functional groups on the polysaccharides, other than hydroxyl groups, which are reactive enough with reagents commonly or practically used for functionalization of units with which bonding is desired. Activation of the polysaccharide to form an activated polysaccharide, reaction with bis-nucleophiles to form a nucleophile-functionalized polysaccharide, and functionalization with reagents generating either electrophilic sites or thiol groups, are all directed to covalently-modifying the polysaccharide and developing functional groups on the polysaccharide in preparation for conjugation.

In the next step, the solubilized polysaccharide is activated by reaction with a bifunctional reagent at about 0°-50°C, while stirring for ten minutes to one hour, with the crucial weight ratio of activating agent to polysaccharide in the range of 1:5 to 1:12. In the past, this activation has been accomplished by reaction of the polysaccharide with cyanogen bromide. However, derivatives activated with cyanogen bromide, which has a "proclivity" for vicinal diols, have shown transient stability during dialysis against a phosphate buffer. Therefore, while activation with cyanogen bromide is still possible according to the present invention, this reagent is poorly utilized in activation of polysaccharides and is not preferred. Instead, preferred bifunctional reagents for activating the polysaccharide include carbonic acid derivatives,

$$R^2-\overset{\overset{O}{\|}}{C}-R^3,$$

wherein $R^2$ and $R^3$ may be independently, azolyl, such as imidazolyl; halides; or phenyl esters, such as p-nitrophenyl, or polyhalophenyl.

Carbonyldiimidazole, a particularly preferred reagent, will react with the hydroxyl groups to form imidazolylurethanes of the polysaccharide, and arylchloroformates, including, for example, nitrophenylchloroformate, will produce mixed carbonates of the polysaccharide. In each case, the resulting activated polysaccharide is very susceptible to nucleophilic reagents, such as amines, and is thereby transformed into the respective urethanes.

In the next stage, the activated polysaccharide is reacted with a nucleophilic reagent, such as an amine, particularly diamines, for example,

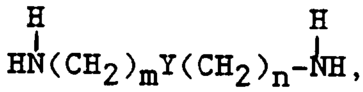

wherein m is O to 4, n is O to 3, and Y is $CH_2$, O, S, NR′, $CHCO_2H$, where R′ is H or a $C_1$- or $C_2$-alkyl, such that if Y is $CH_2$, then both m and n cannot equal zero, and if Y is O or S, then m is greater than 1, and n is greater than 1, in a gross excess of amine (i.e., for example, a 50-to 100-fold molar excess of amine vs. activating agent used). The reaction is kept in an ice bath for from 15 minutes to one hour then kept for 15 minutes to one hour at about 17° to 40°C.

An activated polysaccharide, when reacted with a diamine, e.g., 1,4-butanediamine, would result in a urethane-form polysaccharide with pendant amines, which may then be further functionalized by acylating. Mixed carbonates will also readily react with diamines to result in pendant amine groups.

Alternatively, the activated polysaccharide may be reacted with a nucleophile, such as a monohaloacetamide of a diaminoalkane, for example, 4-bromoacetamidobutylamine (see, W. B. Lawson et al., Hoppe Seyler's Z. Physiol Chem., 349, 251 (1968)), to generate a covalently-modified polysaccharide with pendant electrophilic sites. Or, the activated polysaccharide may be reacted with an aminothiol, such as cysteamine (aminoethanethiol) or cysteine, examples of derivatives of which are well-known in the art of peptide synthesis, to produce a polysaccharide with pendant thiol groups. In both cases, no additional functionalization is necessary prior to coupling the covalently-modified polysaccharide to the modified bacterial "carrier" protein.

The last step in preparing the polysaccharide, the further functionalization, if necessary, of the polysaccharide, may take the form of either reacting the nucleophile-functionalized polysaccharide with a reagent to generate electrophilic (i.e., thiophilic) sites, or with a reagent to generate thiol groups.

Reagents suitable for use in generating electophilic sites, include for example, those for acylating to α-haloacetyl or α-halopropionyl, derivative such as

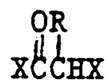

(wherein R is H or $CH_3$; X is Cl, Br or I; and X′ is nitrophenoxy, dinitrophenoxy, pentachlorophenoxy, pentafluorophenoxy, halide, O-(N-hydroxysuccinimidyl) or azido), particularly chloroacetic acid or α-bromopropionic acid, with the reaction being run at a pH of 8 to 11 (maintained in this range by the addition of base, if necessary) and at a temperature of about 0° to 35°C, for ten minutes to one hour. An amino-derivatized polysaccharide may be acylated with activated maleimido amino acids (see, 0. Keller et al, Helv. Chim. Acta.,58, 531 (1975)) to produce maleimido groups,

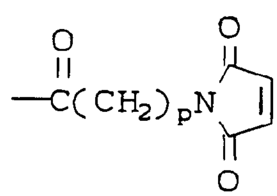

wherein p is 1 to 3; with a 2-haloacetyling agent, such as p-nitrophenylbromoacetate; or with an α-haloketone carboxylic acid derivative, e.g.,

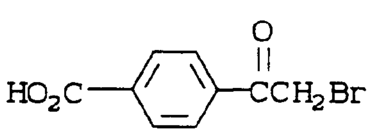

(Ber., 67, 1204, (1934)) in order to produce appropriately functionalized polysaccharides susceptible to thiosubstitution.

Reagents suitable for use in generating thiol groups include, for example, acylating reagents, such as thiolactones, e.g.,

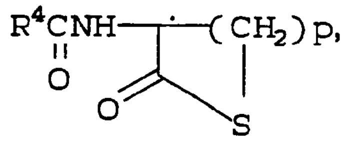

wherein $R^4$ is $C_1$- to $C_4$-alkyl or mono- or bicyclic aryl, such as $C_6H_5$ or $C_{10}H_{13}$, and p is 1 to 3;

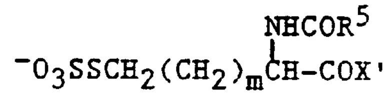,

wherein m is 0 to 4, $R^5$ is $C_1$-to $C_4$-alkyl or $C_6H_5$, and X′ is as defined above, followed by treatment with $HSCH_2CH_2OH$; or

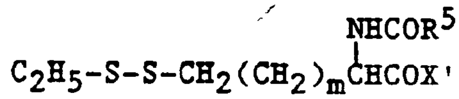,

wherein m, $R^5$ and X′ are as defined immediately above, then treatment with dithiothreitol. Such reactions are carried out in a nitrogen atmosphere, at about 0° to 35°C and at a pH of 8 to 11 (with base added, as necessary, to keep th pH within this range), for one to twenty-four hours. For example, an amino-derivatized polysaccharide may be reacted with

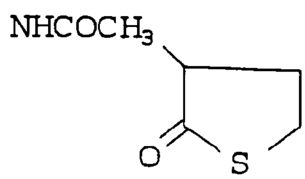

to produce an appropriately-functionalized polysaccharide.

By these steps then, covalently-modified polysaccharides of the forms, Ps-A-E*- or Ps-A′-SH-, wherein E* is -CCHX or

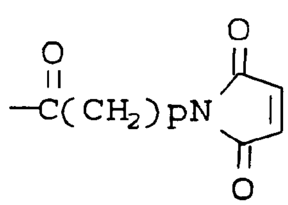

and A, A′, R, X and p are as defined above, are produced.

Separate functionalization of the protein to be coupled to the polysaccharide, involves reaction of the protein with one or more reagents to generate a thiol group, or reaction of the protein with one or more reagents to generate an electrophilic (i.e., thiophilic) center.

In preparation for conjugation with an electrophilic-functionalized polysaccharide, the protein is reacted in one or two steps with one or more reagents to generate thiol groups, such as those acylating reagents used for generating thiol groups on polysaccharides, as discussed on pages 15-17 above. Thiolated proteins may also be prepared by aminating carboxy-activated proteins, such as those shown in Atassi et al., Biochem et Biophys Acta, 670, 300, (1981), with aminothiols, to create the thiolated protein. A preferred embodiment of this process step involves the direct acylation of the pendant amino groups (i.e., lysyl groups) of the protein with N-acetylhomocysteinethiolactone at about 0° to 35°C and pH 8-11, for from five minutes to two hours, using equiweights of reactants.

When E′B′ is

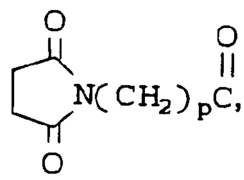

the conditions and method of preparing the functionalized protein are as discussed above for preparing the counterpart polysaccharide by reaction with activated maleimido acids.

In preparing for conjugation with a covalently-modified bacterial polysaccharide with pendant thiol groups, the protein is acylated with a reagent generating an electrophilic center, such acylating agents including, for example,

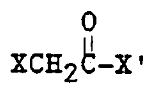

and

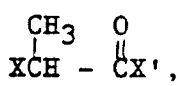

wherein X and X′ are as defined above; and

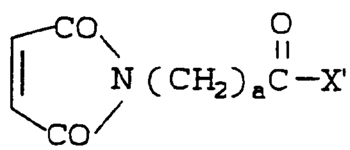

,

wherein X′ is as defined above. Suitable proteins with electophilic centers also include, for example, those prepared by acylation of the pendant lysyl amino groups with a reagent, such as activated maleimido acids, for example,

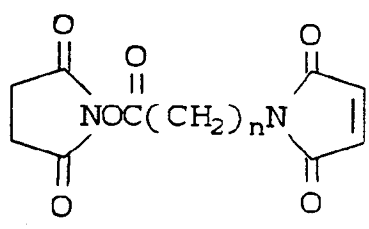

or by reacting the carboxy-activated protein with monohaloacetyl derivatives of diamines. In both preparation reactions, the temperature is from 0° to 35°C for from five minutes to one hour and the pH is from 8 to 11.

Formation of the conjugate is then merely a matter of reacting any of the covalently-modified polysaccharides having pendant electrophilic centers with of the bacterial protein MIEP having pendant thiol groups at a pH of 7 to 9, in approximate equiweight ratios, in a nitrogen atmosphere, for from six to twenty-four hours at from about 17° to 40°C, to give a covalent conjugate. Examples of such reactions include:

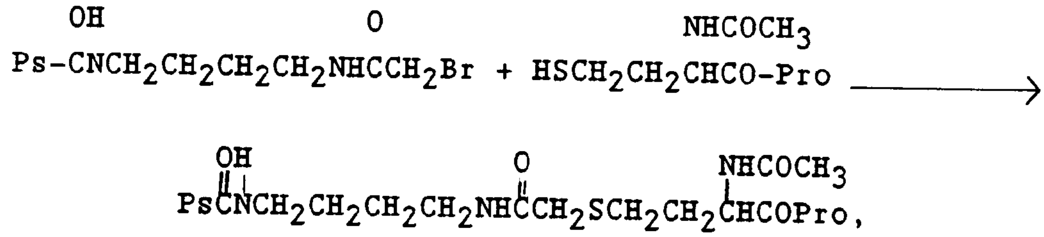

wherein an activated polysaccharide which has been reacted with 4-bromoacetamidobutylamine is reacted with a protein which has been reacted with N-acetyl-homocysteinethiolactone, to form a conjugate, and:

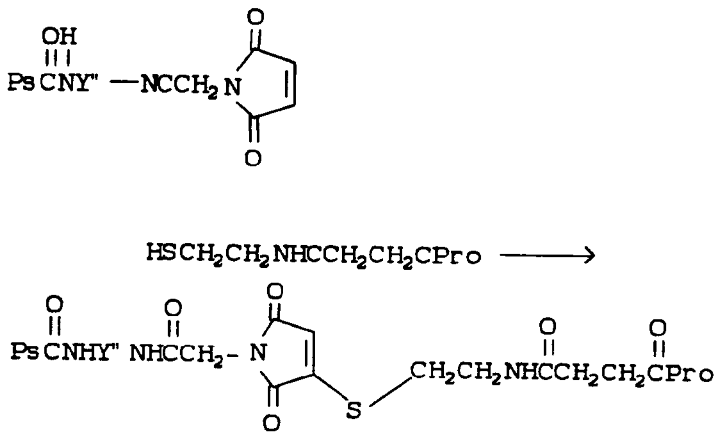

(where Y″ is a $C_2$-$C_8$alkyl radical), wherein an amino-derivatized polysaccharide which has been reacted with activated maleimido acids is reacted with a carboxy-activated protein which has been aminated with an aminothiol, to form a conjugate.

Similarly, any of the covalently-modified polysaccharides with pendant thiol groups may be reacted with the bacterial protein MIEP having pendant electrophilic centers to give a covalent conjugate. An example of such a reaction is:

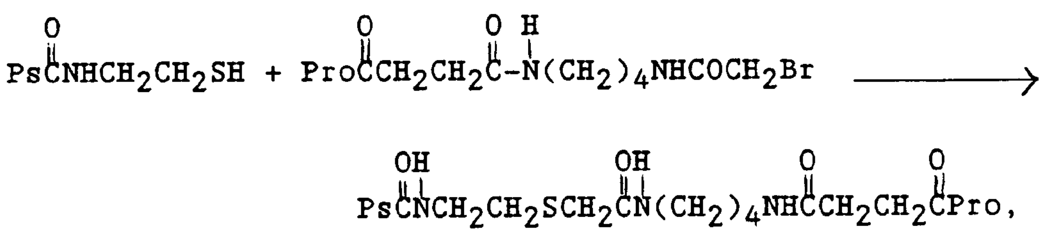

wherein an activated polysaccharide which has been reacted with an aminothiol is reacted with a carboxy-activated protein which has been reacted with monohaloacetyl derivatives of a diamine, to form a conjugate.

Should the electrophilic activity of an excess of haloacetyl groups need to be eliminated, reaction of the conjugate with a low molecular weight thiol, such as n-acetylcysteamine, will accomplish this purpose. Use of this reagent, n-acetylcysteamine, also allows confirmation accounting of the haloacetyl moieties used (see Section D), because the S-carboxymethylcysteamine which is formed may be uniquely detected by the method of Spackman, Moore and Stein.

These conjugates are then centrifuged at about 100,000 x g using a fixed angle rotor for about two hours at about 1° to 20°C, or are submitted to any of a variety of other purification procedures, including gel permeation, ion exclusion chromatography, gradient centrifugation, or other differential adsorption chromatography, to remove non-covalently-bonded polysaccharides and proteins, using the covalency assay for the bigeneric spacer (see below) as a method of following the desired biological activity.

The further separation of reagents may be accomplished by size-exclusion chromatography in a column, or in the case of very large, non-soluble proteins, separation may be accomplished by ultracentrifugation.

Analysis of the conjugate to confirm the covalency, and hence the stability of the conjugate, is accomplished

by hydrolyzing (preferably with 6N HCl at 110°C for 20 hours) the conjugate, then quantitatively analyzing for the amino acid of the hydrolytically-stable spacer containing the thioether bond and constituent amino acids of the protein. The contribution of the amino acids of the protein may be removed, if necessary, by comparison with the appropriate amino acid standard for the protein involved, with the remaining amino acid value reflecting the covalency of the conjugate, or the amino acid of the spacer may be designed to appear outside the amino acid standard of the protein in the analysis. The covalency assay is also useful to monitor purification procedures to mark the enhancement of concentration of the biologicallyactive components. In the above examples, hydrolysis of

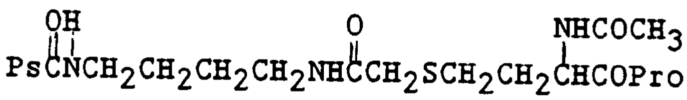

results in the release of S-carboxymethylhomocysteine,

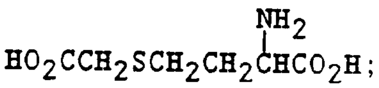

hydrolysis of

In mice this requirement exists for secondary, as well as primary, antibody responses and is carrier-specific, i.e. a secondary antibody response occurs only if the T helper cells have previously been sensitized with the carrier protein used for the secondary immunization. Therefore, the ability of a mouse to make a secondary antibody response to a PRP-protein conjugate is dependent on the presence of primed T lymphocytes with specificity for the carrier protein.

Demonstration of the ability of MIEP to provide carrier priming for anti-PRP antibody responses was done in mice adoptively primed with PRP covalently linked to a heterologous carrier, diphtheria toxoid (DT). Adoptive transfer was used in order to determine whether the administration of lymphocytes primed with MIEP alone was sufficient to generate effective helper-T cell activity for anti-PRP antibody formation in response to PRP-OMPC. Comparable secondary anti-PRP antibody responses were elicited by PRP-OMPC when lymphocytes primed with MIEP or OMPC were transferred, indicating that the T cell recognition of OMPC resides in the MIEP moiety.

PRP-MIEP conjugates were tested for immunogenicity in mice as well as infant Rhesus monkeys. The immune response in both of these animal models share, with infant humans, a dificiency in their ability to generate antibody responses against T-independent antigens such as bacterial polysaccharides. These animals are commonly used as models for assessment of the immune response of infant humans to various antigens.

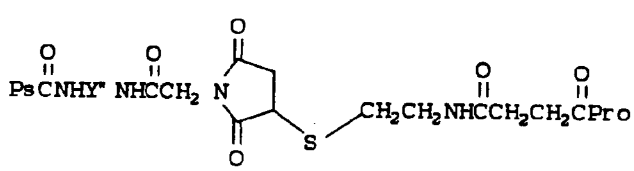

results in the release of the aminodicarboxylic acid,

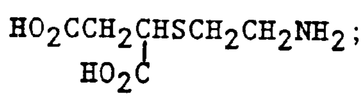

and hydrolysis of

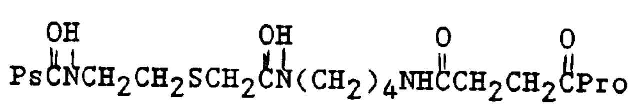

results in the release of S-carboxymethylcysteamine, $H_2NCH_2CH_2SCH_2CO_2H$ by cleavage of the Ps-A-E-S-B-Pro molecule at peptide linkages and other hydrolytically-unstable bonds. Chromatographic methods, such as

those of Spackman, Moore, and Stein, may then be conveniently applied and the ratio of amino acid constituents determined.

Optimal production of IgG antibody requires collaboration of B and T lymphocytes with specificity for the aritigen of interest. T lymphocytes are incapable of recognizing polysaccharides but can provide help for anti-polysaccharide IgG antibody responses if the polysaccharide is covalently linked to a protein which the T cell is capable of recognizing.

Likewise, MIEP-peptide conjugates, for example where the peptide is an HIV principal neutralizing determinant (PND) peptide, may be prepared. One method of making such conjugates includes the formation of a bigeneric spacer between activated MIEP and activated HIV PND peptides. The linker may include a polysaccharide moiety.

The novel conjugate of this invention comprises MIEP, the major immuno enhancing protien of the outer membrane protein complex (OMPC) of Neisseria meningitidis b, covalently linked to HIV PND peptides.

The conjugates are prepared by the process of covalently coupling activated peptide to an activated protein. The peptide and protein components are separately activated to display either pendant electrophilic or nucleophilic groups so that covalent bonds will form between the peptide and the protein upon contact.

The covalent conjugate immunogens that result from the series of reactions described above may conveniently be thought of as a conjugate in which multiple peptide functionalities are built upon a foundation of MIEP.

When the peptide components of the conjugate are capable of eliciting HIV neutralizing immune responses, the conjugates of this invention may be administred to mammals in immunologically effective amounts, with or without additional immunomodulatory, antiviral, or antibacterial compounds, and are useful for inducing mammalian immune responses against the peptidyl portion of the conjugates, for inducing HIV-neutralizing antibodies in mammals, or for making vaccines for administration to humans to prevent contraction of HIV infection or disease including AIDS, or for administration to humans afflicted with HIV infection or disease including AIDS.

In a preferred embodiment, the conjugate of the invention has the general structure:

$$_{j}(\text{PEP-A-})\text{-MIEP}$$

or pharmaceutically acceptable salts thereof, wherein:

PEP is an HIV PND peptide, or a peptide capable of raising mammalian immune responses which recognize HIV PNDs;

MIEP is an immunogenic protein of the outer membrane protein complex (OMPC) of Neisseria meningitidis b either recombinatly produced or purfied from OMPC;

-A- is a covalent linkage, preferably a bigeneric spacer;

j is the percentage by mass of peptide in the coconjugate, and is preferably between 1% and 50% of the total protein mass in the conjugate.

The conjugate of the invention may be prepared by any of the common methods known in the art for preparation of peptide-protien conjugates, such as, for example, the bigeneric chemistry disclosed in U.S. patent 4,695,624 and Marburg et al. J.A.C.S. 108, 5282 (1986), and in Applications USSN 362,179; 55,558; 555,974; 555,966 and 555,339. In a preferred embodiment, a process that utilizes the available nucleophilic functionalities, found in proteins, such as the amino group of lysine, the imidazole group of histidine, or the hydroxyl groups of serine, threonine, or tyrosine is used. In practical terms, the number of available protien necleophilic sites may be determined by an appropriate assay which may comprise thiolation with N-acetyl homocysteine thiolactone, followed by Ellman Assay [Ellman, G.L., Arch Biochem Biophys., 82, 70 (1959)] for determination of total free sulfydryl groups and/or by alkylation with a bromoacetyl amino acid, assayable by amino acid analysis.

The preferred process can be carried out in several ways in which the sequence, method of activation, and reaction of protein and peptide groups can be varied. The process may comprise the steps of:

Process 1:

1a. reacting the protein nucleophilic groups with a reagent, for example with N-acetyl homocysteine thiolactone, which generates thiol groups on the protein; and

1b. reacting the product of step 1a. with peptides previously derivatized so as to append an electrophilic group preferably comprising moleimide, on the peptide. A preferred embodiment of this invention, which may be prepared according to this process, has the structure:

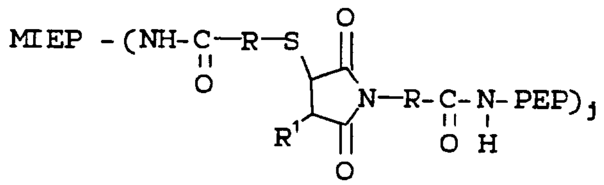

or pharmaceutically acceptable salts thereof, wherein:
PEP, MIEP, and j, are as defined supra;
-R- is:
a) -lower alkyl-,
b) -substituted lower alky-,
c) -cycloalkyl-,
d) -substituted cyloalkyl-,
e) -phenyl-;
$-R^1$ is:
a) -hydrogen,
b) -lower alkyl, or
c) $-SO_3H$; and
-S- is sulfur

Likewise, a preferred embodiment of the invention having the structure:

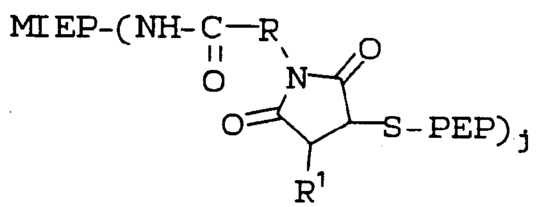

wherein all variables are as defined above, may be prepared by process 2, which comprises the steps of:

2a. reacting the protein nucleophilic groups with a bifunctional electrophilic reagent, such as maleimidoalkanoic acid hydroxysuccinimide ester, so as to generate an electrophilic protein; and

2b. reacting the product of step 2a. with a peptide containing a nucleophile, such as a thiol group.

A highly preferred embodiment of process 1, is described in detail below and in Scheme A. According to the scheme, the immunogenic protein is the class II protein of the outer membrane protein complex (OMPC) of Neisseria meningitidis b, either purified from the bacterial membrane or produced by recombinant means. The process comprises the steps of:

a.i. reacting MIEP (I), having nucleophilic groups, including free amino groups due to the presence of lysines or protein amino-termini, with a thiolating agent, preferably N-acetyl homocysteine thiolactone, to generate MIEP (II) having "m" moles of sulfhydryl groups available for reaction with a thiophile; a.ii. quantitating the number of available sulfhydryls appended to MIEP in step 1a.i. to determine the value of "m", preferably by Ellman assay [Ellman, G.L., Arch. Biochem. Biochem. Biophys., 82, 70 (1959)]; and b. contacting the product of step a. with an excess, (≥m), of an HIV PND which has been previously derivatized so as to append an electrophilic group, preferably with a maleimido-alkanoic acid, and most preferably with maleimido-propionic acid (this derivatization is achieved by N-protecting all amino groups on the peptide that should not be derivatized, and reacting the free peptide amino groups with a bifunctional reagent, preferably maleimidoalkanoyloxysuccinimide, and most preferably maleimidopropionyloxysuccinimide), to generate the conjugate of this invention (III).

The conjugate product may be purified by, for example, dialysis in a buffer having an ionic strength between 0.001M and 1M and a pH between 4 and 11, and most preferably in an aqueous medium having an ionic strength of between 0.01 and 0.1M and a pH of between 6 and 10.

SCHEME A

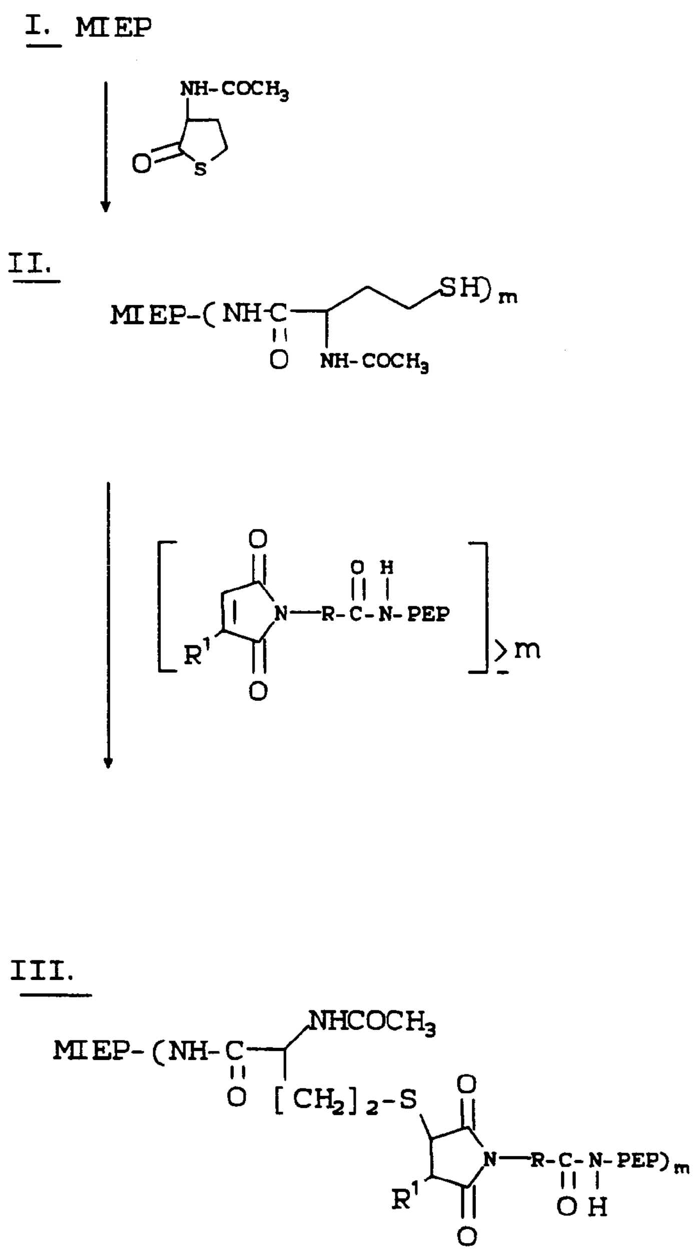

The process described above and depicted in Scheme A may be modified so that MIEP is derivatized so as to be covalently linked to a thiophile, such as a derivative of maleimide, while the peptide is activated so as to be covalently linked to free sulfhydryls. This and other alternate processes, naturally fall within the scope of this disclosure, including variations on these processes, such as variations of sequence of reaction of activated species, or ratios of reactants.

The process for making the conjugates of this invention may be applied to making any conjugate wherein a peptide-protein conjugate is desired and is particularly significant where enhanced immunogenicity of the peptide is required.

The conjugates herein described may be included in compositions containing an inert carrier and are useful when appropriately formulated as a vaccine. This may include prior adsorption onto alum or combination with emulsifiers or adjuvants known in the art of vaccine formulation. Methods of using the covalent conjugate immunogens of this invention include: (a) use as a laboratory tool to characterize HIV PND peptide structure-function relationships; (b) use as an immunogen to raise HIV-neutralizing antibodies in a mammal which antibodies may be isolated and administered to a human so as to prevent infection by HIV, or to limit HIV proliferation post-infection, or to treat humans afflicted by HIV infection or disease including AIDS. (c) use as a vaccine to immunize humans against infection by HIV or to treat humans post-infection, or to boost an HIV-neutralizing immune response in a human afflicted with HIV infection or disease including AIDs.

As a laboratory tool, the conjugate is useful when administered to a mammal in an immunologically effective amount, to generate anti-PND peptide, anti-HIV, or HIV-neutralizing immune responses. The mammal may be boosted with additional conjugate to elevate the immune response. Antiserum is obtained from such a mammal by bleeding the mammal, centrifuging the blood to separate the cellular component from the serum, and isolating antibody proteins from the serum if necessary, according to methods known in the art. Such antiserum or antibody preparations may be used to characterize the efficacy of an HIV PND peptide in a conjugate in raising mammalian anti-PND peptide, anti-HIV, or HIV-neutralizing antibodies in a mammal. ELISA assays using the unconjugated peptide and the antiserum are useful in vitro assays for measuring the elicitation of anti-peptide antibodies. An in vitro assay for measuring the HIV-neutralizing ability of antiserum comprises incubating a preparation of live HIV with a preparation of the antiserum, then incubating the antiserum-treated HIV preparation with CD4 receptor bearing cells, and measuring the extent of cellular protection afforded by the antiserum. These assays and the characteristics of antiserum produced by a given conjugate may be used to study the PND peptide stucture-function relationship.

The conjugate is useful for inducing mammalian antibody responses as described in the previous paragraph, and such antibodies may be used to passively immunize humans to prevent HIV infection, or to limit HIV proliferation post-infection, or to treat humans afflicted with HIV infection or disease including AIDS.

The conjugate is useful as a vaccine which may be administered to humans to prevent HIV infection or proliferation, or to humans suffering from HIV disease of HIV infection, including AIDS and related complexes, or to humans testing seropositive for the HIV virus. The conjugate may be administered in conjunction with other anti-HIV compounds, such as AZT, or more general anti-viral compounds, or in conjunction with other vaccines, antibiotics, or immunomodulators (see Table I below).

The form of the immunogen within the vaccine takes various molecular configurations. A single molecular species of the antigenic conjugate III will often suffice as a useful and suitable antigen for the prevention or treatment of HIV disease including AIDS or ARC. Other antigens in the form of cocktails are also advantageous, and consist of a mixture of conjugates that differ by, for example, the mass ratio of peptide to total protein. In addition, the conjugates in a mixture may differ in the amino acid sequence of the PND.

An immunological vector, carrier or adjuvant may be added as an immunological vehicle according to conventional immunological testing or practice.

Adjuvants may or may not be added during the preparation of the vaccines of this invention. Alum is the typical and preferred adjuvant in human vaccines, especially in the form of a thixotropic, viscous, and homogeneous aluminum hydroxide gel. For example, one embodiment of the present invention is the prophylactic vaccination of patients with a suspension of alum adjuvant as vehicle and a cocktail of conjugates as the selected set of immunogens or antigens.

The vaccines of this invention may be effectively administered, whether at periods of pre-exposure or post-exposure, in combination with effective amounts of the AIDS antivirals, immuno-modulators, antibiotics, or vaccines of Table I [source: Market Letter, Nov. 30, 1987, p. 26-27; Genetic Engineering News, Jan. 1988, Vol. 8, p. 23.]

TABLE I[1]

A. Antivirals

| Drug Name | Manufacturer | Indication |
|---|---|---|
| AL-721 | Ethigen | ARC, PGL |
| BETASERON (interferon beta) | Triton Biosciences | AIDS, ARC, KS |
| CARRISYN (polymannoacetate) | Carrington Labs | ARC |
| CYTOVENE (ganciclovir) | Syntex | CMV |
| DDC (dideoxycytidine) | Hoffmann-La Roche | AIDS, ARC |
| FOSCARNET (trisodium phosphonoformate) | Astra AB | HIV inf, CMV retinitis |
| HPA-23 | Rhone-Poulenc Sante | HIV infection |

---

[1]Abbreviations: AIDS (Acquired Immune Deficiency Syndrome); ARC (AIDS related complex); CMV (Cytomegalovirus, which causes an opportunistic infection resulting in blindness or death in AIDS patients); HIV (Human Immunodeficiency Virus, previously known as LAV, HTLV-III or ARV); KS (Kaposi's sarcoma); PCP (Pneumonocystis carinii pneumonia, an opportunistic infection); PGL (persistent generalized lymphadenopathy).

| Drug Name | Manufacturer | Indication |
| --- | --- | --- |
| ORNIDYL (eflornithine) | Merrell Dow | PCP |
| PEPTIDE T (octapeptide sequence) | Peninsula Labs | AIDS |
| RETICULOSE (nucleophospho-protein) | Advanced Viral Research | AIDS, ARC |
| IR (zidovudine; AZT) | Burroughs Wellcome | AIDS, advanced ARC pediatric AIDS, KS, asympt HIV, less severe HIV, neurological involvement. |

| | | |
|---|---|---|
| RIFABUTIN (ansamycin LM 427) | Adria Labs | ARC |
| (trimetrexate) | Warner-Lambert | PCP |
| UA001 | Ueno Fine Chem Industry | AIDS, ARC |
| VIRAZOLE (ribavirin) | Viratek/ICN | AIDS, ARC, KS |
| WELLFERON (alfa interferon) | Burroughs Wellcome | KS, HIV, in comb with RETROVIR |
| ZOVIRAX (acyclovir) | Burroughs Wellcome | AIDS, ARC, in comb with RETROVIR |

B. Immunomodulators

| Drug Name | Manufacturer | Indication |
|---|---|---|
| ABPP (bropirimine) | Upjohn | Advanced AIDS, KS |
| AMPLIGEN (mismatched RNA) | DuPont HEM Research | ARC, PGL |
| (Anti-human alpha interferon antibody) | Advanced Biotherapy Concepts | AIDS, ARC, KS |

| | | |
|---|---|---|
| Colony Stimulating Factor (GM-CSF) | Sandoz Genetics Institute | AIDS, ARC, HIV, KS |
| CL246,738 (CL246,738) | American Cynamid | AIDS |
| IMREG-1 | Imreg | AIDS, ARC, PGL, KS |
| IMREG-2 | Imreg | AIDS, ARC, PGL, KS |
| IMUTHIOL (diethyl dithio carbamate) | Merieux Institute | AIDS, ARC |
| IL-2 (interleukin-2) | Cetus | AIDS, KS |
| <u>Drug Name</u> | <u>Manufacturer</u> | <u>Indication</u> |
| IL-2 (interleukin-2) | Hoffmann-La Roche Immunex | AIDS, KS |
| INTRON-A (interferon alfa) | Schering-Plough | KS |
| ISOPRINOSINE (inosine pranobex) | Newport Pharmaceuticals | ARC, PGL, HIV seropositive patients |
| (methionine enkephalin) | TNI Pharmaceuticals | AIDS, ARC |

| | | |
|---|---|---|
| MTP-PE (muramyl-tripeptide) | Ciba-Geigy | KS |
| THYMOPENTIN (TP-5) (thymic compound) | Ortho Pharmaceuticals | HIV infection |
| ROFERON (interferon alfa) | Hoffmann-La Roche | KS |
| (recombinant erythropoietin) | Ortho Pharmaceuticals | severe anemia assoc with AIDS & RETROVIR therapy |
| TREXAN (naltrexone) | DuPont | AIDS, ARC |
| TNF (tumor necrosis factor) | Genentech | ARC, in combination interferon gamma |
| | C. Antibiotics | |
| PENTAM 300 (pentamidine isethionate) | LyphoMed | PCP |
| | D. Vaccines | |
| Gag | Merck | AIDS,ARC |

It will be understood that the scope of combinations of the vaccines of this invention with AIDS antivirals, immunomodulators, antibiotics or vaccines is not limited to the list in the above Table, but includes in principle any combination with any pharmaceutical composition useful for the treatment of AIDS. The AIDS or HIV vaccines of this invention include vaccines to be used pre- or post-exposure to prevent or treat HIV infection or disease, and are capable of producing an immune response specific for the immunogen.

The conjugates of this invention, when used as a vaccine, are to be administered in immunologically effective amounts. Dosages of between 1 μg and 500 μg of conjugate protein, and preferably between 50 μg and 300 μg of conjugate protein are to be administered to a mammal to induce anti-peptide, anti-HIV, or HIV-neutralizing immune responses. About two weeks after the initial administration, a booster dose may be administered, and then again whenever serum antibody titers diminish. The conjugate should be administered

intramuscularly or by any other convenient or efficacious route, at a concentration of between 10 μg/ml and 1 mg/ml, and preferably between 50 and 500 μg/ml, in a volume sufficient to make up the total required for immunological efficacy. The conjugate may be preadsorbed to aluminum hydroxide gel or to the Ribi adjuvant (GB 2220211A, US priority document 212,919 filed 29/06/1988) and suspended in a sterile physiological saline solution prior to injection.

The protein moiety should behave as an immune enhancer. It is desirable, in the choice of protein, to avoid those that result in non-specific activation of the recipient's immune response (reactogenicity). In U.S. Patent 4,695,624, Marburg et al. used the outer membrane protein complex (OMPC) derived from Neisseria meningitidis to prepare polysaccharide-protein conjugates. OMPC has proven to be suitable though other immunogenic proteins may be used. The instant invention utilizes the Class II major immune enhancing protein (MIEP) of OMPC.

Various methods of purifying OMPC from the gram-negative bacteria have been devised [Frasch et al., J. Exp. Med. 140, 87 (1974); Frasch et al., J. Exp. Med. 147, 629 (1978); Zollinger et al., US Patent 4,707,543 (1987); Helting et al., Acta Path. Microbiol. Scand. Sect. C. 89, 69 (1981); Helting et al., US patent 4,271,147]. OMPC may be used herein essentially according to the Helting process, from which MIEP may be further purified [Murakami, K., et al., Infection and Immunity, 57, 2318 (1989)], to provide immune enhancement necessary to induce mammalian immune responses to HIV PND peptides. MIEP may be derived by dissociation of the isolated OMPC, or alternatively, produced through recombinant expression of the desired immunogenic portions of OMPC. Methods of preparing and using an OMPC subunit are disclosed in co-pending US application serial Nos. 555,329; 555,978; and 555,204 (Merck Case # 's 18159, 18110, and 18160 respectively).

The HIV PND peptides that may be used for making species of the conjugate of this invention may be linear or cyclic peptides. The linear peptides may be prepared by known solid phase peptide synthetic chemistry, by recombinant expression of DNA encoding desireable peptide sequences, or by fragmentation of isolated HIV proteins. Cyclic HIV PND peptides may be prepared by cyclization of linear peptides, for example (a) by oxidizing peptides containing at least two cysteines to generate disulfide bonded cycles; (b) by forming an amide bonded cycle; (c) by forming a thioether bonded cycle. Processes for making such peptides are described herein but this description should not be construed as being exhaustive or limiting. The conjugates of this invention are useful whenever a component peptide is an HIV PND or is capable of priming mammalian immune responses which recognize HIV PNDs.

PND peptides, both those known in the art and novel compounds disclosed herein are defined as peptidyl sequences capable of inducing an HIV-neutralizing immune response in a mammal, including the production of HIV-neutralizing antibodies.

A major problem overcome by the instant invention is the HIV interisolate sequence variability. For example, in the PND which occurs in the third hypervariable region of gp120 (see below), although certain amino acids have been found to occur at given locations in a great many isolates, no strictly preserved primary sequence motif exists. This difficulty is surmounted by this invention because it allows conjugation of a cocktail of peptides having PND sequences from as many different HIV isolates as necessary to attain broad protection. Alternatively, a broadly protective cocktail of conjugates may be prepared by mixing conjugates, each of which is prepared separately with a peptide moiety providing protection against a single or several HIV isolates.

The amino acids found near or between amino acids 296 and 341 of gp120 have been shown to meet the criteria which define a PND. In the IIIB isolate of HIV, a 41-amino-acid sequence has been reported as follows (SEQ ID: 1:):

-Ile Asn Cys Thr Arg Pro Asn Asn Asn Thr Arg Lys Ser Ile Arg Ile Gln Arg Gly Pro Gly Arg Ala Phe Val Thr Ile Gly Lys Ile Gly Asn Met Arg Gln Ala His Cys Asn Ile Ser-, with the two cysteines being disulfide bonded to each other to form a loop. The trimer -Gly Pro Gly- is exposed at the PND loop tip. Peptides from different HIV isolates from this same region of gp120 raise isolate-specific neutralizing antibodies when presented to goat and guinea pig immune systems as conjugates with keyhole-limpet hemocyanin. The major neutralizing epitope within the 41-mer sequence, presented above, is comprised by the eight amino acids surrounding and including the -Gly Pro Gly- trimer [Javaherian et al., PNAS USA 86, 6768 (1989)]. In Table II below a number of linear peptides of different length and composition that can be used to prepare the conjugates of this invention are presented. The name of the isolate containing a peptide having the sequence of the tabulated peptide is given, along with a name herein ascribed to that peptide for ease of reference. The letter r- on the left hand side of each peptide represents the possibility of linking the peptide to an immunogenic protein, such as the MIEP at that position. In addition, marker amino acids, such as norleucine and ornithine may form part of r-.

TABLE II

LINEAR HIV PND PEPTIDES

| HIV Isolate | Peptide Sequence | Name | SEQ ID NO: |
|---|---|---|---|
| MN | r-Tyr Asn Lys Arg Lys Arg Ile His Ile Gly Pro Gly Arg Ala Phe Tyr Thr Thr Lys Asn Ile Ile Gly Thr | PND142 | 2 |
| SC | r-Asn Asn Thr Thr Arg Ser Ile His Ile Gly Pro Gly Arg Ala PheTyr Ala Thr Gly Asp Ile Ile Gly Asp Ile | PND-SC | 3 |

| | | | |
|---|---|---|---|
| IIIB | r-Asn Asn Thr Arg Lys Ser Ile Arg Ile Gln Arg Gly Pro Gly Arg Ala Phe Val Thr Ile Gly Lys Ile Gly Asn | PND135 | 4 |
| IIIB | r-Arg Ile Gln Arg Gly Pro Gly Arg Ala Phe Val Thr Ile Gly Lys Ile Gly Asn | PND135-18 | 5 |
| IIIB | r-Arg Ile Gln Arg Gly Pro Gly Arg Phe Val Thr | PND135-12 | 6 |
| MN | r-His Ile Gly Pro Gly Arg Ala Phe | PND-MN8 | 7 |
| MN | r-Gly Pro Gly Arg Ala Phe | PND-MN6 | 8 |
| LAV-1 | r-Ile Gln Arg Gly Pro Gly Arg Ala Phe | PND-LAV-1 | 9 |
| SF2 | r-Ile Tyr Ile Gly Pro Gly Arg Ala Phe | PND-SF2 | 10 |
| NY5 | r-Ile Ala Ile Gly Pro Gly Arg Thr Leu | PND-NY5 | 11 |
| CDC4 | r-Val Thr Leu Gly Pro Gly Arg Val Trp | PND-CDC4 | 12 |
| RF | r-Ile Thr Lys Gly Pro Gly Arg Val Ile | PND-RF | 13 |

| | | | |
|---|---|---|---|
| ELI | r-Thr Pro Ile Gly Leu Gly Gln Ser Leu | PND-ELI | 14 |
| Z6 | r-Thr Pro Ile Gly Leu Gly Gln Ala Leu | PND-Z6 | 15 |
| MAL | r-Ile His Phe Gly Pro Gly Gln Ala Leu | PND-MAL | 16 |
| Z3 | r-Ile Arg Ile Gly Pro Gly Lys Val Phe | PND-Z3 | 17 |

This list is not an exhaustive list of possible PND sequences. Rather, it is provided as a suggestive and illustrative guide as to useful PND primary sequences. Therefore, peptides conjugated as herein described to form the immunogen of this invention are any of the tabulated peptides or immunologically-equivalent variants on the theme suggested by these peptidyl sequences. The nature of the variations is considered next.

The primary sequence of this HIV PND appears to have a conserved core amino acid sequence, comprised by the tetramer sequence -Gly Pro Gly Arg-, (SEQ ID: 18:) with increacing divergence on either side of this sequence among HIV isolates. Some isolates have sequences that diverge even within the tetramer, having -Gly Pro Gly Lys- (SEQ ID: 19:), -Gly Pro Gly Gln- (SEQ ID :20:), and even -Gly Leu Gly Gln- (SEQ ID: 21) core sequences. All of these possible sequences come within the scope of this disclosure as being peptide sequences that are advantageous for conjugation according to this invention.

The length of the peptide is a significant factor in promoting cross reactive immune responses. That is, an immune response raised against a given peptidyl epitope may recognize similar epitopes from the same or different HIV isolate based on the number of amino acids in the epitope over and above the critical neutralizing epitope. In addition, the length of the peptide is also responsible for determining the probability of exposure to the immune system of the determinant responsible for generating an HIV-neutralizing response.

In order to maximize the probability of relevant epitope presentation, chemistry was developed whereby the PND peptides may be locked into a given three-dimensional configuration. It is known that the 41-amino-acid PND of the HIV IIIB isolate, represented above, is configured as a loop by the presence of the cysteine-to-cysteine disulfide bond. Disulfides, however, may be labile under certain conditions and therefore may allow the loop to open and the peptide to exist in a linear form. Therefore, in addition to linear peptides, disulfide-bonded cyclic peptides and novel HIV PND peptides having nonlabile cyclic structures disclosed herein may all be utilized as the PEP component in the formation of the conjugates of this invention.

The peptides that may be used in formation of these conjugates may be derived as fragments of natural proteins (gp120 for example), by recombinant expression of portions thereof, or by chemical synthesis according to known methods in the art. In addition, novel cyclic PNDs may be prepared synthetically according to the processes herein described. The sequences may contain both natural L-amino acids, or unusual or D-amino acids. In addition, the conjugation chemistry is sufficiently flexible so that the appropriate choice of peptide derivatization reagents allows for successful conjugation.

Synthetic peptides have been prepared by a number of strategies conducted either in solution or on solid supports. Excellent texts covering the basic principles and techniques are: Principles of Peptide Synthesis, Bodanszky. M., Springer-Verlag (1984); Solid Phase Peptide Synthesis, Stewart J. M., Young, J. D., Pierce Chemical Company (2nd. ed. 1984); and The Peptides, Gross, E., Meienhofer, J., Academic Press, Inc., (1979). The processes described herein, however, are not limited to the disclosure of these texts.

Synthetic cyclic peptides may be prepared in two phases. First, the linear peptide may be synthesized on a Milligen 9050 peptide or an ABI 431A synthesizer using 9-fluorenylmethyloxy-carbonyl (Fmoc) chemistry and side-chain-protected Fmoc-amino acid pentafluorophenyl esters which are known reagents or using derivatized Wang resin, Fmoc chemistry, and side-chain protected Fmoc-amino acid symmetrical anhydrides, prepared in situ, as reagents.

Second, the linear peptide may be cyclized, either in solution or with the peptide still attached to the solid phase resin. Cyclization may be accomplished by any technique known in the art, which may comprise, for

example: a) incorporating cysteine residues into the linear peptide on either end of the sequence which is to form the loop and allowing disulfide bond formation under oxidizing conditions known in the art; b) preparing a cysteine containing peptide as in (a) but retaining the cysteines as free sulfhydryls (or as Acm protected thiols which are deprotected to the free sulfhydryls) and treating the peptide with o-xylylene dibromide or similar reagent, such as the diiodide, dichloride, or a dihalogenated straight or branched chain lower alkyl having between two and eight carbon atoms; such reagents react with the sulfur atoms of the cysteines to form a cyclic structure containing two nonlabile thioether bonds to the benzene or the alkyl; c) allowing a free group on one side of the loop amino acids to become amide bonded to a free carboxyl group on the other side of the loop amino acids through DPPA, BOP, or similar reagent mediated peptide bond formation. Each of these strategies is taken up in more detail below, after presentation of a generalized description of the cyclic peptides produced by these methods.

Thus, without limiting the conjugate invention to the following peptides or methods of producing them, the PND peptides which may be conjugated after removal of appropriate protecting groups as necessary, according to this invention include those represented by the structure PEP, which includes the linear peptides of Table II above and cyclic peptides:

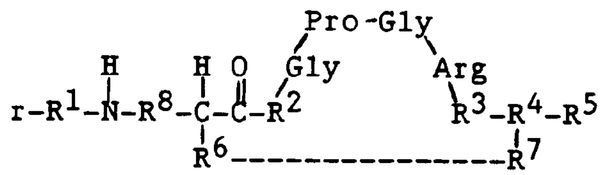

wherein:

r is the position of linkage between PEP and MIEP, optionally comprising a marker amino acid, if $R^1$ is not a marker amino acid;

$R^1$ is:

a) a bond, or

b) a peptide of 1 to 5 amino acids, optionally including a marker amino acid which migrates at a position in the amino acid analysis spectrum which is isolated from the signal of the 20 naturally occuring amino acids; preferably norleucine, gamma aminobutyric acid, β-alanine, or ornithine;

$R^2$ is :

a) either a bond or a peptide of up to 17 amino acids if $R^3$ is a peptide of at least 2 amino acids, or

b) a peptide of between 2 to 17 amino acids, if $R^3$ is a bond;

$R^3$ is:

a) either a bond or a peptide of up to 17 amino acids if $R^2$ is a peptide of at least 2 amino acids, or

b) a peptide of between 2 to 17 amino acids, if $R^2$ is a bond;

$R^4$ is:

a) -NH-CH-CO-, with $R^7$ bonded to the methine carbon, if $R^7$ is $R^8$, or

b) a bond from $R^3$ to $R^7$ and $R^5$, if $R^7$ is carbonyl or $-COCH_2CH_2CH(CONH_2)NHCO-$;

$R^5$ is:

a) a peptide of one to five amino acids, optionally including a marker amino acid,

b) -OH,

c) -COOH,

d) $-CONH_2$,

e) $-NH_2$, or

f) -absent;

$R^6$ is:

a) an amino acid side chain, selected from the side chain of any of the common L or D amino acids, (see table of Definitios and Abbreviations), if the optional bond (--------) to $R^7$ is absent,

b) $-R^8$-S-S-, or $-R^8-S-R^8-R^9-R^8-S-$, if $R^7$ is $R^8$, or

c) $-R^8$-NH- if $R^7$ is

-C=O, OR 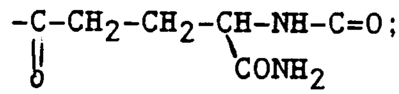

$R^7$ is:

a) $-R^8-$,

b) -C=O, or

c) 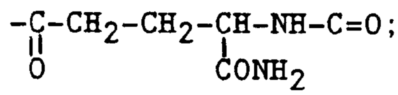

$R^8$ is a bond or lower alkyl of between one and eight carbons;

$R^9$ is:

a) $R^{10}$, or

b) xylylene

$R^{10}$ is:

a) lower alkyl, or

b) $-CH_2-O-CH_2-$; and

every occurrence of a variable is independent of every other occurrence of the same variable. When a peptide has been synthesized with a protected amino terminal amino acid, the amino terminal protecting group such as benzyloxy carbonyl (Z) for protecting amines, or acetamidomethyl (Acm) for protecting sulfhydryls, may be removed according to methods known in the art and exemplified herein. The deprotected group thus revealed may be utilized in covalent bond formation, through the linker r, to the immunogenic protein.

Hereinafter, amino acids $-R^2$-Gly Pro Gly Arg-$R^3$-, which form the "core" of the PND peptides, and go toward formation of the loop of a cyclic peptide, will be referred to as loop or core amino acids. When the optional bond between $R^6$ and $R^7$ is absent however, the structure, PEP, is linear, and encompasses all of the linear peptides of Table II.

Whether the peptide is linear or cyclic, the amino acid sequences comprising $R^2$ and $R^3$ of PEP may be any combination of amino acids, including sequences surrounding the core -Gly Pro Gly Arg-tetramer in any of the sequences of Table II. Thus, the core amino acids represented by $-R^2$-Gly Pro Gly Arg-$R^3$- may be further defined as having the core amino acid structure:

$-X_nX_1X_2$-Gly Pro Gly Arg-$X_3X_4X_m$-

wherein:

$X_1$ is a constituent of $R^2$ selected from:

a) serine,

b) proline,

c) arginine,

d) histidine,

e) glutamine, or

f) threonine;

$X_2$ is a constituent of $R^2$ selected from:

a) isoleucine,

b) arginine,

c) valine, or

d) methionine;

$X_n$ is is a constituent of $R^2$ and is either a bond or a peptide of up to 15 amino acids;

$X_3$ is a constituent of $R^3$ selected from:

a) alanine,

b) arginine, or

c) valine;

$X_4$ is a constituent of $R^3$ and is selected from:

a) phenylalanine,

b) isoleucine,

c) valine, or

d) leucine;

$X_m$ is a constituent of $R^3$ and is a bond or a peptide of up to 15 amino acids.

The cyclic peptides may be disulfide bonded structures or a cycle formed through a nonlabile bond or structure. The term "nonlabile bond" means a covalent linkage, other than a disulfide bond. These covalent linkages may be through a bridge structure, such as xylylene, through a lower alkyl, through $-CH_2-O-CH_2$, or through an amino acid amide-bonded bridge. By altering the bridge structure and/or the number and combination of amino acids included in the peptide, the conformation of the loop structure of the cycle may be optimized, allowing for fine-tuning of the PND epitope presented to the immune system. For example, use of an o-xylylene bridge generates a "tighter" loop structure than when, for example, an eight carbon straight chain lower alkyl is used as the bridge. Thus, the conjugates of this invention are useful both as reagents to analyze the structure-function relationship of the PND epitope in raising anti-peptide, anti-HIV, HIV-neutralizing, and anti-AIDS immune responses in mammals, and as components for formulation of anti-HIV disease, including AIDS, vaccines.

Synthetic products obtained may be characterized by fast-atom-bombardment mass spectrometry [FAB-MS], reverse phase HPLC, amino acid analysis, or nuclear magnetic resonance spectroscopy (NMR).

a. <u>Cyclic Peptides through Disulfide-Bonded Cysteines:</u>

Peptides containing cysteine residues on either side of the loop amino acids may be cyclized under oxidizing conditions to the disulfide-bonded cycles. Methods for achieving disulfide bonding are known in the art. An example of disulfide bonded peptides useful in this invention is given infra in Example 10, wherein cPND4 is produced and Example 18 wherein cPND33 is produced. In Example 10, a process utilizing the Acm derivative of cysteine to generate disulfide bonded cPNDs is used, but other processes are equally applicable. In Example 18, the peptide containg two sulfhydryls is oxidized in dilute acid. The disulfide bonded peptides are preferred in the instant invention.

Thus, in a preferred embodiment of this invention, the peptide has the structure (SEQ ID 18:):

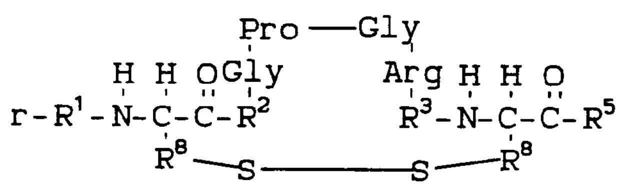

or pharmaceutically acceptable salts thereof, wherein:

r is:

a) hydrogen,

b)

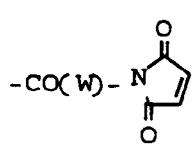 ,

wherein W is preferably $-(CH_2)_2-$ or $-(CH_2)_3-$ or $R^6$, where $R^6$ is

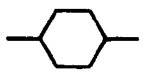 , 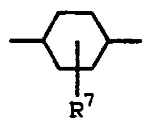 , 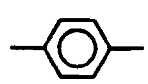 or 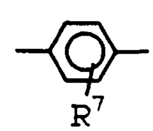

wherein $R^7$ is lower alkyl, lower alkoxy, or halo;

$R^1$ is:

a) a bond, or

b) a peptide of 1 to 5 amino acids, optionally including a marker amino acid;

$R^2$ is : a peptide of 3 to 10 amino acids

$R^3$ is: a peptide of 3 to 10 amino acids

$R^5$ is:

a) -OH,

b) a peptide of 1 to 5 amino acids, optionally including a marker amino acid, or

c) $-NH_2$;

$R^8$ is lower alkyl of between one and eight carbons.

Lower alkyl consists of straight or branched chain alkyls having from one to eight carbons unless otherwise specified. Hereinafter, amino acids -$R^2$-Gly Pro Gly Arg-$R^3$-, which go toward formation of the loop of a cyclic peptide, will be referred to as loop amino acids.

In one embodiment of the invention, the cyclic peptide having the structure (SEQ ID: 18:):

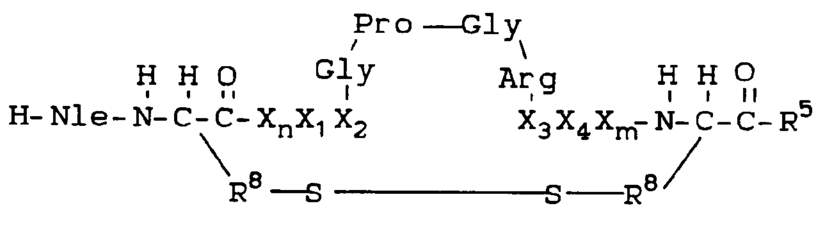

is prepared by cyclizing a linear peptide having the structure:

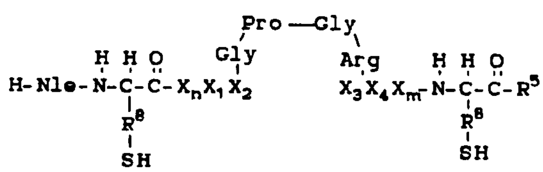

wherein:

$X_1$ is a constituent of $R^2$ selected from:

a) serine,

b) proline,

c) arginine,

d) histidine,

e) glutamine, which is preferred, or

f) threonine;

$X_2$ is a constituent of $R^2$ selected from:

a) isoleucine, which is most preferred,

b) arginine, which is preferred,

c) valine, or

d) methionine;

$X_n$ is a constituent of $R^2$ and is an amino acid or a peptide of up to 8 amino acids;

$X_3$ is a constituent of $R^3$ selected from:

a) alanine,

b) arginine, or

c) valine;

$X_4$ is a constituent of $R^3$ and is selected from:

a) phenylalanine,

b) isoleucine,

c) valine, or

d) leucine;

$X_m$ is a constituent of $R^3$ and is an amino acid or a peptide of up to 8 amino acids.

$X_2$ is preferably Isoleucine.

The novel disulfide bonded cyclic peptides used in this invention (and separately claimed in co-filed Merck case 18068IB, USSN , ) may be prepared in essentially two phases: First the linear peptide is synthesized on a Milligen 9050 or an ABI-431A peptide synthesizer using 9-fluorenyl-methyloxycarbonyl (Fmoc) chemistry and appropriately side-chain protected Fmoc-amino acid pentafluoro-phenyl esters as reagents or using derivatized Wang resin, Fmoc chemistry, and side-chain protected Fmoc-amino acid symmetrical anhydrides, prepared in situ, as reagents.

Second, the linear peptide is cyclized, either in solution or with the peptide still attached to the solid phase resin by incorporating cysteine residues into the linear peptide at either end of the sequence which is to form the loop, and oxidizing these to the disulfide. In a preferred embodiment, cyclization is accomplished by exposure of the peptide to (a) $H_2O_2$, (b) atmospheric oxygen, (c) aqueous $CH_3CN$ containing about 0.1 - 0.5% TFA, or (d) about 0.1M ferricyanide. The preferred method is exposure to atmospheric oxygen.

Products obtained may be characterized by fast atom bombardment-mass spectrometry [FAB-MS], reverse phase HPLC, amino acid analysis or nuclear magnetic resonance spectroscopy (NMR).

Thus, the peptides useful in this invention may be prepared as further described below in (i) and (ii):

i. Peptide Cyclization in the Solid State:

A linear peptide containing $C^1$ and $C^2$ on either side of the loop amino acids, where $C^1$ and $C^2$ are both cysteine or another amino acid containing free sulfhydryl groups in the side chain, is prepared according to known synthetic procedures (see discussion supra). In the completed cyclic PND, the sulfhydryl containing side chains, ($-R^8$-SH), go toward making up the $-R^8$-S-groups of the completed cyclic HIV PND structure shown above. Amino acids to be incorporated which have reactive side chains (R groups) are used in an appropriately R-group protected form. For example, histidine is triphenylmethyl (Trt), or Boc protected, and arginine is 4-methoxy-2,3,6-trimethylphenyl sulfonyl (Mtr) protected.

Preferably, a resin is purchased with $C^2$ in its Acm protected form already attached to the resin, for example, Fmoc-L-Cys(Acm)-O-Wang resin. The cysteine incorporated at the amino terminal side of the loop amino acids, $C^1$, may also be the Acm derivative. Either $C^1$ or $C^2$ may be bound to additional amino acids, $R^1$ or $R^5$ respectively, which may be utilized in the formation of conjugates with carrier molecules or may serve as marker amino acids for subsequent amino acid analysis, such as when norleucine or ornithine is used.

The sulfur of the acetamidomethylated cysteines are reacted, at room temperature for about 15 hours in a solvent compatible with the resin, as a 1-50% concentration of an organic acid, preferably about 10% acetic acid in anhydrous dimethylformamide (DMF), with about a four fold molar excess of a heavy metal salt, such as mercuric acetate $[Hg(OAc)_2]$ for each Acm group. The resulting heavy metal thioether, for example the mercuric acetate thioether of the peptide, PEP(S-HgOAc), is then washed and dried. Addition of excess hydrogen sulfide in DMF yields insoluble metal sulfide, e.g. mercuric sulfide (HgS), and the peptide with free sulfhydryl groups. The free sulfhydryls are then oxidized by one of the aforementioned methods. Alternatively, the Acm protected thiols may be converted directly to the cyclic disulfide by treatment with iodine in a methanol/DMF solvent.

ii. Cyclization of Peptides in Solution:

Essentially the same process described above for solid state cyclization applies with two main variants: If the peptide is cleaved (95% TFA/4% ethanedithiol/1% thioanisole) from a pepsyn KA resin, acid labile side chain protecting groups are also removed, including Cys(Trt) which provides the necessary free -SH function. If however, Cys(Acm) protection is used, then mercuric acetate/hydrogen sulfide cleavage to the free -SH group is required as an independent procedure, with the linear peptide either on or off the resin.

One method however, is the use of Cys(Acm) protection and Sasrin or Pepsyn KH resin, and cleavage of the linear, fully protected peptide from the resin with 1% $TFA/CH_2Cl_2$. Mercuric acetate/hydrogen sulphide then selectively converts Cys(Acm) to the free -SH group, and cyclization is effected on the otherwise protected peptide. At this point, the peptide may be maleimidated in situ , selectively on the N-terminus. Acid labile side chain protecting groups are cleaved with 98% TFA/2% thioanisole, and the cyclic peptide is isolated by HPLC. The preferred method, however, is to cleave the peptide from the resin, and allow cyclization by one of the aforementioned methods. The most preferred method is to allow air oxidation for about one to fifty hours of between 10 and 40°C.

Thus, in a particularly preferred embodiment of this invention, a peptide (CPND 33) having the structure (SEQ ID: 22:):

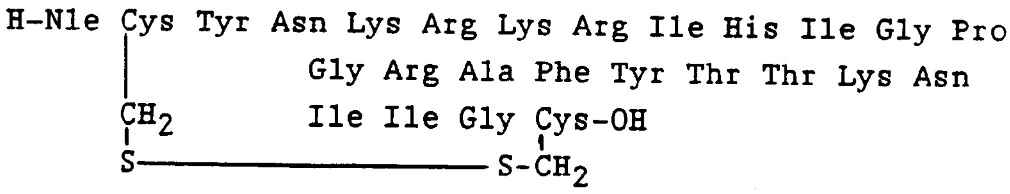

is conjugated to MIEP through either the amino terminal Nle or one of the internal lysines to generate one or a mixture of all of the structures:

e-1)

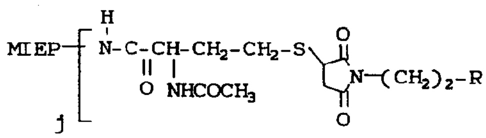

wherein R is:

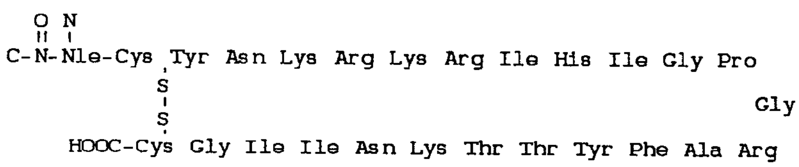

,

e-2)

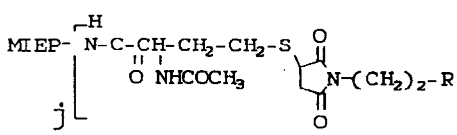

wherein R is:

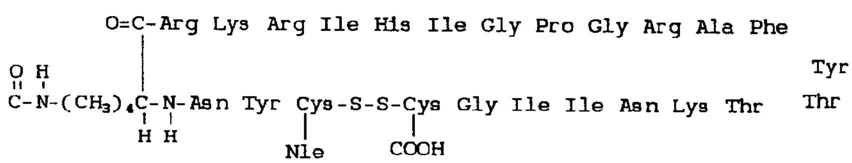

,

e-3)

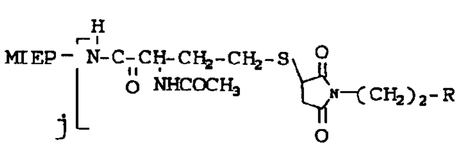

wherein R is:

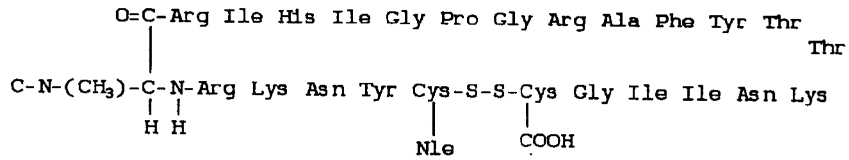

, or

e-4)

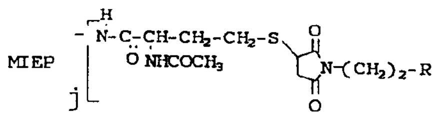

wherein R is:

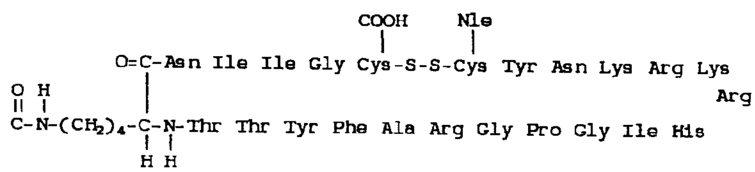

.

wherein j is the percentage by mass of peptide in the conjugate, and is preferably between 11 and 50% of the total protein mass in the conjugate.

b. Cyclic Peptides through Thioether Linkage to o-Xylylene or Lower Alkyls:

i. Peptide Cyclization in the Solid State:

A linear peptide containing $C^1$ and $C^2$ on either side of the loop amino acids, where $C^1$ and $C^2$ are both cysteine or another sulfhydryl containing amino acid, is prepared according to known synthetic procedures (see discussion supra). In the completed cylic PND, $C^1$ and $C^2$ become part of the $R^6$ and $R^7$ groups of the PEP structure shown above. Amino acids to be incorporated which have reactive side chains (R groups) are used in an appropriately R-group-protected form. For example, histidine is triphenylmethyl- (Trt) protected, arginine may be 4-methoxy-2,3,6-trimethylphenyl sulfonyl (Mtr) protected. [Principles of Peptide Synthesis, Bodanszky. M., Springer-Verlag (1984); Solid Phase Peptide Synthesis, Stewart J. M., Young, J. D., Pierce Chemical Company (2nd. ed. 1984); and The Peptides, Gross, E., Meienhofer, J., Academic Press, Inc., (1979)].

Preferably, a resin is purchased with $C^2$ in its Acm-protected form already attached to the resin, for example, Fmoc-L-Cys(Acm)-O-Wang resin. The cysteine incorporated at the amino terminal side of the loop amino acids, $C^1$, may also be the Acm derivative. Either $C^1$ or $C^2$ may be bound to additional amino acids, $R^1$ or $R^5$ respectively, which may be utilized in the formation of conjugates with carrier molecules or may serve as marker amino acids for subsequent amino acid analysis, such as when norleucine or ornithine is used.

The sulfur of the acetamidomethylated cysteines is reacted, at room temperature for about 15 hours in a solvent compatible with the resin, such as 10% acetic acid in anhydrous dimethylformamide (DMF), with about a four-fold molar excess of a heavy metal salt, such as mercuric acetate [$Hg(OAc)_2$] for each Acm group. The resulting heavy metal thioether, for example the mercuric acetate thioether of the peptide, PEP(S-HgOAc), is then washed and dried. Addition of excess hydrogen sulfide in DMF yields insoluble metal sulfide, e.g., mercuric sulfide (HgS), and the peptide with free sulfhydryl groups.

A mixture of about an equimolar amount, as compared with peptide, of o-xylylene dibromide or dichloride, a dibrominated or dichlorinated lower alkyl, 1,3-dihalogenenated -CH-O-CH-, or similar reagent which will provide a desirable bridge length, is added to the derivatized resin. A large excess of tertiary amine, preferably triethylamine ($NEt_3$) in DMF is added slowly. The reaction with the bis-sulfhydryl peptide moiety is allowed to proceed for about sixteen hours at room temperature, yielding the bridge group derivatized cyclic peptide bound to resin. Deprotection of acid sensitive side chain protecting groups and cleavage from the resin is achieved

by treatment with 95% trifluoroacetic acid (TFA) in the presence of 4% 1,2-ethanedithiol and 1% thioanisole. The dissolved cyclic peptide may then be separated from the resin by filtration. The filtrate is evaporated and the crude residual product is purified by high performance liquid chromatography (HPLC) according to known methods, for example by reverse phase HPLC.

ii. Cyclization of Peptides in Solution:

Essentially the same process described above for solid state cyclization applies with two main variants: If the peptide is cleaved (95% TFA/4% ethanedithiol/1% thioanisole) from a pepsyn KA resin, acid labile side chain protecting groups are also removed, including Cys(Trt) which provides the necessary free -SH function. If however, Cys(Acm) protection is used, then mercuric acetate/hydrogen sulfide cleavage to the free -SH group is required as an independent procedure, with the linear peptide either on or off the resin.

The preferred method however, is the use of Cys(Acm) protection and Sasrin or Pepsyn KH resin, and cleavage of the linear, fully protected peptide from the resin with 1% TFA/$CH_2Cl_2$. Mercuric acetate/hydrogen sulphide then selectively converts Cys(Acm) to the free -SH group, and cyclization is effected on the otherwise protected peptide. Acid labile side chain protecting groups are cleaved with 95% TFA/4% ethanedithiol/1% thioanisole, and the cyclic peptide is isolated by HPLC.

Removal of excess reagents, such as unreacted xylylene dibromide, prior to acid cleavage of the protecting groups is conveniently achieved by, for example, a step gradient reverse phase HPLC run prior to more selective gradient elution.

Cyclic HIV PND peptides prepared according to the method of this subsection include, but are not limited to, the sample cPNDs represented below. The methods of this subsection are generally applicable to small peptides, and particularly applicable to peptides of between 5 and 30 amino acids. An optimal ring size may include between 5 and 10 amino acids, including the -Gly-Pro-Gly- trimer, and this ring size is easily maintained by production of cycles from linear peptides having the appropriate number and combination of amino acids.

Representative peptides resulting from the process described in this subsection b. parts (i). and (ii) are disclosed inapplication U.S.S.N. 555,227. The conjugate invention should, however, not be construed as being limited to use those particular embodiments of HIV cyclic PND peptides. Other linear HIV PND peptide sequences may be cyclized in essentially the same fashion used to provide those peptides. Series of peptides having divergent primary sequences could be generated and would be beneficial in this invention as long as they continue to elicit an anti-peptide, anti-HIV, or HIV-neutralizing immune response.

c. Cyclization through Amide Bond Formation:

Novel amide bonded cyclic HIV PND peptides may be prepared for conjugation in essentially two phases: First, the linear peptide is prepared, for example on an ABI-431A peptide synthesizer, by known solid phase peptide synthetic chemistry, for example using Fmoc chemistry and appropriately side-chain protected Fmoc-amino acids as reagents.

Second, the linear peptide is cleaved from the resin and cyclized in solution by allowing the free amino terminus of the peptide, the free amino group of an amino terminal isoglutamine, or a free ε-amino or α-amino group of a lysine on one side of the loop amino acids to be amide bonded to a free carboxyl group on the carboxy-terminal side of the loop amino acids through DPPA, BOP, or similar reagent mediated peptide bond formation.

Products obtained may be characterized by fast atom bombardment-mass spectrometry [FAB-MS], reverse phase HPLC, amino acid analysis, or nuclear magnetic resonance spectroscopy (NMR).

Thus, highly preferred embodiments of this invention are conjugates having covalent linkages from MIEP to an amide bonded cyclic HIV PND, prepared as described hereinabove. Where the PND is from a predominant isolate, such as the HIV IIIB or the HIV MN isolate, a conjugate vaccine, or a mixture of such conjugate vaccines is highly advantageous for prophylaxis or treatment of AIDS or ARC. Examples of such preferred embodiments having the structure:

a)

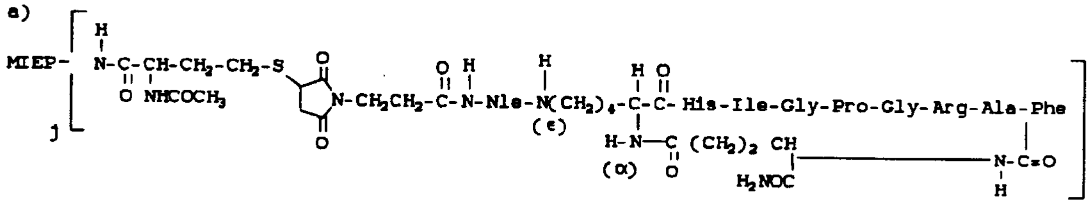

b)

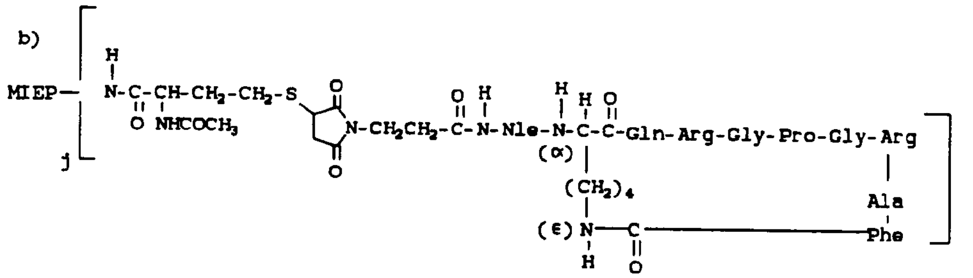

or pharmaceutically acceptable salts thereof, wherein:

j is the percentage by mass of peptide in the conjugate, and is preferably between 1% and 50% of the total protein mass in the conjugate;

are useful for inducing anti-peptide immune responses in mammals, for inducing HIV-neutralizing antibodies in mammals, for formulating vaccines to prevent HIV-disease or infection, or for treating humans afflicted with HIV-disease or infection, including AIDS and ARC.

One or more of the conjugate vaccines of this invention may be used in mammalian species for either active or passive protection prophylactically or therapeutically against infectious agents such as, in the preferred embodiment of this invention, Haemophilus influenzae serotype B, or human immunodeficiency virus induced diseases. Active protection may be accomplished by injecting an effective quantity capable of producing measurable amounts of antibodies (e.g., about 1 microgram to about 50 μg, depending on the antigen) of an antigen (e.g. PRP, HIV PND peptides) in the MIEP-conjugate form of each of the conjugates being administered per dose. The use of an adjuvant (e.g., alum) is also intended to be within the scope of this invention. Passive protection may be accomplished by injecting whole antiserum obtained from animals previously dosed with the MIEP-conjugate or conjugates, or globulin or other antibody-containing fractions of said antisera, with or without a pharmaceutically-acceptable carrier, such as sterile saline solution. Such globulin is obtained from whole antiserum by chromatography, salt or alcohol fractionation or electrophoresis. Passive protection may be accomplished by standard monoclonal antibody procedures or by immunizing suitable mammalian hosts.

In a preferred embodiment of this invention, the conjugate is used for active immunogenic vaccination of humans, especially infants, children, or immunocompromised individuals. For additional stability, these conjugates may also be lyophilized in the presence of lactose (for example, at 20 μg/mL of PRP/4 mg/mL lactose) prior to use.

A preferred dosage level is an amount of each of the MIEP-conjugates, or derivative thereof to be administered, corresponding to between approximately 2 to 20-μg of PRP, or about 1 microgram to 5 milligarams of peptide in the MIEP-conjugate form for conjugates of H. influenzae serotype B polysaccharide, or HIV PND peptide, in a single administration. If necessary, an additional one or two doses of the MIEP-conjugate, or derivative thereof, in a dosage comparable to that described above.

The invention is further defined by reference to the following examples, which are intended to be illustrative and not limiting.

EXAMPLE 1

Preparation of Neisseria meningitidis B11 Serotype 2 OMPC

**A. Fermentation**

1. Neisseria meningitidis Group B11

A tube containing the lyophilized culture of Neisseria meningitidis (obtained from Dr. M. Artenstein, Walter Reed Army Institute of Research (WRAIR), Washington, D.C.) was opened and Eugonbroth (BBL) was added. The culture was streaked onto Mueller Hinton agar slants and incubated at 37°C with 5% $CO_2$ for 36 hours, at which time the growth was harvested into 10% skim milk medium (Difco), and aliquots were frozen at -70°C. The identity of the organism was confirmed by agglutination with specific antiserum supplied by WRAIR, and typing serum supplied by Difco.

A vial of the culture from the second passage was thawed and streaked onto 10 Columbia Sheep Blood agar plates (CBAB-BBL). The plates were incubated at 37°C with 5% $CO_2$ for 18 hours after which time the growth was harvested into 100 mL of 10% skim milk medium, aliquots were taken in 0.5 mL amounts and frozen

at -70°C. The organism was positively identified by agglutination with specific antiserum, sugar fermentation and gram stain.

A vial of the culture from this passage was thawed, diluted with Mueller-Hinton Broth and streaked onto 40 Mueller-Hinton agar plates. The plates were incubated at 37°C with 6% $CO_2$ for 18 hours after which time the growth harvested into 17 mL of 10% skim milk medium, aliquotted in 0.3 mL amounts and frozen at -70°C. The organism was positively identified by Gram stain, agglutination with specific antiserum and oxidase test.

2. Fermentation and collection of cell paste

a. Inoculum Development-

The inoculum was grown from one frozen vial of Neisseria memingitidis Group B, B-11 from above (passage 4). Ten Mueller-Hinton agar slants were inoculated, and six were harvested approximately 18 hours later, and used as an inoculum for 3 250 mL flasks of Gotschlich's yeast dialysate medium at pH 6.35. The $O.D._{660}$ was adjusted to 0.18 and incubated until the $OD_{660}$ was between 1 and 1.8. 1 mL of this culture was used to inoculate each of 5 2L. Erlenmeyer flasks (each containing 1 liter of medium; see below) and incubated at 37°C in a shaker at 200 rpm. The O.D. was monitored at hourly intervals following inoculation. 4 liters of broth culture, at an $O.D._{660}$ of 1.28 resulted.

70 Liter Seed Fermenter- Approximately 4 liters of seed culture was used to inoculate a sterile 70-liter fermenter containing about 40 liters of complete production medium (see below). The conditions for the 70-liter fermentation included 37°C, 185 rpm with 10 liters/minute air sparging and constant pH control at about pH 7.0 for about 2 hours. For this batch, the final $O.D._{660}$ was 0.732 after 2 hours.

800-Liter Production Fermenter

Approximately 40 liters of seed culture were used to inoculate a sterile 800 liter fermenter containing 568.2 liters of complete production medium (see below). The batch was incubated at 37°C, 100 rpm with 60 liters/minute air sparging and constant pH control at pH 7.0. For this batch, the final O.D. was 5.58 thirteen hours after inoculation.

3. Complete Medium for Erlenmeyer flasks and 70-and 800-liter fermenters

| Fraction A | |
|---|---|
| L-glutamic acid | 1.5 g/liter |
| NaCl | 6.0 g/liter |
| $Na_2HPO_4$.anhydrous | 2.5 g/liter |
| $NH_4Cl$ | 1.25 g/liter |
| KCl | 0.09 g/liter |
| L-cysteine HCl | 0.02 g/liter |

**Fraction B (Gotschlich's Yeast Dialysate):**

1280 g of Difco Yeast Extract was dissolved in 6.4 liters of distilled water. The solution was dialyzed in 2 Amicon DC-30 hollow fiber dialysis units with three H10SM cartridges. 384 g $MgSO_4$.7-$H_2O$ and 3200 g dextrose were dissolved in the dialysate and the total volume brought to 15 liters with distilled water. The pH was adjusted to 7.4 with NaOH, sterilized by passage through a 0.22 μ filter, and transferred to the fermenter containing Fraction A.

For the Erlenmeyer flasks: 1 liter of Fraction A and 25 mL of Fraction B were added and the pH was adjusted to 7.0-7.2 with NaOH.

For the 70 liter fermenter: 41.8 liters of Fraction A and 900 mL of Fraction B were added and the pH was

adjusted to 7.0-7.2 with NaOH.

For the 800 liter fermenter: 553 liters of Fraction A and 15.0 liters of Fraction B were added and the pH was adjusted to 7.1-7.2 with NaOH.

d. Harvest and Inactivation

After the fermentation was completed, phenol was added in a separate vessel, to which the cell broth was then transferred, yielding a final phenol concentration of about 0.5%. The material was held a room temperature with gentle stirring until the culture was no longer viable (about 24 hours).

e. Centrifugation

After about 24 hours at 4°C, the 614.4 liters of inactivated culture fluid was centrifuged through Sharples continuous flow centrifuges. The weight of the cell paste after phenol treatment was 3.875 kg.

**B. OMPC Isolation**

Step 1. Concentration and diafiltration

The phenol inactivated culture was concentrated to about 30 liters and diafiltered in sterile distilled water using 0.L micro-hollow fiber filters (ENKA).

Step 2. Extraction

An equal volume of 2X TED buffer [0.1 M TRIS 0.01 M EDTA Buffer, pH 8.5, with 0.5% sodium deoxycholate] was added to the concentrated diafiltered cells. The suspension was transferred to a temperature regulated tank for OMPC extraction at 56 °C with agitation for 30 minutes.

The extract was centrifuged at about 18,000 rpm in a Sharples continuous flow centrifuge at a flow rate of about 80 mL/minute, at about 4°C. The viscous supernatant was then collected and stored at 4°C. The extracted cell pellets were reextracted in TED buffer as described above. The supernatants were pooled and stored at 4°C.

Step 3. Concentration by Ultrafiltration

The pooled extract was transferred to a temperature regulated vessel attached to AG-Tech 0.1 micron polysulfone filters. The temperature of the extract was held at 25°C in the vessel throughout the concentration process. The sample was concentrated tenfold at an average transmembrane pressure of between 11 and 24 psi.

Step 4. Collection and Washing of the OMPC

The retentate from Step 3 was centrifuged at about 160,000 x g (35,000 rpm) at about 70°C in a continuous flow centrifuge at a flow rate between 300 to 500 mL/minute, and the supernatant was discarded.

The OMPC pellet was suspended in TED Buffer (190 mL buffer; 20 mL/g pellet) Step 2 and Step 4 were repeated twice (skipping Step 3).

Step 5. Recovery of OMPC Product

The washed pellets from Step 4 were suspended in 100 mL distilled water with a glass rod and a Dounce homogenizer to insure complete suspension. The aqueous OMPC suspension was then filter sterilized by passage through a 0.22 μ filter, and the TED buffer was replaced with water by diafiltration against sterile distilled water using a 0.1 μ hollow fiber filter.

EXAMPLE 2

Preparation of H. Influenzae Type b Capsular Polysaccharide (PRP)

**Inoculum and Seed Development**

A Stage:

A lyophilized tube of Haemophilus influenzae type b, (cultured from Ross 768, received from State University of New York) was suspended in 1 mL of sterile Haemophilus inoculum medium (see below) and this suspension was spread on 9 Chocolate Agar slants (BBL). The pH of the inoculum medium was adjusted to 7.2 ± 0.1 (a typical value was pH 7.23) and the medium solution was sterilized prior to use by autoclaving at 121°C for 25 minutes. After 20 hours incubation at 37°C in a candle jar, the growth from each plate was resuspended in 1-2 mL Haemophilus inoculum medium, and pairs of slants were pooled.

| Haemophilus Inoculum Medium | |
|---|---|
| Soy Peptone gm/liter | 10 |
| NaCl gm/liter | 5 |
| $NaH_2PO_4$ gm/liter | 3.1 |
| $Na_2HPO_4$ gm/liter | 13.7 |
| $K_2HPO_4$ gm/liter | 2.5 |
| Distilled Water | To Volume |

The resuspended cells from each pair of slants was inoculated into three 250 mL Erlenmeyer flasks containing about 100 mL of Haemophilus Seed and Production medium. The 250 mL flasks were incubated at 37°C for about 3 hours until an $OD_{660}$ of about 1.3 was reached. These cultures were used to inoculate the 2 liter flasks (below).

B Stage:

2 Liter non-baffled Erlenmeyer flasks- 5 mL of culture from "A stage" (above) were used to inoculate each

of five two-liter flasks, each containing about 1.0 liter of complete Haemophilus seed and production medium (see below). The flasks were then incubated at 37°C on a rotary shaker at about 200 rpm for about 3 hours. A typical $OD_{660}$ value at the end of the incubation period was about 1.0.

| Complete Haemophilus Seed And Production Medium | Per liter |
|---|---|
| $NaH_2PO_4$ | 3.1 g/L |
| $Na_2HPO_4$ | 13.7 g/L |
| Soy Peptone | 10 g/L |
| Yeast extract diafiltrate (1) | 10 g/L |
| $K_2HPO_4$ | 2.5 g/L |
| NaCl | 5.0 g/L |
| Glucose (2) | 5.0 g/L |
| Nicotinamide adenine dinucleotide (NAD) (3) | 2 mg/L |
| Hemin (4) | 5 mg/L |

The salts and soy peptone were dissolved in small volumes of hot, pyrogen-free water and brought to correct final volume with additional hot, pyrogen-free water. The fermenters or flasks were then sterilized by autoclaving for about 25 minutes at 121°C, and after cooling yeast extract diafiltrate (1), glucose (2), NAD (3), and hemin (4) were added aseptically to the flasks or fermenters prior to inoculation.

(1) Yeast extract diafiltrate: 100 g brewers' yeast extract (Amber) was dissolved in 1 liter distilled water and ultrafiltered using an Amicon DC-30 hollow fiber unit with H10 x 50 cartridges with a 50 kd cutoff. The filtrate was collected and sterilized by passage through a 0.22 μ filter.

(2) Glucose was prepared as a sterile 25% solution in distilled water.

(3) A stock solution of NAD containing 20 mg/mL was sterilized by passage through a (0.22 μ filter) and added aseptically just prior to inoculation.

(4) A stock solution of Hemin 3X was prepared by dissolving 200 mg in 10 mL of 0.1 M NaOH and the volume adjusted with distilled, sterilized water to 100 mL. The solution was sterilized for 20 minutes at 121°C and added aseptically to the final medium prior to inoculation.

C Stage:

70 Liter Seed Fermenter- Three liters of the product of B Stage was used to inoculate a fermenter containing about 40 liters of Complete Haemophilus Seed And Production medium (prepared as described above) and 17 mL UCON B625 antifoam agent. The pH at inoculation was 7.4.

D Stage:

800 Liter Production Fermenter-Approximately 40 liters of the product of "C Stage" was used to inoculate an 800 liter fermenter containing 570 liters of Haemophilus Seed and Production medium (prepared as described above), scaled to the necessary volume, and 72 mL of UCON LB625 antifoam agent was added.

The fermentation was maintained at 37°C with 100 rpm agitation, with the $O.D._{660}$ and pH levels measured about every two hours until the $O.D._{660}$ was stable during a two-hour period, at which time the fermentation was terminated (a typical final $O.D._{660}$ was about 1.2 after about 20 hours).

HARVEST AND INACTIVATION

Approximately 600 liters of the batch was inactivated by harvesting into a "kill tank" containing 12 liters of 1% thimerosal.

CLARIFICATION

After 18 hours of inactivation at 4°C, the batch was centrifuged in a 4-inch bowl Sharples contiuous flow centrifuge at a flow rate adjusted to maintain product clarity (variable between 1.3 and 3.0 liters per minute). The supernatant obtained after centrifugation (15,000 rpm) was used for product recovery.

ISOLATION AND CONCENTRATION BY ULTRAFILTRATION

The supernatant from two production fermentations was pooled and concentrated at 2 to 8°C in a Romicon XM-50 ultrafiltration unit with twenty 50 kd cut-off hollow fiber cartridges (4.5 $m^2$ membrane area; 2.0 Lpm air flow and 20 psi). Concentration was such that after approximately 4.5 hours, about 1,900 liters had been concentrated to 57.4 liters. The filtrate was discarded.

48% AND 61% ETHANOL PRECIPITATION

To the 57.4 liters of ultrafiltration retentate, 53 liters of 95% ethanol was added dropwise over 1 hour with stirring at 4°C to a final concentration of 48% ethanol by volume. The mixture was stirred two additional hours at 4°C to insure complete precipitation, and the supernatant was collected following passage through a single 4-inch Sharples continuous flow centrifuge at 15,000 rpm at a flow rate of about 0.4 liters per minute. The pellet was discarded and the clarified fluid was brought to 82% ethanol with the addition of 40.7 liters of 95% ethanol over a one hour period. The mixture was stirred for three additional hours to insure complete precipitation.

RECOVERY OF THE SECOND PELLET

The resulting 48% ethanol-soluble-82% ethanol-insoluble precipitate was collected by centrifugation in a 4 inch Sharples continuous flow centrifuge at 15,000 rpm with a flow rate of about 0.24 liters per minute and the 82% ethanol supernatant was discarded. The crude product yield was about 1.4 kg of wet paste.

CALCIUM CHLORIDE EXTRACTION

The 1.4 kg grams of 82% ethanol-insoluble material, was mixed in a Daymax dispersion vessel 2°-8°C with 24.3 liters of cold, distilled water. To this mixture, 24.3 liters of cold 2M $CaCl_2.2H_2O$ was added, and the mixture was incubated at 4°C for 15 minutes. The vessel was then rinsed with 2 liters of 1 M $CaCl_2.2H_2O$, resulting in about 50 liters final volume.

23% ETHANOL PRECIPITATION

The 50 liters of $CaCl_2$ extract was brought to 25% ethanol by adding 16.7 liters of 95% ethanol dropwise, with stirring, at 4°C over 30 minutes. After additional stirring for 17 hours, the mixture was collected by passage through a Sharples continuous flow centrifuge at 4°C. The supernatant was collected and the pellet was discarded.

38% ETHANOL PRECIPITATION AND COLLECTION OF CRUDE PRODUCT PASTE

The 25% ethanol-soluble supernatant was brought to 38% ethanol by the addition of 13.9 liters of 95% ethanol, dropwise with stirring, over a 30 minute period. The mixture was then allowed to stand with agitation for one hour, then without agitation for 14 hours, to insure complete precipitation. The resulting mixture was then centrifuged in a 4 inch Sharples continuous flow centrifuge at 15,000 rpm (flow rate of 0.2 liters per minute) to collect the precipitated crude H. influenzae polysaccharide.

TRITURATION

The pellet from the centrifugation was transferred to a 1 gallon Waring Blender containing 2 to 3 liters of absolute ethanol and blended for 30 seconds at the highest speed. Blending was continued for 30 seconds on, and 30 seconds off, until a hard white powder resulted. The powder was collected on a Buchner funnel with a teflon filter disc and washed sequentially, in situ, with two 1 liter portions of absolute ethanol and two 2 liter portions of acetone. The material was then dried, in vacuo, at 4°C for 24 hours, resulting in about 337 g (dry weight) of product.

PHENOL EXTRACTION

About 168 grams of the dry material from the trituration step (about half of the total) was resuspended in 12 liters of 0.488 M sodium acetate, pH 6.9, with the aid of a Daymax dispersion vessel. The sodium acetate solution was immediately extracted with 4.48 liters of a fresh aqueous phenol solution made as follows: 590 mL of 0.488 M sodium acetate, pH 6.9, was added to each of eight 1.5 kg bottles of phenol (Mallinckrodt crystalline) in a 20 liter pressure vessel and mixed into suspension. Each phenol extract was centrifuged for 2.5 hours at 30,000 rpm and 4°C in the K2 Ultracentrifuge (Electronucleonics). The aqueous effluent was extracted three additional times with fresh aqueous phenol solution as described above. The phenol phases were discarded.

ULTRAFILTRATION

The aqueous phase from the first phenol extraction above (12.2 liters) was diluted with 300 liters of cold, distilled water and diafiltered at 4°C on an Amicon DC-30 ultrafiltration apparatus using 3 H10P10, 10 kd cutoff cartridges, to remove the carryover phenol. The Amicon unit was rinsed and the rinse added to the retentate, such that the final volume was 31.5 liters. The ultrafiltrate was discarded.

67% ETHANOL PRECIPITATION

0.81 liters of 2.0 M $CaCl_2$ was added to the 31.5 liters of dialysate from the previous step (final $CaCl_2$ concentration was 0.05 M) and the solution was brought to 82% ethanol with dropwise addition and rapid stirring over one hour, of 48.5 liters of 95% ethanol. After 4 hours of agitation, then standing for 12 hours at 4°C, the supernatant was siphoned off and the precipitate was collected by centrifugation in a 4 inch Sharples continuous flow centrifuge (15,000 rpm), at 4°C for 45 minutes. The resulting polysaccharide pellet was triturated in a 1 gallon Waring blender using 30 second pulses with 2 liters of absolute ethanol, collected on a Buchner funnel fitted with a teflon filter disc, and washed, in situ, with four 1 liter portions of absolute ethanol followed by two 1 liter portions of acetone. The sample was then dried in a tared dish, in vacuo, at 4°C for 20 hours. The yield was about 102 grams of dry powder. The yield from all phenol extractions was pooled resulting in a total of 212.6 grams of dry powder.

ULTRACENTRIFUGATION IN 29% ETHANOL AND COLLECTION OF FINAL PRODUCT

The 212.6 grams of dry powder from above was dissolved in 82.9 liters of distilled water, to which was added 2.13 liters of 2 M $CaCl_2.2H_2O$, (0.05M $CaCl_2$), 2.5 mg polysaccharide/mL), and the mixture was brought 29% ethanol with the dropwise addition of 29.86 liters of 95% ethanol over 30 minutes. The mixture was clarified immediately by centrifugation in a K2 Ultracentrifuge containing a K3 titanium bowl and a K11 Noryl core (30,000 rpm and 150 mL per minute flow rate) at 4°C. The pellet was discarded and the supernatant was brought to 38% ethanol by the addition of 17.22 liters of 95% ethanol over 30 minutes with stirring. After stirring 30 additional minutes the mixture was allowed too stand without agitation at 4°C for 17 hours and the precipitate was collected using a 4 inch Sharples continuous flow centrifuge at 15,000 rpm (45 minutes was required).

The resulting paste was transferred to a 1-gallon Waring blender containing 2 liters of absolute ethanol and blended at the highest speed by 4 or 5 cycles of 30 seconds on, 30 seconds off, until a hard, white powder formed. This powder was collected on a Buchner funnel fitted with a Zitex teflon disc and rinsed sequentially, in situ, with two fresh 0.5 liter portions and one 1 liter portions of absolute ethanol, and with two 1 liter portion of acetone. The product was removed from the funnel and transferred to a tared dish for drying, in vacuo, at 4°C (for 25 hours). The final yield of the product was 79.1 grams dry weight.

EXAMPLE 3

Cloning of Genomic DNA Encoding MIEP.

About 0.1 g of the phenol inactivated N. meningitidis cells (see Example 1) was placed in a fresh tube. The phenol inactivated cells were resuspended in 567 μL of TE buffer [10mM TRIS-HCl, 1mM EDTA, pH 8.0]. To the resuspended cells was added 30 μL of 10% SDS, and 3 μL of 20 mg/mL proteinase K (Sigma). The cells were mixed and incubated at 37°C for about 1 hour, after which 100 μL of 5 M NaCl was added and mixed thoroughly. 80 μL of 1% cetyltrimethylamonium bromide (CTAB) in 0.7 M NaCl was then added, mixed thoroughly, and incubated at 65°C for 10 minutes. An equal volume (about 0.7 to 0.8 mL) of chloroform/isoamyl alcohol (at a ratio of 24:1, respectively) was added, mixed thoroughly and centrifuged at about 10,000 x g for about 5 minutes. The aqueous (upper) phase was transferred to a new tube and the organic phase was discarded. An equal volume of phenol/chloroform/isoamyl alcohol (at a ratio of 25:24:1, respectively) was added to the aqueous phase, mixed thoroughly, and centrifuged at 10,000 x g for about 5 minutes. The aqueous phase (upper) was transferred to a new tube and 0.6 volumes (about 420 μL) of isopropyl alcohol was added, mixed thoroughly, and the precipitated DNA was pelletted by centrifugation at 10,000 x g for 10 minutes. The supernatant was discarded, and the pellet was washed, with 70% ethanol. The DNA pellet was dried and resuspended in 100 μL of TE buffer, and represents N. meningitidis genomic DNA.

Two DNA oligonucleotides were synthesized which correspond to the 5′ end of the MIEP gene and to the 3′ end of the MIEP gene [Murakami, E.C. et al., (1989), Infection and Immunity, 57, pp.2318-23]. The sequence of the DNA oligonucleotide specific for the 5′ end of the MIEP gene was: 5′-ACTAGTTGCAATGAAAAAATCCCTG-3′; and for the 3′ end of the MIEP gene was: 5′-GAATTCAGATTAGGAATTTGTT-3′. These DNA oligonucleotides were used as primers for polymerase chain reaction (PCR) amplification of the MIEP gene using 10 nanograms of N. meningitidis genomic DNA. The PCR amplification step was performed according to the procedures supplied by the manufacturer (Perkin Elmer).

The amplified MIEP DNA was then digested with the restriction endonucleases SpeI and EcoRI. The 1.3 kilobase (kb) DNA fragment, containing the complete coding region of MIEP, was isolated by electrophoresis on a 1.5% agarose gel, and recovered from the gel by electroelution [Current Protocols in Molecular Biology, (1987), Ausubel, R.M., Brent, R., Kingston, R.E., Moore, D.D., Smith, J.A., Seidman, J.G. and Struhl, K., eds., Greene Publishing Assoc.]

The plasmid vector pUC-19 was digested with SpeI and ECoRI. The gel purified SpeI-EcoRI MIEP DNA was ligated into the SpeI-EcoRI pUC-19 vector and was used to transform E. coli strain DH5. Transformants containing the pUC-19 vector with the 1.3 kbp MIEP DNA were identified by restriction endonuclease mapping, and the MIEP DNA was sequenced to ensure its identity.

EXAMPLE 4

Construction of the pcl/I.Gall0p(B)ADH1$_t$ vector:

The Gal 10 promoter was isolated from plasmid YEp52 [Broach, et al., (1983) in Experimental Manipulation of Gene Expression, Inouye, M(Ed) Academic Press pp. 83-117] by gel purifying the 0.5 kilobase pair (Kbp) fragment obtained after cleavage with Sau 3A and Hind III. The ADH1 terminater was isolated from vector pGAP.tADH2 [Kniskern, et al., 91986), Gene, 46, pp. 135-141] by gel purifying the 0.35 Kbp fragment obtained by cleavage with Hind III and SpeI. The two fragments were ligated with T4 DNA ligase to the gel purified puc-18ΔHind III vector (the Hind III site was eliminated by digesting pUC18 with Hind III, blunt-ending with the Klenow fragment of E. coli DNA polymerase I, and ligating with T4 DNA ligase) which had been digested with BamHI and SphI to create the parental vector pGallo-tADH1. This has a unique Hind III cloning site at the Gall0p.ADH1$_t$ junction.

The unique Hind III cloning site of pGall0.t ADH1 was changed to a unique BamHI cloning site by digesting pGall0.tADH1 with Hind III, gel purifying the cut DNA, and ligating, using T4 DNA ligase, to the following Hind III-BamHI linker:

```
5'-AGCTCGGATCCG-3'
     3'-GCCTAGGCTCGA-5'.
```

The resulting plasmid, pGall0(B)t ADH1, has deleted the Hind III site and generated a unique BamHI cloning site.

The Gall0p.tADH1 fragment was isolated from pGall0(B)t ADH1 by digestion with SmaI and SphI, blunt-ended with T4 DNA polymerase, and gel purified. The yeast shuttle vector pCl/1 [Brake et al., (1984), Proc. Nat'1. Acad. Sci. USA, 81, pp.4642-4646] was digested with SpHI, blunt-ended with T4 DNA polymerase, anpurified. This fragment was ligated to the vector with T4 DNA ligase. The ligation reaction mixture was then used to transform E. coli HB101 cells to ampicillin resistance, and transformants were screened by hybridization to a single strand of the $^{32}$P-labelled HindIII BamHI linker. The new vector construction, pcill.Gall0p(B) $ADH1_t$ was confirmed by digestion with HindIII and BamHI.

EXAMPLE 5

Construction of a Yeast MIEP Expression Vector with MIEP + Leader DNA Sequences

A DNA fragment containing the complete coding region of MIEP was generated by digestion of pUC19.M1EP #7 with SpeI and EcoRI, gel purification of the MIEP DNA, and blunt-ended with T4 DNA polymerase.

The yeast internal expression vector pCl/1.Gall0p(B) $ADH1_t$ was disgested with Bam HI, dephosphorylated with calf intestinal alkaline phosphatase, and blunt-ended with T4 DNA polymerase. The DNA was gel purified to remove uncut vector.

The 1.1 Kbp blunt-ended fragment of MIEP was ligated to the blunt-ended pcl/l.Gall0p(B) $ADH1_t$ vector, and the ligation reaction mixture was used to transform competent E. coli DH5 cells to ampicillin resistance. Transformants were screened by hybridization to a $^{32}$P-labelled DNA oilgoncleotide:

5'...AAGCTCGGATCCTAGTTGCAATG...3',which was designed to be homologous with sequences overlapping the MIEP-vector junction. Preparations of DNA were made from hybridization positive transformants and digested with KpnI and SalI to verify that the MIEP fragment was in the correct orientation for expression from the Gall0 promoter. Further confirmation of the DNA construction was obtained by dideoxy sequencing from the Gall0 promoter into the MIEP coding region.

Expression of MIEP by the transformants was detected by Western blot analysis. Recombinant MIEP produced in the transformants comigrated on polyacrylamide gels with MIEP purified from OMPC vesicles, and was immunologically reactive with antibodies specific for MIEP.

EXAMPLE 6

Construction of yeast MIEP expression vector with a 5'-Modified MIEP DNA Sequence.

A DNA oligonucleotide containing a HindIII site, a conserved yeast 5' nontranslated leader (NTL), a methionine start codon (ATG), the first 89 codons of the mature MIEP (beginning with Asp at position +20) and a KpnI site (at position +89) was generated using the polymerase chain reaction (PCR) technique. The PCR was performed as specified by the manufacturer (Perkin Elmer Cetus) using the plasmid pUC19M1EP42#7 as the template and the following DNA oligomers as primers:

5'CTAAGCTTAACAAAATGGACGTTACCTTGTACGGTACAATT3', and 5'ACGGTACCGAAGCCGCCTTTCAAG3'.

To remove the 5' region of the MIEP clone, plasmid pUC19M1EP42#7 was digested with KpnI and HindIII and the 3.4 Kbp vector fragment was agarose gel purified. The 280 bp PCR fragment was digested with KpnI and HindIII, agarose gel purified, and ligated with the 3.4 Kbp vector fragment. Transformants of E. coli HB101 (BRL) were screened by DNA oilgonucleotide hybridization and the DNA from positive transformants was analyzed by restriction enzyme digestion. To ensure that no mutations were introduced during the PCR step, the 280 bp PCR generated DNA of the positive transformants was sequenced. The resulting plasmid contains a HindIII - EcoRI insert consisting of a yeast NTL, ATG codon, and the entire open reading frame (ORF) of MIEP beginning at the Asp codon (amino acid +20).

The yeast MIEP expression vectors were constructed as follows. The pGAL10/pcl/l and pGAP/pCl/1 vectors [Vlasuk, G.P., et al., (1989) J.B.C., 264, pp.12,106-12,112] were digested with BamHI, flush-ended with the Klenow fragment of DNA polymerase I, and dephosphorylated with calf intestinal alkaline phosphatase. These linear vectors were ligated with the Klenow treated and gel purified HindIII- EcoRI fragment described above, which contains the yeast NTL, ATG and ORF of MIEP are forming pGall0/pcl/MIEP and pGAP/pGAP/pCl/MIEP.

Saccharomyces cerevisiae strain U9 (gall0pga14-) were transformed with plasmid pGall0/p/pCl/MIEP. Recombinant clones were isolated and examined for expression of MIEP. Clones were grown at 37°C with shaking in synthetic medium (leu-) containing 2% glucose (w/v) to an $O.D._{660}$ of about 6.0. Galactose was then added to 2% (w/v) to induce expression of MIEP from the Gall0 promoter. The cells were grown for an additional 45

hours following galactose induction to an O.D.$_{660}$ of about 9.0. The cells were then harvested by centrifugation. The cell pellet was washed with distilled water and frozen.

Western Blot For Recombinant MIEP

To determine whether the yeast was expressing MIEP, Western blot analysis was done. Twelve percent, 1 mm, 10 to 15 well Novex Laemmli gels are used. The yeast cells were broken in $H_2O$ using glass beads (sodium dodecylsulfate (SDS) may be used at 2% during the breaking process). Cell debris was removed by centrifugation for 1 minute at 10,000 x g.

The supernatant was mixed with sample running buffer, as described for polyacrylamide gel purification of MIEP. The samples were run at 35 mA, using OMPC as a reference control, until the dye part leaves the gel.

Proteins were transferred onto 0.45 μ pore size nitrocellulose paper, using a NOVEX transfer apparatus. After transfer the nitrocellulose paper was blocked with 5% bovine serum albumin in phosphate buffered saline for 1 hour, after which 15 mL of a 1:1000 dilution of rabbit anti-MIEP antiserum (generated from gel purified MIEP using standard procedures) was added. After overnight incubation at room temperature 15 mL of a 1:1000 of alkaline phosphatase conjugated goat anti-rabbit IgG was added. After 2 hours incubation the blot was developed using FAST RED TR SALT (Sigma) and Naphthol-AS-MX phosphate (Sigma).

## EXAMPLE 7

Bacterial Expression Of Recombinant MIEP

Plasmid pUC19-MIEP containing the 1.3 kilobase pair MIEP gene insert, was digested with restriction endonucleases SpeI and EcoRI. The 1.1kbp fragment was isolated and purified on an agarose gel using standard techniques known in the art. Plasmid pTACSD, containing the two cistron TAC promoter and a unique ECORI site, was digested with ECORI. Blunt ends were formed on both the 1.3 kbp MIEP DNA and the pTACSD vector, using T4 DNA polymerase (Boehringer Mannheim) according to the manufacturer's directions. The blunt ended 1.3 kbp MIEP DNA was ligated into the blunt ended vector using T4 DNA ligase (Boehringer Mannheim) according to the manufacturer's directions. The ligated DNA was used to transform E. coli strain DH5aIQMAX (BRL) according to the manufacturer's directions. Transformed cells were plated onto agar plates containing 25 ug kantamycin/mL and 50 ug penicillin/mL, and incubated for about 15 hours at 37 C. A DNA oligonucleotide with a sequence homologous with MIEP was labelled with $^{32}P$ and used to screen nitrocellulose filters containing lysed denatured colonies from the plates of transformants using standard DNA hybridization techniques. Colonies which were positive by hybridization were mapped using restriction endonucleases to determine the orientation of the MIEP gene.

Expression of MIEP by the transformants was detected by Western blot analysis. Recombinant MIEP produced in the transformants comigrated on polyacrylamide gels with MIEP purified from OMPC vesicles, and was immunologically reactive with antibodies specific for MIEP.

## EXAMPLE 8

Preparation of Purified MIEP from OMPC Vesicles or From Recombinant Cells by Polyacrylamide Gel Electrophoresis

Acrylamide/BIS (37.5:1) gels, 18 x 14 cm, 3 mm thick were used. The stacking gel was 4% polyacrylamide and the separating gel was 12% polyacrylamide. Approximately 5 μg of vesicle protein, or recombinant host cell protein, was used per gel. To 1 mL of OMPC vesicles was added 0.5 mL of sample buffer (4% glycerol, 300 mM DTT, 100 mM TRIS, 0.001% Bromophenol blue, pH 7.0). The mixture was heated to 105°C for 20 minutes and allowed to cool to room temperature before loading onto the gel. The gel was run at 200-400 milliamps, with cooling, until the Bromophenol blue reached the bottom of the gel. A verticle strip of the gel was cut out (about 1-2 cm wide) and stained with Coomassie/cupric acetate (0.1%). The strip was destained until the MIEP band (about 38 Kd) became visible. The strip was then placed into its original gel position and the MIEP area was excised from the remainder of the gel using a scalpel.

The excised area was cut into cubes (about 5 mm) and eluted with 0.01 M TRIS-buffer, pH 0.1. After 2 cycles of elution the eluate was evaluated for purity by SDS-PAGE. The eluate was combined with a common pool of eluates and dialysed against borate-buffer (0.1 M boric acid, pH 11.5). After dialysis the eluted protein was concentrated using an Amicon stirred cell with YM10 membranes (10,000 molecular weight cutoff). The

material was further purified by passage through a PD10 sizing column (Pharmacia, Piscataway, NJ), and was stored at room temperature in borate buffer.

EXAMPLE 9

Carrier activity of MIEP in covalent PRP-OMPC conjugate

Immunizations:

Male C3H/HeN mice (Charles River, Wilmington, MA) were immunized intraperitoneally (IP) with PRP covalently linked to OMPC (PRP-OMPC; comprising 2.5 μg PRP and 17 μg OMPC) or PRP coupled to DT (PRP-DT; containing 2.5-7.5 μg PRP and 1.8-5.4 μg DT) (Connaught Laboratories, Willowdale, ONT), suspended in 0.5 mL of 0.01 M phosphate-buffered saline (PBS). A second group of male C3H-HeN mice, received either 17 μg of MIEP, 17 μg of OMPC, or OMPC-IAA (OMPC derivatized with N-acetyl homocysteine thiolactone, and capped with iodoacetamide). Cell donors for adoptive transfer experiments were twice immunized IP, 21 days apart, and spleen cells were collected 10 days after the second immunization. Adoptive transfer recipients were male C3H/HeN mice given 500R total body gamma-irradiation from a $^{137}Cs$ source and immediately reconstituted by intravenous injection of 8 x $10^7$ spleen cells from each of two syngeneic donors separately primed with PRP-DT, and OMPC, MIEP, or OMPC-IAA. Control mice received 8 x $10^7$ spleen cells from one donor mouse primed with PRP-OMPC and an equal number of spleen cells from an unprimed donor mouse.

ELISA for anti-PRP antibody:

Reactive amines were introduced into purified H. influenzae PRP by treatment with carbonyldiimidazole and reaction with butanediamine as described by Marburg et al., U.S. Patent 4,882,317. This derivatized PRP was chromatographed on Sephadex G-25 in 0.1M sodium bicarbonate buffer, pH 8.4. N-hydroxysuccinimidobiotin (Pierce Chemical, Rockford, IL) in dimethylsulfoxide was added to the eluate to a final concentration of 0.3 mg/mL and reacted in the dark for 4 hours at ambient temperature (about 25-28°C). Unreacted N-hydroxysuccinimido-biotin was removed by gel filtration over Sephadex G-25 in PBS. Costar (Cambridge, MA) polyvinyl chloride ELISA plates were coated with 50 μg/well of avidin (Pierce Chemical) at 10 μg/mL in 0.1 M sodium bicarbonate buffer, pH 9.5, overnight at ambient temperature and 100% humidity. Plates were washed 3 times with 0.05 M TRIS-buffered saline, pH 8.5, containing 0.05% Tween-20 (TBS-T), and blocked with TBS-T plus 0.1% gelatin (blocking buffer) at ambient temperature and 100% humidity for 1 hour. Plates were blotted without washing, 50 μg/well PRP-biotin in PBS at 15-40 μg/mL was added, and the plates were incubated for 1 hour. Plates were washed 3 times with TBS-T prior to sample addition. Samples were added in two-fold serial dilutions in blocking buffer, and incubated for 2 hours at ambient temperature and 100% humidity. The plates were then washed 3 times with TBS-T, and appropriate alkaline-phosphatase conjugated anti-immunoglobulins diluted in blocking buffer were added. The antibodies used were goat anti-mouse IgM (Jackson Immunoresearch, West Grove, PA), IgG (Fc) (Jackson Immunoresearch), IgG1 (gamma) (BRL, Gaithersburg, MD), IgG2a (gamma) (BRL), I gG2b (gamma) (Southern Biotechnology Associates, Birmingham, AL), 1gG3 (gamma) (Southern Biotechnology Associates), and goat anti-rabbit IgG (Jackson Immunoresearch). Plates were incubated for 2 hours at ambient temperature and 100% humidity, washed with blocking buffer, and substrate development was carried out using p-nitrophenyl phosphate (1 mg/mL in 1 M diethanolamine, Kirkegaard and Perry, Gaithersburg, MD). Dilutions were considered positive if the sample absorbance exceeded the mean absorbance plus 3 times the standard deviation of 8 reagent blanks, and the difference in absorbance between successive dilutions was 0.01 or greater. Endpoint titers were defined as the reciprocal of the highest dilution which gave a positive reaction in the ELISA as described above. Logarithms of reciprocal titers were compared between treatment groups by two-way analysis of variance [Lindeman, R.H. et al., (1980), Introduction to Bivariate and Multivariate Analysis, Scott Foresman (pub.), New York].

RIA for anti-PRP antibody quantitation:

The experimental samples of serum to be tested for the amount of anti-PRP antibodies were diluted 1:2, 1:5, and 1:20, using fetal calf serum as the diluent. 25 μL of each diluted serum sample was transferred, in duplicate, to 0.5 mL RIA vials (Sarstedt). A solution of PRP labelled with $^{125}I$ was diluted to yield between 300 and 800 counts per minute (cpm) per 50 μL, using phosphate buffered saline as the diluent. 50 μL of diluted $^{125}I$-PRP was transferred to each RIA vial, mixed thoroughly and incubated for about 15 hours at 4°C. 75 μL of a saturated solution of ammonium sulfate at 4°C was added to each RIA vial, mixed thoroughly and incubated

at 4°C for 1 hour. The RIA vials were then centrifuged for 10 minutes at 10,000 x g, the supernatant was discarded and the cpm in the pellet was measured in a gamma counter (LKB).

A standard curve consisting of serial two-fold dilutions of an antiserum containing a known quantity of anti-PRP antibodies was prepared as described above and were assayed concomitantly with the experimental serum samples. The quantity of anti-PRP antibodies in the standard curve was between 14 μg/mL as the highest quantity of antibodies and 0.056 μg/mL as the lowest quantity of antibodies. All samples were run in duplicate.

The average CPM of the duplicate samples was compared with the standard curve to calculate the amount of anti-PRP antibodies present in the experimental serum samples.

Antibody responses of adoptive transfer recipients:

Recipients of spleen cells primed separately with PRP-DT, and either MIEP or OMPC or IAA-OMPC, responded to immunization with PRP-OMPC by production of equivalent amounts of serum IgG1 and IgG2a anti-PRP antibody within 4 days (see Figure 1). Irradiated mice reconstituted with spleen cells which were carrier-primed with MIEP or OMPC or IAA-OMPC, had significantly higher IgG1 ($p<0.001$) and IgG2a ($p<0.04$) anti-PRP antibody titers after immunization with PRP-OMPC than control mice, given PRP-DT-primed but not OMPC-primed spleen cells. The serum antibody responses to immunization with PRP-OMPC in mice given spleen cells primed separately with PRP-DT and either MIEP or OMPC or IAA-OMPC were comparable to those in mice given spleen cells primed with PRP-OMPC ($p>0.12$ for IgG1 antibody on days 6-13, and $p>0.5$ for IgG2a antibody on days 9-13). No antibody response was seen when irradiated mice reconstituted with PRP-DT-primed and either MIEP or OMPC-primed spleen cells were immunized with PRP without a protein carrier. Stasticial analysis was done by two-was analysis of variance (ANOVA) [Lindeman, R.H. et al., Introduction to Bivariate and Multivariate Analysis, (1980), Scott Foresman, New York].

These results demonstrate that MIEP functioned in mice as well as OMPC to induce a carrier T helper cell response for the generation of anti-PRP IgG antibodies.

EXAMPLE 10

Mitogenic Activity of MIEP

MIEP purified from N. meningitidis. OMPC was tested for mitogenic activity in a lymphocyte proliferation assay. Murine splenic lymphocytes were obtained from C3H/HeN, C3H/FeJ, C3H/HeJ, or Balb/c mice. The mice were either naive or had previously been vaccinated with PRP-OMPC. The spleen cells were passed through a sterile, fine mesh screen to remove the stromal debris, and suspended in K medium [RPMI 1640 (GIBCO) plus 10% fetal calf serum (Armour), 2 mM Glutamine (GIBCO), 10 mM Hepes (GIBCO), 100 u/mL penicillin/100/μg/mL streptomycin (GIBCO), and 50 μM β-mercaptoethanol (Biorad)]. Following pipetting to disrupt clumps of cells, the suspension was centrifuged at 300 x g for 5 minutes, and the pellet was resuspended in red blood cell lysis buffer [90% 0.16 M $NH_4C1$ (Fisher), 10% 0.7 TRIS-HC1 (Sigma), pH 7.2] at room temperature, 0.1 mL cells/mL buffer for two minutes. Cells were underlayered with 5 mL of fetal calf serum and centrifuged at 4,000 x g for 10 minutes, then washed with K medium two times and resuspended in K medium at $5 \times 10^6$ cells/mL. These cells were plated (100 μL/well) into 96 well plates along with 100 μL of protein or peptide sample, in triplicate.

The MIEP of N. meningitidis was purified as previously described in Example 7. Control proteins included bovine serum albumin, PRP-OMPC and OMPC itself, and lipopolysaccharide (endotoxin). All samples were diluted in K medium to concentrations of 1, 6.5, 13, 26, 52, 105, and 130 μg/mL, then plated as described above such that their final concentrations were one-half of their original concentrations. Triplicate wells were also incubated for each type of cell suspended in K medium only, to determine the baseline of cell proliferation.

On day 3, 5, or 7 in culture, the wells were pulsed with 25 μL of $^3$H-thymidine (Amersham) containing 1 mCi/25 μL. The wells were harvested 16-18 hours later on a Skatron harvester, and counts per minute (CPM) was measured in a liquid scintillation counter. The net change in cpm was calculated by subtracting the mean number of cpm taken up per well by cells in K medium alone, from the mean of the experimental cpm. The stimulation index was determined by dividing the mean experimental cpm by the mean cpm of the control wells.

As shown in Figure 2, MIEP as well as OMPC and PRP-OMPC vaccine resulted in proliferation of lymphocytes from previously vaccinated mice. This mitogenic activity did not appear to be due to lipopolysaccharide (LPS) since the MIEP was free of detectable LPS, measured by rabbit pyrogenicity assays, and the proliferative effect was greater than that which could have been caused by LPS present in amounts below the level of detectability on silver stained polyacrylamide gels.

EXAMPLE 11

Conjugation of H. influenzae type-b PRP polysaccharide to N. meningitidis MIEP

Chemical conjugations were conducted according to the method disclosed in U.S. Patent number 4,882,317.

10 mg of MIEP in 3 mL of 0.1 M borate buffer, pH 11.5, was mixed with 10 mg of ethylenediamine tetraacetic acid disodium salt (EDTA, Sigma chemicals) and 4 mg of dithiothreitol (Sigma Chemicals). The protein solution was flushed thoroughly with $N_2$. 125 mg of N-acetylhomocysteinethiolactone (Aldrich Chemicals) was added to the MIEP solution, and the mixture was incubated at room temperature for 16 hours. It was then twice dialyzed under $N_2$ against 2 L of 0.1 M borate buffer, pH 9.5, containing 4 mM EDTA, for 24 hours at room temperature. The thiolated protein was then assayed for thiol content by Ellman's reagent (Sigma Chemicals) and the protein concentration was determined by Bradford reagent (Pierce Chemicals). For conjugation of MIEP to PRP, a 1.5 fold excess (wt/wt) of bromoacetylated H. influenzae serotype b PRP was added to the MIEP solution and the pH was adjusted to 9 - 9.5 with 1 N NaOH. The mixture was allowed to incubate under $N_2$ for 6 to 8 hours at room temperature. At the end of the reaction time, 25 μL of N-acetylcysteamine (Chemical Dynamics) was added to the mixture, and was allowed to stand for 18 hours under $N_2$ at room temperature. The conjugate solution was acidified to between pH 3 to 4 with 1 N HC1, and centrifuged at 10,000 x g for 10 minutes. 1 mL of the supernatant was applied directly onto a column of FPLC Superose 6B (1.6 x 50 cm, Pharmacia) and the conjugate was eluted with PBS. The void volume peak which contains the polysaccharide-protein conjugate (PRP-MIEP), was pooled. The conjugate solution was then filtered through a 0.22 μ filter for sterilization.

EXAMPLE 12

Demonstration of Immunogenicity of PRP-MIEP conjugates

Immunizations:

Male Balb/c mice (Charles River, Wilmington, MA) were immunized IP with PRP covalently conjugated to MIEP as described in Example 11, using 2.5 μg PRP in 0.5 mL of preformed alum. Control mice were immunized with equivalent amounts of PRP given as PRP-CRM (2.5 μg PRP/6.25 μg CRM; 1/4 of the human dose), PRP-DT (2.5 μg PRP/1.8 μg DT; 1/10 of the human dose), and PRP-OMPC (2.5 μg PRP/35 μg OMPC; 1/4 of the human dose).

Infant Rhesus monkeys, 6-13.5 weeks of age, were immunized with PRP-MIEP conjugates adsorbed onto alum. Each monkey received 0.25 mL of conjugate at two different sites of injection, for a total dose of 0.5 mL. The monkeys were immunized on day 0, 28, and 56, and blood samples were taken every two to four weeks.

Antibody responses were measured by the ELISA described in Example 9, which distinguishes the class and subclass of the immunoglobulin response. An RIA which quantitates the total anti-PRP antibody (see Example 9) was also used to evaluate the monkey response. Antibody responses of recipients of PRP-MIEP conjugates are shown in Figure 3.

The results show that PRP-MIEP conjugates are capable of generating an immune response in infant Rhesus monkeys and mice, consisting of IgG anti-PRP antibody and a memory response. This is in contrast to the PRP-CRM and PRP-DT which do not elict measurable anti-PRP antibody. Thus, MIEP functions as an immunologic carrier protein for PRP and is capable of engendering an anti-PRP antibody response when covalently conjugated to the PRP antigen. Purified MIEP is therefore an effective immunologic carrier protein replacing the heterogeneous OMPC in construction of bacterial polysaccharide conjugate vaccines.

EXAMPLE 13

PREPARATION OF MIEP - cPND15 CONJUGATE:

To 10.5 mL of a MIEP solution (1.85 mg/mL, 19.4 mg total) contained in a 50 mL flask was added 2.6 mL of a 0.1 M, pH 11 borate buffer. The pH was adjusted to 10.8 with 5N NaOH after addition of 37 mg EDTA and 11 mg dithiothreitol. Then 346 mg of N-acetylhomocysteine thiolactone was added and the pH again adjusted to 11 with 5N NaOH. This solution was degassed, the air replaced with nitrogen and the solution aged for 23 hours under an atmosphere of nitrogen.

The sample was then dialized against 4L of pH9.5 borate containing 10 mL, EDTA for 7 hr; against a fresh 4L for 22 hrs and finally against a pH 9.5 0.01M borate buffer containing 1.9 mg DTT for 16 hrs. This treatment

afforded a solution that contained a total of 4.84 rmoles of thiol (by Ellman assay). This equates to 249 nanomoles SH/mg protein.

A 10 mg sample of maleimidated cPND15 from Example 13 was dissolved in 1 mL of $H_2O$ and 50 μL of this was used for a maleimide assay by the reverse Ellman method, to reveal 5.4 μmoles (total) of maleimide. A 0.9 mL (4.88 μmoles) aliquot of the solution was added to the thiolated MIEP solution (pH 9.5), which immediately became turbid and after 3 hrs and 40 minutes no thiol titer (by Ellman assay) remained.

The solution (14 mL) was dialyzed twice vs 4L of a pH 9.5, 0.01M borate buffer for 27.5 and 38 hrs respectively. An assay on 100 μL for amino acid composition gave the following results:

nanamoles/0.1 mL sample:

| | |
|---|---|
| norleucine | 15.9 |
| β-alanine | 13.7 |
| lysine | 48.8 |

A Bradford protein assay on 100 μL showed 0.95 mg/mL. Using a molecular weight of 1111, this translates as 176.7 μg/mL of peptide. Thus the peptide to protein loading was 18.6%.

## EXAMPLE 14

### PREPARATION OF MIEP-cPND31 CONJUGATE

To 6.5 mL of a MIEP solution (1.7 mg/mL) was added 1.5 mL of a pH 11, 0.1 M borate buffer and the pH adjusted to 11 with 5 μL of 5N NaOH. To this was added 21 mg of EDTA and 6.5 mg of DTT and solution was effected by tumbling for 15 min. Then 200 mg of N-acetylhomocysteine thiolactone was added, the solution degassed and the air replaced by $N_2$. After aging in the $N_2$ box for 1.5 hrs., the pH was adjusted to 10.66 with 5N NaOH, the degassing process repeated, and ageing continued for 20.5 hrs.

The solution was dialyzed vs 4L of 0.1M pH9.5 borate containing 0.01 M EDTA for 6.5 hr followed by 4L of 0.1M pH 9.6 borate, 10 mM EDTA containing 1 mg dithiolthreitol for 17 hr. An Ellman assay indicated 2.27 μmoles (total) of thiol which is equivalent to 205 nanomoles SH/mg protein.

To this thiolated protein solution was added 0.55 mL of maleimidated cPND31 from Example 14 (3.77 μmoles/mg, by reverse Ellman assay, 2.07 μmoles total). An instant turbidity was noted. An additional 0.5 mg of maleimidated cPND31 was added and the mixture was aged for 1 hour.

To remove unconjugated peptide, the mixture was dialyzed in dialysis tubing, having a molecular weight exclusion limit of 12,000-14,000, vs 4L of pH 9.48 0.1M borate for 5.25 hours and vs 4L of pH 9.68 0.01M borate for 66 hrs. A total of 8 mL of solution remained from which 200 μL was removed for amino acid analysis:

| | |
|---|---|
| norleucine | 22.8 nanomoles/200 μL. |
| lysine 85.9 | nanomoles/200 μL. |

The solution was then dialyzed vs 200 mL of pH 7.07 0.1 M phosphate buffer which was 5 M in urea, affording a final volume of 6.5 mL. A Bradford protein assay revealed 1.26 mg protein/mL (8.2 mg total). Thus, 0.912 μmoles peptide (8 mL X 22.8 nanomoles/0.2 mL) at a molecular weight of 1204 = 1.1 mg of peptide (total). Therefore, in this case, a peptide to protein loading of 13% was achieved.

## EXAMPLE 15

### Solid State Synthesis of Disulfide-Bonded cPND4:

A linear PND peptide was prepared on Wang resin using an ABI-431A peptide synthesizer, starting from Fmoc-L-Cys(Acm)-O-Wang resin (0.61 meq/gram). Fmoc chemistry and Fmoc-Amino Acid symmetrical anhydrides (4X excess, prepared in situ) were used as reagents on a 0.25 mmole scale to generate 745 mg of the peptide:

```
              Acm                         Mtr
               |                           |
Fmoc-Nle-Cys-His-Ile-Gly-Pro-Gly-Arg-Ala-Phe-Cys-O-Wang Resin.
                   |                                  |
                  Trt                                Acm
```

A solution of iodine in 5% methanol/anhydrous DMF (1 ml) was added to the dried, derivatized Wang resin shown above and stirred at room temperature for 4 hours. The resin was filtered, washed with anhydrous DMF (5 x 2 ml), and finally resuspended in DMF (2 ml). Two drops of a 0.1 M solution of sodium thiosulphate in water were added, and stirred for a few seconds. The resin was washed with aqueous 95% DMF (3 x 2 ml), anhydrous

DMF (2 ml), methylene chloride (3 x 2 ml), ether (3 x 2 ml) and dried.

The Fmoc and other protecting groups were removed by treatment with 20% piperidine in DMF over 20 minutes, and the resin was washed and dried. The resin was cleaved from the disulfide bonded cyclic peptide by treatment with 95% TFA/4% ethane dithiol/1% thioanisole (1 ml) at room temperature for 6 hours. The solution was filtered, the resin washed with additional 100% TFA (3 x 1 ml), and the combined filtrate dried. Material that was insoluble in ether was removed by extraction (3 x 2 ml) and the solution redried.

Preparative HPLC using two 2.12 x 25 cm Vydac C18 reverse phase columns in series and a gradient elution of 20 to 24% $CH_3CN$ over 90′ allowed isolation of a sharp peak eluting at 36.66′ under these conditions. Analytical HPLC yielded a single peak upon co-chromatography of a known disulfide bonded cyclic standard with the product obtained from preparative HPLC. FAB-MS gave a $[M+H]^+$ of 1171, which is consistent with the the disulfide bonded cyclic structure cPND4 (SEQ ID: 23:):

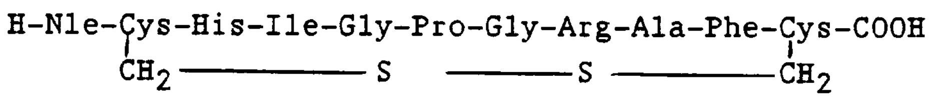

EXAMPLE 16

1. Solution Synthesis of Peptide Bonded cPN15:

The linear peptide Cbz-Nle-Lys(Boc)-Gln-Arg(Mtr)-Gly-Pro-Gly-Arg(Mtr)-Ala -Phe was synthesized following solid-phase methods on an ABI 431A peptide synthesizer using 373 milligrams (0.1 mmoles) of commercially available Fmoc-Phenylalanyl-p- alkoxybenzyl alcohol resin. With the exception of norleucine, which was purchased in the benzyloxycarbonyl (Cbz) protected form, L-amino acids used were the fluorenylmethoxycarbonyl (Fmoc) derivatives having the appropriate acid-labile side chain protecting groups. The polypeptide-derivatized resin product was transferred to a sintered glass funnel, washed with dichloromethane, and dried, to yield 0.6 g of polypeptide-resin product.

The peptide was cleaved from the resin by treatment with 6 ml of a 95:2:3 mixture of TFA:1,2 ethanediol:anisole for 16 hours. The reaction mixture was filtered through a sintered glass funnel, the resin washed with 10 ml TFA, and the filtrates combined. Following concentration to about 1 to 2 ml of yellow oil, the linear peptide was recovered by trituration with 400 ml of diethyl ether, in 50 ml portions, and filtration on a sintered glass funnel. Dissolution with 100 ml 1% TFA followed by lyophilization yielded 298 mg of linear peptide.

The peptide powder was dissolved in 800 ml DMF, neutralized with 0.42 ml diisopropylethylamine, and treated with 0.077 ml diphenylphosphorylazide. The solution was stirred in the dark for 70 hours at 4°C to allow formation of the cyclic lactam. After quenching by addition of 3 ml glacial acetic acid, the reaction mixture was concentrated to about 1 to 2 ml of oil, dissolved in 10% aqueous acetic acid, and lyophilized.

The cyclic peptide was purified by G-15 size exclusion chromatography using 5% acetic acid as the mobile phase. Fractions, monitored by UV detection, containing the peptide were pooled and lyophilized to yield 135 mg of dry cyclic peptide. All results obtained were consistent with the structure cPND15:

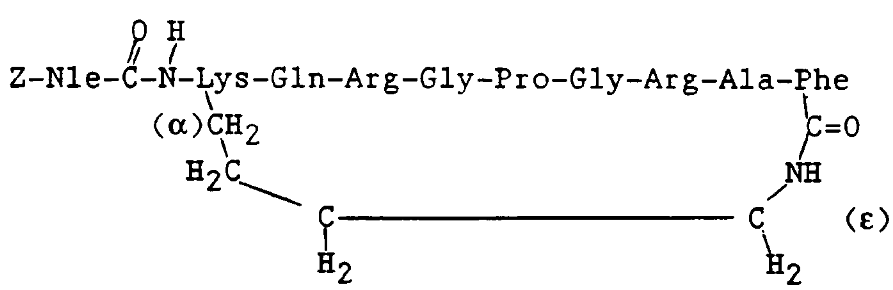

which may also be represented as:

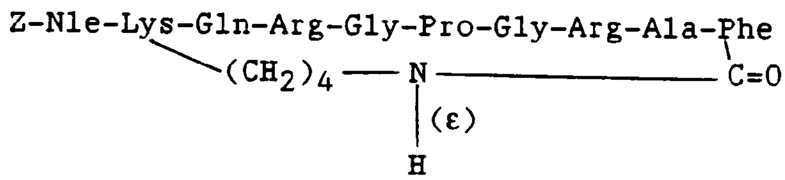

2. Deprotection of cPND15 to yield the hydrogen form:

Deprotection of cPND15 was achieved by dissolving the cyclic peptide in 20 ml of 30% aqueous acetic acid and hydrogenation at 40 psi for 16 hours over 100 mg of 10% palladium on carbon. The reaction mixture was filtered over celite to remove the catalyst, and the filtrate was lyophilized. Reverse phase HPLC using a Vydac C18 semi-prep column was utilized to obtain 8.5 mg of pure deprotected cyclic peptide. This method of deprotection is applicable to all peptides synthesized as the benzyloxycarbonyl N-protected peptide, to yield the free hydrogen form of the peptide which may now be activated at the amino terminus in preparation for conjugation. The structure of the product was confirmed by FAB-MS, analytical HPLC and amino acid analysis, and all results were consistent with the structure cPND15:

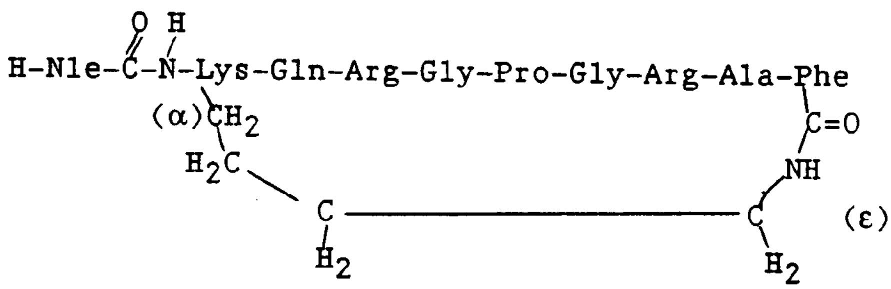

which may also be represented as

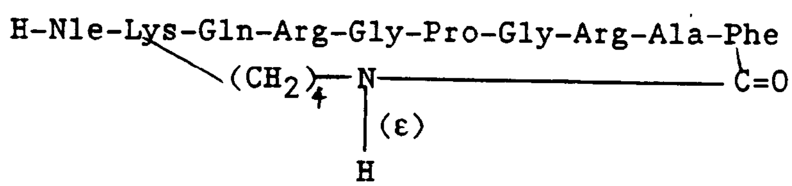

EXAMPLE 17

Synthesis of cPND31:

Two grams (0.6 meq/gram) of Fmoc-Phe-Wang resin was loaded on an ABI 431A synthesizer. Fmoc single coupling protocols were used to add Fmoc-Ala, Fmoc-Arg(Tos), Fmoc-Pro, Fmoc-Ile, Fmoc-His(Trt), Boc-Lys-(Fmoc), and Cbz-Nle to produce 3.7 grams of linear peptide resin having the sequence:
Boc-Lys($N^{\epsilon}$-Z-Nle)-His(Trt)-Ile-Gly-Pro-Gly-Arg(Tos)-Ala-Phe.

The peptide was cleaved from the resin by treating with 95% TFA, 5% water for two hours. The resin was removed by filtration, the TFA removed from the filtrate by evaporation in vacuo, and the residue was triturated with diethyl ether. The precipitate was recovered by filtration and drying to yield 1.7 grams of linear peptide having the sequence:
H-Lys($N^{\epsilon}$-Z-Nle)-His-Ile-Gly-Pro-Gly-Arg(Tos)-Ala-Phe.

The peptide was treated with Boc-isoglutamine-ONp (0.71 grams, 2 nmoles,) and DIEA (0.35 ml, 2 mmoles) in DMF (10 ml) overnight at room temperature. The DMF was evapoiated, and the residue treated with diethyl ether. The precipitate was recovered by filtration and washed with ethyl acetate. The dried peptide (1.9 grams) was treated with TFA (100 ml) for 0.5 hours. The TFA was evaporated in vacuo, the residue triturated with diethyl ether and the precipitate was recovered by filtration and dried.

The peptide was desalted on Sephadex G-10 in 10% aqueous acetic acid as the eluent. Peptide fractions were lyophilized to yield 1.2 grams (0.79 mmoles) of:

H-isoGln-Lys(N$^{\varepsilon}$-Z-Nle)-His-Ile-Gly- Pro-Gly-Arg(Tos)-Ala-Phe

Two batches (0.55 gm, 0.36 mmoles) of the peptide were separately dissolved in 1000 mL ice cold DMF and DIEA (0.16 mL, 0.9 mmoles) and DPPA (0.12 mL were added and the solutions were stirred overnight at room temperature. The DMF was evaporated in vacuo and the residues combined and solubilized in $CHCl_3$. The organic fraction was washed with 5% aqueous citric acid, then dried over $MgSo_4$ and evaporated to yield 0.78 gm of crude cyclic peptide. This material was treated with liquid HF (10 mL) containing anisole (1 mL) for two hours at 0°C. The HF was evaporated and the residue was purified by graidien elution on reveresed phase HPLC (Vydac C-18, 0-50% $CH_3CN$, over 50 minutes using 0.1 % aqueous TFA as the buffer) to give 250 mg of pure cPND31 (M+H=1204).

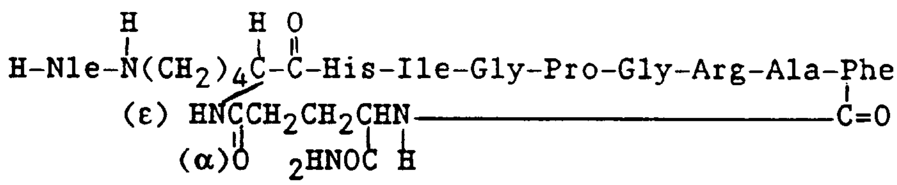

EXAMPLE 18

Preparation of MaleimidoPropionyl-cPND15

10 milligrams of cPND15 trifluoroacetate salt was dissolved in 0.3 ml of a 1:2 mixture of $H_2O$:MeCN. The solution was cooled in an ice bath and then 100 μL of 0.345 M $NaHCO_3$ solution, followed by 3.5 mg of maleimidopropionic acid N-hydroxysuccinimide ester, was added. The reaction was allowed to proceed with stirring for one hour, followed by quenching with 3 μL of trifluroacetic acid. The reaction mixture was filtered through a 0.2 micron filter, and the filter was washed with 0.2 ml of water. The combined filtrates were injected onto a 2.15 X 25 cm Vydac C18 reverse phase column. The column was eluted isocratically for 10 minutes at a flow rate of 10 ml/min. with 25% MeCN/0.1% TFA, followed by gradient elution from 25 to 40% MeCN/0.1% TFA, over 20 minutes. The product eluting between 20 and 32 min was concentrated and lyophilized, yielding 7 mg of the trifluoroacetate salt of maleimidopropionyl-cPND15 as a white amorphous powder. FAB-MS revealed a major ion (M+H) at 1262. Titration for maleimide by Ellman assay quenching gave a concentration of 0.54 μmoles per mg of the maleimidopropionyl-cPND15.

EXAMPLE 19

Preparation Of Maleimidopropionyl-cPND31:

Following the procedure of Example 13, 37.6 mg of the trifluoroacetate salt of cPND31 was reacted with 8.3 mg of maleimidopropionyl N-hydroxy-succinimide ester in 0.4 ml of a 0.322 M $NaHCO_3$ solution and 1.2 ml of 1:2 $H_2O$:MeCN, followed by quenching with 10.5 μl of TFA. Preparative HPLC (30% MeCN/0.1% TFA isocratic for 10 minutes followed by gradient elution from 30-50% MeCN over 5 min gave a product peak eluting between 18-25 min. The lyophilized product weighed 26 mg, and the maleimide titer was 0.57 μM/mg. FAB-MS gave a major ion (M+H) at 1356. Amino acid analysis gave Nle=460, β-alanine=420 and Lys=460 nmoles/mg. NMR analysis gave a singlet at 6.93 ppm (maleimide H).

EXAMPLE 20

Protocol for Inoculation of Animals with the MIEP-cPND15 and MIEP-cPND31 conjugate of this Invention:

Alum was used as an adjuvant during the inoculation series. The inoculum was prepared by dissolving the conjugate in physiologic saline at a final conjugate concentration of 300 μg/ml. Preformed alum (aluminum hydroxide gel) was added to the solution to a final level of 500 μg/ml aluminum. The conjugate was allowed to adsorb onto the alum gel for two hours at room temperature. Following adsorption, the gel with the conjugate was washed twice with physiologic saline and resuspended in saline to a protein concentration of 300 μg/ml.

African green monkeys were individually inoculated with three 300 μg doses or three 100 μg doses of the conjugate adsorbed onto alum. Each dose was injected intramuscularly. The doses were delivered one month apart (week 0, 4, 8, 28). The animals were bled at intervals of two weeks. Serum samples were prepared from

each bleed to assay for the development of specific antibodies as described in the subsequent examples.

EXAMPLE 21

Analysis of Sera for Anti-Peptide IgG Antibodies:

Each serum sample is analyzed by enzyme-linked immunoadsorbent assay (ELISA). Polystyrene microtiter plates were coated with 0.5 μg per well of the synthetic peptide (not conjugated to MIEP) in phosphate-buffered physiological saline (PBS) at 4°C. Each well was then washed with PBS containing 0.05% TWEEN-20 (PBS-T). Test serum, diluted serially in PBS-T, was added to the peptide-containing wells and allowed to react with the adsorbed peptide for one hour at 36°C. After washing with PBS-T, alkaline phosphatase-conjugated goat anti-human IgG was added to the test wells and was allowed to react for one hour at 36°C. The wells were then washed extensively in PBS-T. Each well received 0.1% p-nitrophenyl phosphate in 10% diethanolamine, pH 9.8, containing 0.5 mM $MgCl_2 \cdot 6H_2O$. The ensuing reaction was allowed to proceed at room temperature for 30 minutes, at which time it was terminated by the addition of 3.0 N NaOH.

The greater the interaction of antibodies in the test serum with the peptide substrate, the greater is the amount of alkaline phosphatase bound onto the well. The phosphatase enzyme mediates the breakdown of p-nitrophenyl phosphate into a molecular substance which absorbs light at a wavelength of 405 nm. Hence, there exists a direct relationship between the absorbance at 405 nm of light at the end of the ELISA reaction and the amount of peptide-bound antibody.

All the monkeys inoculated with the maleimidopropionyl-cPND15-MIEP and malemidopropinyl cPND31-MIEP conjugates developed antibodies specifically capable of binding the peptide.

EXAMPLE 22

Analysis of Sera for Activity which Specifically Neutralizes HIV Infectivity:

Virus-neutralizing activity is determined with an assay described by Robertson et al., J. Virol. Methods 20: 195-202 (1988). The assay measures specific HIV-neutralizing activity in test serum. The assay is based on the observation that MT-4 cells, a human T-lymphoid cell line, are readily susceptible to infection with HIV and, after a period of virus replication, are killed as a result of the infection.

The test serum is treated at 56°C for 60 minutes prior to the assay. This treatment is required to eliminate non-specific inhibitors of HIV replication. Heat treated serum, serially diluted in RPMI-1640 cell culture medium, is mixed with a standard infection dose of HIV. The dose is determined prior to the assay as containing the smallest quantity of virus required to kill all the MT-4 cells in the assay culture after a period of 7-8 days. The serum-virus mixture is allowed to interact for one hour at 37°C. It then is added to $1.0 \times 10^5$ MT-4 cells suspended in RPMI-1640 growth medium supplemented with 10% fetal bovine serum. The cultures are incubated at 37°C in a 5% $CO_2$ atmosphere for 7 days.

At the end of the incubation period, a metabolic dye, DTT, is added to each culture. This dye is yellow in color upon visual inspection. In the presence of live cells, the dye is metabolically processed to a molecular species which yields a blue visual color. Neutralized HIV cannot replicate in the target MT-4 cells and therefore does not kill the cells. Hence, positive neutralization is assessed by the development of blue color following addition of the metabolic dye.

EXAMPLE 23

Preparation of a cyclic disulfide for conjugation:

1. PREPARATION OF cPN33 (SEQ ID : 22:): H-Nle Cys Tyr Asn Lys Arg Lys Arg Ile His Ile Gly Pro Gly Arg Ala Phe Tyr Thr Thr Lys Asn Ile Ile Gly Cys-OH ($C_{135}H_{220}N_{42}O_{33}S_2$, formula weight = 3023.6)

The 26mer was assembled on the Milligen # 9050 synthesizer, starting from partially racemised Fmoc-L-Cys(Trt)-OPKA resin (Milligen batch B 090426, 0.081 meq/g), using 2.47 g (0.200 meq). The theoretical yield is 604 mg. The resin was mixed with an equal volume of glass beads (Sigma 150-212 μm). The mixture completely filled two 1 x 10 cm columns, connected in series. Reagents were Fmoc-Pfp ester (except for threonine, which was dHBt), using four fold molar excess in N-methyl pyrrolidine solvent. Side chain protection was: Tyr (tert-butyl); Lys (Boc); Arg (Mtr); His (Boc); Thr (tert-butyl); Cys (Trt). The protocol was modified to give double coupling with $Lys^7$; $Ile^9$: $Ile^{11}$; $Gly^{12}$; $Pro^{13}$; $Gly^{14}$; $Arg^{15}$; $Phe^{17}$; $Tyr^{18}$; $Thr^{19}$; $Thr^{20}$; $Ile^{23}$; $Ile^{24}$. Acylation recycle

times were extended from 30 to 60 mminutes for all units, except for Gly[14] and Ala[16], and to 90 minutes for Ile[9] (2x); Ile[11] (2x); Ile[23] (2x) and Ile[24] (2x). The derivatized resin was maintained as the free terminal amine which was washed with $CH_2Cl_2$ and air-dried.

The mixture of dry derivatized resin and glass beads was resuspended in 95% TFA, 4% ethane dithiol, 1% $CH_3SPh$ (30 mL) at 23°C in a sealed flask, with gentle stirring on an oscillating tray for 8 hours. The bright yellow mixture was then filtered and the insolubles were thoroughly extracted with 100% TFA (3 x 20 mL). The combined dark orange filtrates were evaporated to give a pale tan, oily gum. On trituration with ether (20 mL) this material instantly became a colorless solid, which was transferred to a filter by triturating with additional ether (3 x 20 mL). After drying, the crude product was obtained as a fine colorless powder (583 mg).

Analytical reverse phase HPLC [aqueous 0.1% TFA/22% $CH_3CN$, $\lambda$ = 215 nm, A = 0.05, 2.0 mL/min.] on a 0.46 x 25.0 cm Vydac $C_{18}$ column of about a 50 µg sample, dissolved in 50 µL aqueous 0.1 % TFA/20% $CH_3CN$, 4 µL injected, revealed a major component (36.29′) and a later eluting minor component. These were separately collected after injection of a 30 mg and another 50 mg aliquot of the sample onto two 2.21 x 25.0 cm preparative Vydac $C_{18}$ columns in series [linear gradient over 60′: 0.1% TFA/23-27% $CH_3CN$, $\lambda$ = 215 nm, A = 3.00, 10 mL/min]. A total of 35.2 mg of the earlier eluting material (44.45′) and 8.2 mg of the later eluting material was recovered following lyophilization. FAB-MS of the major product gave a $[M+H]^+$ = 3022.1 and an $[M+Na]^+$ = 3044.2, which is consistent with the calculated mass.

2. PREPARATION OF THE CYCLIC DISULFIDE: (SEQ ID: 22:):

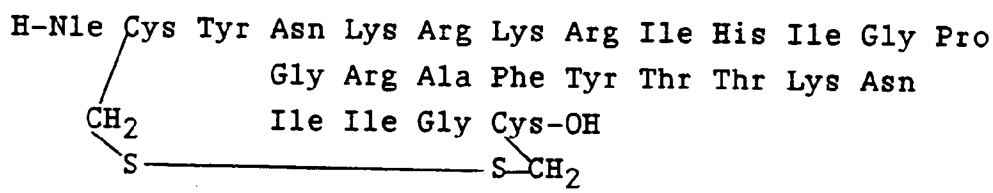

a. $K_3Fe(CN)_6$ INDUCED OXIDATION:

The linear 26 mer dithiol compound (35.0 mg) was dissolved in degassed distilled water (38 mL) at 23 °C to give a clear colorless solution at pH 2.73. The pH was adjusted to 8.5 with 0.1 N $NH_4OH$, and the solution was covered with an atmosphere of nitrogen. An aliquot of the material was immediately run on analytical reverse phase HPLC and found to be undergoing oxidation as evidenced by the appearance of an early peak.

With magnetic stirring, a freshly prepared solution of 0.01 M $K_3Fe(CN)_6$ was added by power driven hypodermic syringe at 23° C under nitrogen. Analysis of a small aliquot by HPLC revealed total conversion of starting material to an earlier elution time. The reaction mixture (pH 8.3) was mixed with 10% aqueous acetic acid and stirred to give a pH of 4.0. The solution was filtered to remove insoluble material, and the faintly yellow solution was evaporated and then lyophilized to give about 27.9 mg of a pale yellow powder. The material was dissolved in 0.1% TFA/20% $CH_3CN$ and gradient eluted on a preparative HPLC. A major early eluting peak and a later eluting peak (4:1) were separately collected and lyophilized to yield 6.1 mg of the early and 1.5 mg of the late eluting material. FAB-MS analysis of the early eluting material: $[M+H]^+$ 3019.7; $[M+Na]^+$ 3042.5; FAB-MS analysis of the late eluting material: $[M+H]^+$ 3020.0; $[M+Na]^+$ early material = 3041.5; all of which corresponds to the correct mass for the cyclized cPND33. The later eluting material is the D-cysteine carboxy terminus diastereomer.

Amino acid analysis of the products gave the predicted amino acid compositions for the cyclized products and confirmed that the later eluting material is the D-cysteine containing diastereomer.

b. AIR OXIDATION:

The linear 26 mer prepared in (1) above (86 mg, 28.4 µmoles) was dissolved in aqueous 0.1% TFA/20% acetonitrile (284 mL) at 23° C and the solution was allowed to stand open to the air.

Cyclization was monitored by reverse phase HPLC and the sample was found to be almost completely converted to the early eluting material, with almost complete dissappearance of starting linear material, by t = 24 hours. The clear, colorless solution was evaporated to about 8 mL at which point an additional 10 mg sample prepared in the same way as the 86 mg, was added. The combined sample was evaporated to about 9 mL. The cloudy colorless solution was subjected to HPLC separation, in two separate runs, on two 2.12 x 25.0 cm Vydac $C_{18}$ columns in series. Two fractions were separately collected, an early eluting peak and a later eluting

peak. Each peak was separately evaporated and lyophilized to yield 30.1 mg and 9.7 mg of the early and late materials respectively. The early eluting material was combined with other preparations of early eluting cyclized material to yield a total of 47.5 mg of a faintly bluish fluffy powder. Analytical HPLC of this material gave a single peak.

3. PREPARATION OF 3- MALEIMIDOPROPIONIC ACID ANHYDRIDE

3-Maleimidopropionic acid (226 mg) was covered with 5 mL of acetic anhydride and the mixture was heated at 130°C for 3.75 hr, and then aged over night at room temperatue. The solution was concentrated to an oil and the NMR spectrum ($CDCl_3$) indicated a mixture of the homoanhydride and the mixed anhydride of acetic and maleimidopropionic acids. The starting acid shows the methylene adjacent to the carbonyl as a triplet centered at 2.68 ppm whereas in the anhydride these resonances appear at 2.81 ppm. Purification was effected by fractional sublimation, first at 70°C and 0.2 mm and then at 120°C and 0.2 mm. The latter fraction was removed from the apparatus by dissolving in $CDCl_3$, affording 34 mg of pure homoanhydride on evaporation of the solvent. This was recrystallized from $CDCl_3$ and cyclohexane affording material melting at 143-147°C.
Calcd. for $C_{14}$ $H_{12}N_2O_7$: C,52,51;H,3.78;N,8.75.
Found: C,51.73;H3.67;N,8.16. 200 MHz NMR ($CDCl_3$):2.83 (2H,t)3.84 (2H,t),6.73 (2H,s).

4. "SELECTIVE" ACYLATION OF cPND33

cPND33 (22.5 mg; at estimated 70% peptide is equivalent to 15.75 mg or 5.212 micromoles) was dissolved in 12.0 mL of a 0.1M pH 5.25 morpholinoethane sulfonic acid buffer and cooled in an ice bath. Analysis of this solution and progress of the reaction was followed by HPLC on a 25 cm ODS column using 25% aqueous acetonitrile: 0.1% trifluoroacetic acid (TFA) as eluent. Maleimidopropionic acid anhydride (2.0 mg, 6.25 micromoles) was dissolved in 0.600 mL of dry tetrahydrofuran, and 0.5 mL of this solution (corresponding to 5.2 micromoles of anhydride) was added to the above peptide solution. After 30 sec., a 7 microliter aliquot was removed and evaluated by HPLC. This assay was repeated at 0.25, 0.50, 1.25, 2.25 and 3.0 hr. After 3.5 hr the solution was lyophilized. The lyophylizate was dissolved in 2.0 mL of 20% aqueous acetonitrile, filtered through a 0.2 micron filter and preparatively chromatographed in three 0.700 mL runs on a 21.2 mm x 25 cm Zorbax C-18 column. The following elution program was used: flow rate = 10 mL/min; isocratic elution with 25% aqueous acetonitrile/0.1% TFA (12 min); gradient to 28% acetonitrile (10 min); gradient to 35% acetonitrile (8 min). The tail fractions were isolated by concentration and lyophilization to afford 8.9 mg of recovered starting material (penultimate fraction) and 9.6 mg of a product which had a mass spectrum (FAB) indicatiing a molecular weight of 3172 (i.e the mono-maleimidopropionyl derivative of cPND33).

The product was further characterized by a sequence analysis looking for the absence of lysine (the absence of any sequence would imply terminal amino acylation). The results indicate that most but not all of the maleimidopropionyl moiety is bonded to the lysine closest to the carboxy terminus.

EXAMPLE 24

Interleukin-2-induction following exposure to MIEP.

CTLL cells (commerically available from the American Type Culture Collection designated ATCC TIB 214)[Gillis, S., and Smith, K. (1977) Nature, 268, pp.154-156] were maintained in RPMI 1640 (GIBCO), supplemented with 10% Defined Fetal Bovine Serum (HyClone), L-glutamine 2 μm (GIBCO), 100 u/mL Penicillin, 100 μM Streptomycin (GIBCO), 2-Mercaptoethanol 20 μm(BioRad), 10% Rat T-Cell Polyclone (Collaborative Research, Inc.). CTLL cells were seeded every 2 to 3 days at 1-2 X $10^4$ cells/mL.
Supernatants to be tested for IL-2 were prepared by culturing peripheral blood lymphocytes at 4 X $10^6$ cells/mL with MIEP at 50 μg/mL, PRP-OMPC at 50 μg/mL, Bovine Serum Albumin (Pierce) at 50 μg/mL, or medium alone in a 6-well plate (CoStar), containing equal volumes of each. Supernatants were harvested at intervals of 2, 3, or 4 days in culture and frozen until assay. The assay for IL-2 was performed as described by Gillis et al., J. Immunol. 120 pp.2027-2032 (1978). Two-fold serial dilutions of supernatants were prepared, and 100 μL placed into wells containing 4,000 CTLL cells which had been starved for one hour in the above medium without Rat T-Cell Polyclone. All samples were prepared in triplicate in a 96-well plate (CoStar). Plates were incubated overnight at 37°C, 8% $CO_2$. Each well was pulsed with 1 μCi $^3$H-Thymidine (Amersham) for 10 hours, harvested onto a BetaPlate filtermate (Pharmacia/LKB), and read in a BetaPlate 1205 (LKB Instruments). An IL-2 standard curve was determined simultaneously using Recombinant Human IL-2 (Cellular Products, Inc.). Quantitative determinations of IL-2 were calculated using the methodology of Gillis et al., J. Immunol. 120: pp.2027-2032

(1978). Probit analysis using the Recombinant IL-2, defined 1 U/mL of IL-2 as 50% of maximal $^3$H-Thymidine incorporation. Test supernatants were also expressed as a percent of maximum $^3$H-Thymidine incorporation from which the U/mL of IL-2 could be determined.

Figure 4 shows the result of this experiment demonstrating the ability of MIEP to induce an increase in Il-2 concentration. The results shown in Figure 4 were derived from the 3 day timepoint.

While the foregoing specification teaches the principles of the present invention, with examples provided for the purpose of illustration, it will be understood that the practice of the invention encompasses all the usual variations, adaptations, modifications, or deletions as come within the scope of the following claims and its equivalents.

## Claims

1. A protein in substanitally pure form purified from the outer membrane of a Gram-negative bacterium, which possesses immunologic enhancement and mitogenic activity in mammals.

2. The protein, according to Claim 1, which possesses immunologic enhancement and mitogenic activity in adult and infant humans.

3. The protein, according to Claim 2, wherein the Gram-negative bacterium is of the genus Neisseria.

4. The protein, according to Claim 3, wherein the bacterium is Neisseria meningitidis.

5. The protein, according to Claim 4, which corresponds to the Class II protein of the outer membrane of the bacterium Neisseria meningitidis,

6. The protein according to Claim 5, which corresponds to the Class II protein of the outer membrane of Neisseria meningitidis serogroup B .

7. The protein, according to Claim 6, which possesses immunological carrier activity.

8. The protein according to Claim 6, which possesses immunological mitogenic activity.

9. A recombinant protein in substantially pure form, produced in a recombinant host cell, which corresponds to an outer membrane protein of a Gram-negative bacterium, and which possesses immunologic enhancement and mitogenic activity in mammals.

10. The recombinant protein of Claim 9 wherein the recombinant host is either yeast cells or bacterial cells.

11. The recombinant protein, according to Claim 10, which possesses immunologic enhancement and mitogenic activity in adult and infant humans.

12. The recombinant protein, according to Claim 11, wherein the Gram-negative bacterium is of the genus Neisseria.

13. The recombinant protein, according to Claim 12, wherein the bacterium is Neisseria meningitidis.

14. The recombinant protein, according to Claim 13, which corresponds to the Class II protein of the outer membrane of the bacterium Neisseria meningitidis.

15. The recombinant protein, according to Claim 14, which corresponds to the Class II protein of the outer membrane of Neisseria meningitidis, serogroup B.

16. The recombinant protein, according to Claim 15, which possesses immunological carrier activity.

17. The recombinant protein, according to Claim 15, which possesses immunological mitogenic activity.

18. A vaccine for use in mammals, comprising the Class II protein of the outer membrane of Neisseria meningitidis serogroup B coupled to an antigen, which in mammals said vaccine will induce or enhance the formation of antibodies specific for said antigen.

19. The vaccine according to Claim 18 wherein the antigens are derived from bacteria, viruses, mammalian cells, fungi, rickettsia, allergens, poisons or venoms, synthetic peptides, and polypeptide fragments.

20. The vaccine, according to Claim 18, wherein the Class II protein of the outer membrane of Neisseria meningitidis serogroup B is a recombinant protein produced in a recombinant host cell.

21. A composition comprising an effective amount for immunization of mammalian species, of the polysaccharide/protein conjugate which comprises Haemophilus influenzae serotype B polysaccharide and the Class II outer membrane protein of Neisseria meningitidis serogroup B, and a pharmaceutically acceptable carrier.

22. The use of a protein, in substantially pure form, purified from the outer menbrane of a Gram-negative bacterium for the preparation of a medicament for increasing the immune response to antigens.

23. The use as claimed in Claim 22 wherein the protein is the Class II protein of the outer membrane of Neisseria meningitidis, serogroup B.

24. The use of the Class II protein of the outer membrane of Neisseria meningitidis, in substantially pure form, together with one or more antigens, for the preparation of a medicament for increasing the immune response to antigens.

25. The use as claimed in Claim 24 wherein the antigens are derived from bacteria, viruses, mammalian cells, fungi, rickettsia, allergens, poisons or venoms, synthetic peptides, and polypeptide fragments.

26. The use as claimed in Claim 22 wherein the protein is produced in a recombinant host cell.

27. The use as claimed in Claim 23 wherein the protein is produced in a recombinant host cell.

28. The use as claimed in Claim 24 wherein the protein is produced in a recombinant host cell.

29. The use as claimed in Claim 25 wherein the protein is produced in a recombinant host cell.

30. The use of a protein, in substantially pure form, purified from the outer membrane of a Gram-negative bacterium, for the preparation of a medicament for increasing the level of cytokines in a mammal.

31. The use as claimed in Claim 30 wherein the protein is the Class II protein of the outer membrane of Neisseria meningitidis, serogroup B.

32. The use as claimed in Claim 31 wherein the protein is produced in a recombinant host cell.

33. The use of a protein, in substantially pure form, purified from the outer membrane of a Gram-negative bacterium, for the preparation of a medicament for increasing the level of interleukin-2 in a mammal.

**34.** The use as claimed in Claim 33 of a recombinant protein produced in a recombinant host cell.

**35.** The use as claimed in Claim 33 wherein the protein is the Class II protein of the outer membrane of Neisseria meningitidis, serogroup B.

**36.** The use as claimed in Claim 35 wherein the protein is produced in a recombinant host cell.

**Claims for the following Contracting States: ES and GR**

**1.-** A process for preparing a major immunoenhancing protein (MIEP) which possesses immunologic enhancement and mitogenic activity in mammals, which comprises:

a) lysing of the outer membrane of a Gram-negative bacterium vesicles by sodium dodecylsulfate (SDS) follwed by SDS polyacrylamide gel electrophoresis (PAGE); and

b) eluting the MIEP from the gel, dialysing against a high pH buffer and concentrating.

**2.-** A process for preparing the protein, according to claim 1, which possesses immunologic enhancement and mitogenic activity in adult and infant humans.

**3.-** A process for preparing the protein, according to claim 1, wherein the Gram-negative bacterium is of the genus Neisseria.

**4.-** A process for preparing the protein, according to claim 1, wherein the bacterium is Neisseria meningitidis.

**5.-** A process for preparing the protein, according to claim 1, which corresponds to the Class II protein of the outer membrane of the bacterium Neisseria meningitidis.

**6.-** A process for preparing the protein according to claim 1, which corresponds to the Class II protein of the outer membrane of Neisseria meningitidis. serogroup B.

**7.-** A process for preparing the protein, according to claim 1, which possesses immunological carrier activity.

**8.-** A process for preparing the protein according to claim 1, which possesses immunological mitogenic activity.

**9.-** A process for preparing a recombinant protein in substantially pure form, which possesses immunologic enhancement and mitogenic activity in mammals, which comprises:

a) extracting the genomic DNA from an outer membrane protein of a Gram negative bacterium;

b) closing the genomic DNA into an apprpriate cloning vector; or, alternatively,

using the polymerasa chain reaction technique to amplfy specific DNA sequences in genomic DNA.

c) expressing the DNA in a recombinant host cell; and

d) separating the recombinant MIEP from the other cellular proteins, using an affinity column.

**10.-** A process according to claim 9 wherein the recombinant host is either yeast cells or bacterial cells.

**11.-** A process for preparing the recombinant protein according to claim 10, which possesses immunologic enhancement and mitogenic activity in adult and infant humans.

**12.-** A process according to claim 11, wherein the Gram-negative bacterium is of the genus Neisseria.

**13.-** A process, according to claim 12, wherein the bacterium is Neisseria meningitidis.

**14.-** A process for preparing the recombinant protein, according to claim 13, which corresponds to the Class II protein of the outer membrane of the bacterium Neisseria meningitidis.

**15.-** A process for preparing the recombinant protein, according to claim 14, which corresponds to the Class II protein of the outer membrane of Neisseria meningitidis, serogroup B.

**16.-** A process for preparing the recombinant protein, according to claim 15, which possesses immunological carrier activity.

**17.-** A process for preparing the recombinant protein, according to claim 15, which possesses immunological mitogenic activity.

**18.-** A process for preparing a vaccine for use in mammals, in which mammals said vaccine will induce or anhance the formation of antibodies specific for said antigen, which comprises:

coupling the Class II protein of the outer membrane of Neisseria meningitidis serogroup B to an antigen.

**19.-** A process for preparing the vaccine according to claim 18, wherein the antigens are derived from bacteria, viruses, mammalian cells, fungi, rockettsia, allergens, poisons or venoms, sythetic peptides, and polypeptide fragments.

**20.-** A process for preparing the vaccine, according to claim 18, wherein the Class II protein of the outer membrane of Neisseria meningitidis serogroup B is a recombinant protein produced in a recombinant host cell.

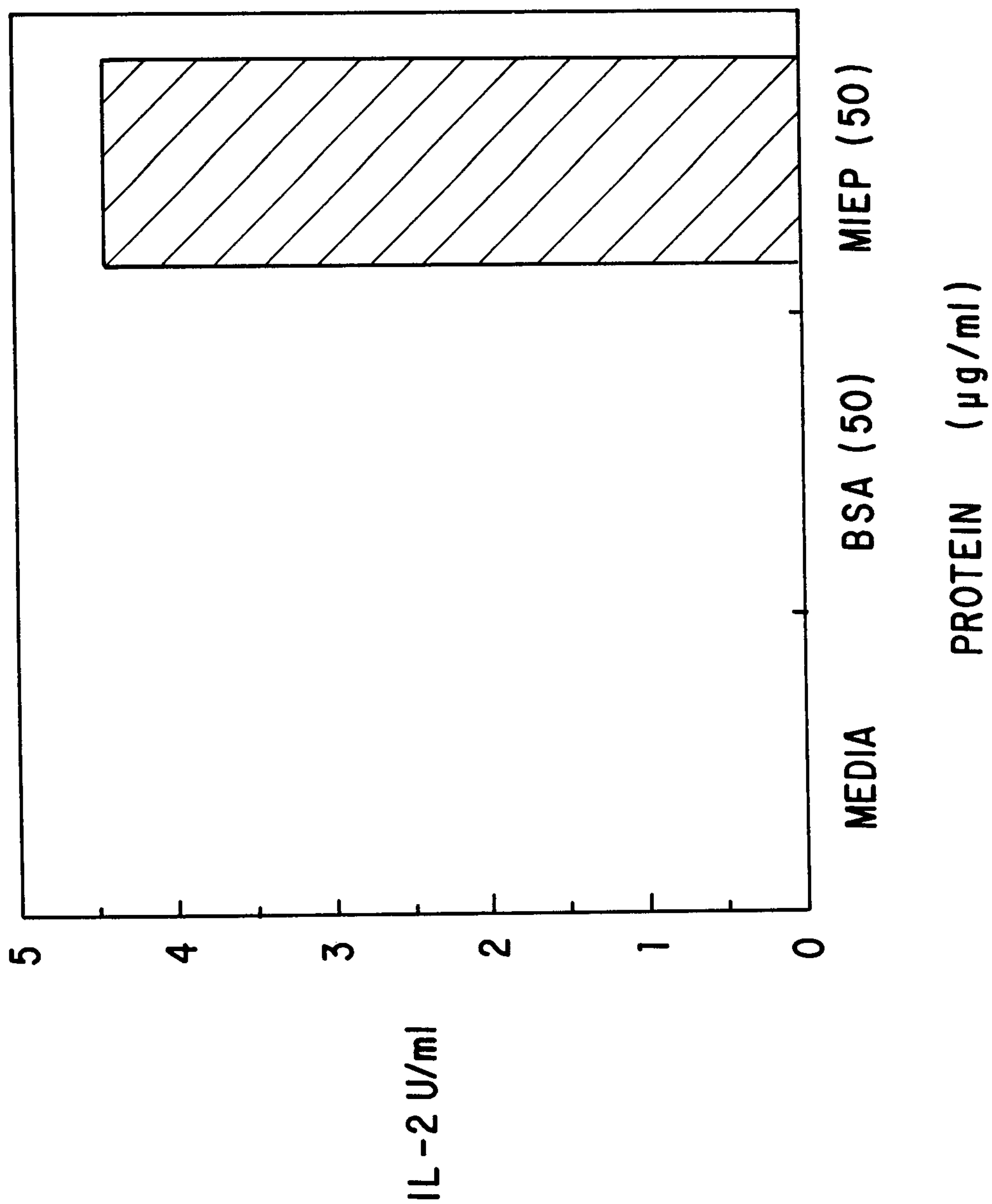


FIG. 4

European Patent Office

# EUROPEAN SEARCH REPORT

Application Number
EP 91306618.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | US - A - 4 459 286 (HILLEMAN) * Totality * -- | 1,18, 19,24, 25 | C 07 K 13/00<br>C 07 K 3/28<br>C 12 N 15/09<br>A 61 K 39/39<br>A 61 K 39/095 |
| D,Y | INFECTION AND IMMUNITY, vol. 57, No. 8, August 1989, Washington D.C., USA K.. MURAKAMI et al. "Cloning and Characterization of the Structural Gene for the Class 2 Protein of Neisseria meningitidis" pages 2318-2323 * Totality * -- | 1,6,9, 15,20 | |
| P,A | CHEMICAL ABSTRACTS, vol. 113, No. 9, August 27, 1990, Columbus, Ohio, USA J.J. DONNELLY et al. "Mechanisms of enhancement of serum antibody by a Neisseria menigitidis outer membrane protein complex conjugate vaccine" page 575, left column, abstract-No. 76 078d & Vaccines 90: Mod. Approaches New Vaccines Ind. Prev. AIDS, (Conf.) 7th 1989 (Pub. 1990), 419-24 -- | 1,18, 19,24, 25 | **TECHNICAL FIELDS SEARCHED (Int. Cl.5)**<br>C 07 K<br>C 12 N<br>A 61 K |
| A | EP - A2 - 0 372 501 (BEHRINGWERKE) * Claim 1; description * ---- | 1,18, 19,24, 25 | |

The present search report has been drawn up for all claims

| Place of search | Date of completion of the search | Examiner |
|---|---|---|
| VIENNA | 08-10-1991 | AUGUSTIN |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

SEQUENCE LISTING

(1) GENERAL INFORMATION:

(i) APPLICANT: Oliff, Allen I
Liu, Margaret A
Friedman, Arthur
Tai, Joseph Y
Donnelly, John J

(ii) TITLE OF INVENTION: THE CLASS II PROTEIN OF THE OUTER MEMBRANE OF NEISSERIA MENINGITIDIS HAVING IMMUNOLOGIC CARRIER AND ENHANCEMENT PROPERTIES, AND VACCINES CONTAINING SAME

(iii) NUMBER OF SEQUENCES: 24

(iv) CORRESPONDENCE ADDRESS:
(A) ADDRESSEE: Merck & Co., Inc.
(B) STREET: P.O. Box 2000
(C) CITY: Rahway
(D) STATE: New Jersey
(E) COUNTRY: USA
(F) ZIP: 07065

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:

(A) APPLICATION NUMBER:

(B) FILING DATE:

(C) CLASSIFICATION:

(viii) ATTORNEY/AGENT INFORMATION:

(A) NAME: Pfeiffer, Hesna J

(B) REGISTRATION NUMBER: 22640

(C) REFERENCE/DOCKET NUMBER: 18159IA

(ix) TELECOMMUNICATION INFORMATION:

(A) TELEPHONE: 908-594-4251

(B) TELEFAX: 908-594-4720

(2) INFORMATION FOR SEQ ID NO:1:

(i) SEQUENCE CHARACTERISTICS:

(A) LENGTH: 41 amino acids

(B) TYPE: amino acid

(D) TOPOLOGY: linear

(ii) MOLECULE TYPE: peptide

(iii) HYPOTHETICAL: NO

(v) FRAGMENT TYPE: internal

(ix) FEATURE:

(A) NAME/KEY: Disulfide-bond

(B) LOCATION: 3..38

(xi) SEQUENCE DESCRIPTION: SEQ ID NO:1:

Ile Asn Cys Thr Arg Pro Asn Asn Asn Thr Arg Lys Ser Ile Arg Ile
1 5 10 15

Gln Arg Gly Pro Gly Arg Ala Phe Val Thr Ile Gly Lys Ile Gly Asn
20 25 30

Met Arg Gln Ala His Cys Asn Ile Ser
35 40

(2) INFORMATION FOR SEQ ID NO:2:

(i) SEQUENCE CHARACTERISTICS:

(A) LENGTH: 24 amino acids

(B) TYPE: amino acid

(D) TOPOLOGY: linear

(ii) MOLECULE TYPE: peptide

(xi) SEQUENCE DESCRIPTION: SEQ ID NO:2:

Tyr Asn Lys Arg Lys Arg Ile His Ile Gly Pro Gly Arg Ala Phe Tyr
1 5 10 15

Thr Thr Lys Asn Ile Ile Gly Thr
20

(2) INFORMATION FOR SEQ ID NO:3:

(i) SEQUENCE CHARACTERISTICS:

(A) LENGTH: 25 amino acids

(B) TYPE: amino acid

(D) TOPOLOGY: linear

(ii) MOLECULE TYPE: peptide

(xi) SEQUENCE DESCRIPTION: SEQ ID NO:3:

```
Asn Asn Thr Thr Arg Ser Ile His Ile Gly Pro Gly Arg Ala Phe Tyr
1               5                   10                  15

Ala Thr Gly Asp Ile Ile Gly Asp Ile
            20                  25
```

(2) INFORMATION FOR SEQ ID NO:4:

(i) SEQUENCE CHARACTERISTICS:

(A) LENGTH: 25 amino acids

(B) TYPE: amino acid

(D) TOPOLOGY: linear

(ii) MOLECULE TYPE: peptide

(xi) SEQUENCE DESCRIPTION: SEQ ID NO:4:

```
Asn Asn Thr Arg Lys Ser Ile Arg Ile Gln Arg Gly Pro Gly Arg Ala
1               5                   10                  15

Phe Val Thr Ile Gly Lys Ile Gly Asn
```

(2) INFORMATION FOR SEQ ID NO:5:

(i) SEQUENCE CHARACTERISTICS:

(A) LENGTH: 18 amino acids

(B) TYPE: amino acid

(D) TOPOLOGY: linear

(ii) MOLECULE TYPE: peptide

(xi) SEQUENCE DESCRIPTION: SEQ ID NO:5:

```
Arg Ile Gln Arg Gly Pro Gly Arg Ala Phe Val Thr Ile Gly Lys Ile
1               5                   10                  15

Gly Asn
```

(2) INFORMATION FOR SEQ ID NO:6:

(i) SEQUENCE CHARACTERISTICS:

(A) LENGTH: 11 amino acids

(B) TYPE: amino acid

(D) TOPOLOGY: linear

(ii) MOLECULE TYPE: peptide

(xi) SEQUENCE DESCRIPTION: SEQ ID NO:6:

```
Arg Ile Gln Arg Gly Pro Gly Arg Phe Val Thr
1               5                   10
```

(2) INFORMATION FOR SEQ ID NO:7:

(i) SEQUENCE CHARACTERISTICS:

(A) LENGTH: 8 amino acids

(B) TYPE: amino acid

(D) TOPOLOGY: linear

(ii) MOLECULE TYPE: peptide

(xi) SEQUENCE DESCRIPTION: SEQ ID NO:7:

His Ile Gly Pro Gly Arg Ala Phe
1 5

(2) INFORMATION FOR SEQ ID NO:8:

(i) SEQUENCE CHARACTERISTICS:

(A) LENGTH: 6 amino acids

(B) TYPE: amino acid

(D) TOPOLOGY: linear

(ii) MOLECULE TYPE: peptide

(xi) SEQUENCE DESCRIPTION: SEQ ID NO:8:

Gly Pro Gly Arg Ala Phe
1 5

(2) INFORMATION FOR SEQ ID NO:9:

(i) SEQUENCE CHARACTERISTICS:

(A) LENGTH: 9 amino acids

(B) TYPE: amino acid

(D) TOPOLOGY: linear

(ii) MOLECULE TYPE: peptide

(xi) SEQUENCE DESCRIPTION: SEQ ID NO:9:

Ile Gln Arg Gly Pro Gly Arg Ala Phe
1 5

(2) INFORMATION FOR SEQ ID NO:10:

(i) SEQUENCE CHARACTERISTICS:

(A) LENGTH: 9 amino acids

(B) TYPE: amino acid

(D) TOPOLOGY: linear

(ii) MOLECULE TYPE: peptide

(xi) SEQUENCE DESCRIPTION: SEQ ID NO:10:

Ile Tyr Ile Gly Pro Gly Arg Ala Phe
1 5

(2) INFORMATION FOR SEQ ID NO:11:

(i) SEQUENCE CHARACTERISTICS:

(A) LENGTH: 9 amino acids

(B) TYPE: amino acid

(D) TOPOLOGY: linear

(ii) MOLECULE TYPE: peptide

(xi) SEQUENCE DESCRIPTION: SEQ ID NO:11:

```
Ile Ala Ile Gly Pro Gly Arg Thr Leu
1               5
```

(2) INFORMATION FOR SEQ ID NO:12:

(i) SEQUENCE CHARACTERISTICS:

(A) LENGTH: 9 amino acids

(B) TYPE: amino acid

(D) TOPOLOGY: linear

(ii) MOLECULE TYPE: peptide

(xi) SEQUENCE DESCRIPTION: SEQ ID NO:12:

```
Val Thr Leu Gly Pro Gly Arg Val Trp
1               5
```

(2) INFORMATION FOR SEQ ID NO:13:

(i) SEQUENCE CHARACTERISTICS:

(A) LENGTH: 9 amino acids

(B) TYPE: amino acid

(D) TOPOLOGY: linear

(ii) MOLECULE TYPE: peptide

(xi) SEQUENCE DESCRIPTION: SEQ ID NO:13:

Ile Thr Lys Gly Pro Gly Arg Val Ile
1 5

(2) INFORMATION FOR SEQ ID NO:14:

(i) SEQUENCE CHARACTERISTICS:

(A) LENGTH: 9 amino acids

(B) TYPE: amino acid

(D) TOPOLOGY: linear

(ii) MOLECULE TYPE: peptide

(xi) SEQUENCE DESCRIPTION: SEQ ID NO:14:

Thr Pro Ile Gly Leu Gly Gln Ser Leu
1 5

(2) INFORMATION FOR SEQ ID NO:15:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:15:

Thr Pro Ile Gly Leu Gly Gln Ala Leu
1 5

(2) INFORMATION FOR SEQ ID NO:16:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:16:

Ile His Phe Gly Pro Gly Gln Ala Leu
1 5

(2) INFORMATION FOR SEQ ID NO:17:

(i) SEQUENCE CHARACTERISTICS:

(A) LENGTH: 9 amino acids

(B) TYPE: amino acid

(D) TOPOLOGY: linear

(ii) MOLECULE TYPE: peptide

(xi) SEQUENCE DESCRIPTION: SEQ ID NO:17:

```
Ile Arg Ile Gly Pro Gly Lys Val Phe
1               5
```

(2) INFORMATION FOR SEQ ID NO:18:

(i) SEQUENCE CHARACTERISTICS:

(A) LENGTH: 4 amino acids

(B) TYPE: amino acid

(D) TOPOLOGY: both

(ii) MOLECULE TYPE: peptide

(xi) SEQUENCE DESCRIPTION: SEQ ID NO:18:

```
Gly Pro Gly Arg
1
```

(2) INFORMATION FOR SEQ ID NO:19:

(i) SEQUENCE CHARACTERISTICS:

(A) LENGTH: 4 amino acids

(B) TYPE: amino acid

(D) TOPOLOGY: both

(ii) MOLECULE TYPE: peptide

(xi) SEQUENCE DESCRIPTION: SEQ ID NO:19:

Gly Pro Gly Lys
1

(2) INFORMATION FOR SEQ ID NO:20:

(i) SEQUENCE CHARACTERISTICS:

(A) LENGTH: 4 amino acids

(B) TYPE: amino acid

(D) TOPOLOGY: both

(ii) MOLECULE TYPE: peptide

(xi) SEQUENCE DESCRIPTION: SEQ ID NO:20:

Gly Pro Gly Gln
1

(2) INFORMATION FOR SEQ ID NO:21:

(i) SEQUENCE CHARACTERISTICS:
(A) LENGTH: 4 amino acids
(B) TYPE: amino acid
(D) TOPOLOGY: both

(ii) MOLECULE TYPE: peptide

(xi) SEQUENCE DESCRIPTION: SEQ ID NO:21:

Gly Leu Gly Gln
1

(2) INFORMATION FOR SEQ ID NO:22:

(i) SEQUENCE CHARACTERISTICS:
(A) LENGTH: 26 amino acids
(B) TYPE: amino acid
(D) TOPOLOGY: both

(ii) MOLECULE TYPE: peptide

(ix) FEATURE:
(A) NAME/KEY: Modified-site
(B) LOCATION: 1
(D) OTHER INFORMATION: /label= Nle
/note= "norleucine"

(ix) FEATURE:

(A) NAME/KEY: Disulfide-bond

(B) LOCATION: 2..26

(xi) SEQUENCE DESCRIPTION: SEQ ID NO:22:

```
Leu Cys Tyr Asn Lys Arg Lys Arg Ile His Ile Gly Pro Gly Arg Ala
1               5                   10                  15

Phe Tyr Thr Thr Lys Asn Ile Ile Gly Cys
            20                  25
```

(2) INFORMATION FOR SEQ ID NO:23:

(i) SEQUENCE CHARACTERISTICS:

(A) LENGTH: 11 amino acids

(B) TYPE: amino acid

(D) TOPOLOGY: both

(ii) MOLECULE TYPE: peptide

(ix) FEATURE:

(A) NAME/KEY: Modified-site

(B) LOCATION: 1

(D) OTHER INFORMATION: /label= Nle

/note= "norleucine"

(xi) SEQUENCE DESCRIPTION: SEQ ID NO:23:

```
Leu Cys His Ile Gly Pro Gly Arg Ala Phe Cys
1               5                   10
```

(2) INFORMATION FOR SEQ ID NO:24:

(i) SEQUENCE CHARACTERISTICS:

(A) LENGTH: 4 amino acids

(B) TYPE: amino acid

(D) TOPOLOGY: both

(ii) MOLECULE TYPE: peptide

(xi) SEQUENCE DESCRIPTION: SEQ ID NO:24:

Gly Pro Gly Val

1